# EUROPEAN PATENT SPECIFICATION

(11) **EP 4 041 129 B1**
(45) Date of publication and mention of the grant of the patent: **03.12.2025**
(21) Application number: 20875226.1
(22) Date of filing: 07.10.2020
(51) Int. Cl.: A61F 2/14, A61F 2/16, A61F 9/00, A61F 9/007

(54) **PUNCTAL IMPLANTS**
PUNKTFÖRMIGE IMPLANTATE
IMPLANTS MÉATIQUES

(30) Priority: 07.10.2019 US 201962911786 P
(43) Date of publication of application: 17.08.2022
(73) Proprietor: Glaukos Corporation, San Clemente, CA 92672 (US)
(72) Inventor: CRIMALDI, Doug, San Clemente, California 92672 (US); CURRY, Kenneth, San Clemente, California 92672 (US); BENJAMINSON, Emma, San Clemente, California 92672 (US); KALINA, Chuck, San Clemente, California 92672 (US); FJIELD, Todd, San Clemente, California 92672 (US); MARTIN, Richard, San Clemente, California 92672 (US)
(74) Representative: K&L Gates LLP
(86) International application number: PCT/US2020/054619
(87) International publication number: WO 2021/071975

(56) References cited:
- US-A- 6 149 684
- US-A1- 2009 104 248
- US-A1- 2009 264 861
- US-A1- 2010 189 766
- US-A1- 2010 274 204
- US-A1- 2010 310 622
- US-A1- 2013 184 661
- US-A1- 2018 000 642
- US-B1- 6 290 684

## Description

### BACKGROUND

### Field

This disclosure relates to implantable drug delivery devices structured to provide targeted and/or controlled release of a drug to a desired ocular target tissue and methods of using such devices for the treatment of ocular diseases and disorders. In certain embodiments, this disclosure relates to devices for insertion into the punctum and for delivery of a therapeutic agent or agents to the eye in a controlled manner.

### Description of the Related Art

The mammalian eye is a specialized sensory organ capable of light reception and is able to receive visual images. The retina of the eye consists of photoreceptors that are sensitive to various levels of light, interneurons that relay signals from the photoreceptors to the retinal ganglion cells, which transmit the light-induced signals to the brain. The iris is an intraocular membrane that is involved in controlling the amount of light reaching the retina. The iris consists of two layers (arranged from anterior to posterior), the pigmented fibrovascular tissue known as a stroma and pigmented epithelial cells. The stroma connects a sphincter muscle (sphincter pupillae), which contracts the pupil, and a set of dilator muscles (dilator pupillae) which open it. The pigmented epithelial cells block light from passing through the iris and thereby restrict light passage to the pupil.

Within the eyelid resides a drainage duct for tears produced by the lacrimal glands. The portion of the drainage duct immediately following the lacrimal point, or lacrimal opening, is the lacrimal punctum. One lacrimal punctum exists in each upper and lower eyelid. Each punctum consists of an L-shaped aperture ~0.5 mm in diameter, lined with nonkeratinizing squamous epithelium surrounded by fibrous tissue.

Numerous pathologies can compromise or entirely eliminate an individual's ability to perceive visual images, including trauma to the eye, infection, degeneration, vascular irregularities, and inflammatory problems. The central portion of the retina is known as the macula. The macula, which is responsible for central vision, fine visualization and color differentiation, may be affected by age related macular degeneration (wet or dry), diabetic macular edema, idiopathic choroidal neovascularization, or high myopia macular degeneration, among other pathologies.

The cornea, lacrimal glands, mucous cells, and Meibomian glands are all richly innervated. Parasympathetic, sympathetic and sensory innervation play complex stimulatory or inhibitory roles, and neuronal pathways interact via complex surface results cascades. Abnormalities at any point in these pathways can cause overall dysregulation of lacrimal function. These abnormalities can result in a condition known as dry eye, keratoconjunctivis sicca of keratitis sicca, which is characterized by discomfort, visual disturbance and tear film instability with potential damage to the ocular surface. Allergic conjunctivitis is another condition affected by the interaction of eye tissues, typically resulting from histamine release by mast cells, and characterized by redness, swelling of the conjunctiva, itching and increased production of tears.

Other pathologies, such as abnormalities in intraocular pressure, can affect vision as well. Aqueous humor is a transparent liquid that fills at least the region between the cornea, at the front of the eye, and the lens and is responsible for producing a pressure within the ocular cavity. Normal intraocular pressure is maintained by drainage of aqueous humor from the anterior chamber by way of a trabecular meshwork which is located in an anterior chamber angle, lying between the iris and the cornea or by way of the "uveoscleral outflow pathway." The "uveoscleral outflow pathway" is the space or passageway whereby aqueous exits the eye by passing through the ciliary muscle bundles located in the angle of the anterior chamber and into the tissue planes between the choroid and the sclera, which extend posteriorly to the optic nerve. About two percent of people in the United States have glaucoma, which is a group of eye diseases encompassing a broad spectrum of clinical presentations and etiologies but unified by increased intraocular pressure. Glaucoma causes pathological changes in the optic nerve, visible on the optic disk, and it causes corresponding visual field loss, which can result in blindness if untreated. Increased intraocular pressure is the only risk factor associated with glaucoma that can be treated, thus lowering intraocular pressure is the major treatment goal in all glaucomas, and can be achieved by drug therapy, surgical therapy, or combinations thereof.

Many pathologies of the eye progress due to the difficulty in administering therapeutic agents to the eye in sufficient quantities and/or duration necessary to ameliorate symptoms of the pathology. Often, uptake and processing of the drug component of the therapeutic agent occurs prior to the drug reaching an ocular target site. Due to this metabolism, systemic administration may require undesirably high concentrations of the drug to reach therapeutic levels at an ocular target site. This can not only be impractical or expensive, but may also result in a higher incidence of side effects. Topical administration is potentially limited by limited diffusion across the cornea, or dilution of a topically applied drug by tear-action. Even those drugs that cross the cornea may be unacceptably depleted from the eye by the flow of ocular fluids and transfer into the general circulation. Thus, a means for ocular administration of a therapeutic agent in a controlled and targeted fashion would address the limitations of other delivery routes.

Current treatments for pathologies of the eye involve a number of treatments or combinations thereof, in particular but not limited to artificial tears, pharmaceutical agents (mast cell stabilizer, immunosuppressant, corticosterioid, etc.), Meibomian gland expression, warm compresses or punctal plugs. Topical pharmaceutical agents can also be used to treat allergic conjunctivitis, in particular anti-histamines, NASIDs and corticosteroids. These treatments require frequent application of pharmaceutical agents to the eye by the patients, which can add drug burden and other life restrictions for the patient. A long-term drug delivery solution would alleviate this burden.

Document US-A-2009/104248 discloses an implantable drug delivery device comprising: a proximal portion; a distal portion; and an elongate portion disposed between the proximal portion and the distal portion; wherein the proximal portion is angled, relative to the elongate portion, wherein the distal portion is angled, relative to the elongate portion, wherein the proximal portion comprises flange disposed at a proximal end of the implantable drug delivery device, wherein the proximal portion further comprises a retention portion disposed between the flange and the elongate portion, and wherein the drug delivery device is configured for insertion into a punctum of an eye, such that at least the distal portion and the elongate portion fully reside within the punctum of the eye when the drug delivery device is inserted.

### SUMMARY

In several embodiments, the implants disclosed herein operate to provide a therapeutic effect in the eye of a subject. The implants comprise a punctal plug having one or more drugs inside. The elements of the plug provide for controlled delivery of drug.

The present invention relates to an implantable drug delivery device comprising: a proximal portion; a distal portion; and an elongate portion disposed between the proximal portion and the distal portion; wherein the proximal portion is angled, relative to the elongate portion, wherein the distal portion is angled, relative to the elongate portion, wherein the proximal portion comprises flange disposed at a proximal end of the implantable drug delivery device, wherein the proximal portion further comprises a retention portion disposed between the flange and the elongate portion, wherein the retention portion comprises an elliptical cross-section, and wherein the drug delivery device is configured for insertion into a punctum of an eye, such that at least the distal portion and the elongate portion fully reside within the punctum of the eye when the drug delivery device is inserted.

### BRIEF DESCRIPTION OF THE DRAWINGS

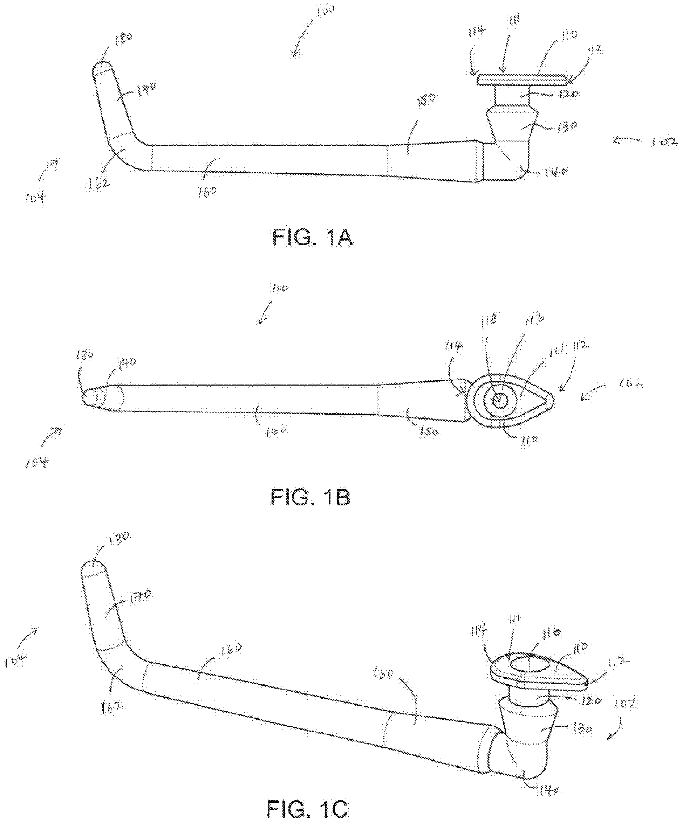

These and other features, aspects, and advantages of the present disclosure will now be described with reference to the drawings of embodiments, which embodiments are intended to illustrate and not to limit the disclosure. One of ordinary skill in the art would readily appreciated that the features depicted in the illustrative embodiments are capable of combination in manners that are not explicitly depicted, but are both envisioned Figures 1A and 1C represent an embodiment of the invention. The remaining features do not represent embodiments of the invention.
Figures 1A-1C illustrate a plurality of views of an implant having a distal retention spring in accordance with embodiments disclosed herein.
Figures 2A-2C illustrate a plurality of views of an alternative embodiment of an implant having a distal retention spring in accordance with embodiments disclosed herein.
Figures 2D-2F illustrate a plurality of views of an implant having a flexible inner portion.
Figures 3A-3C illustrate schematic cross sections of an alternative embodiment of an implant having a distal retention spring in accordance with embodiments disclosed herein.
Figures 4A-4D illustrate top views of a plurality of embodiments of a flange of an implant having a distal retention spring in accordance with embodiments disclosed herein.
Figures 5A-5B illustrate top and cross sectional views of a proximal end of an alternative embodiment of an implant having a distal retention spring in accordance with embodiments disclosed herein.
Figures 6A-6C illustrate top, perspective, and cross sectional views of a proximal end of an alternative embodiment of an implant having a distal retention spring in accordance with embodiments disclosed herein.
Figures 7A-7C illustrate top, perspective, and cross sectional views of a proximal end of an alternative embodiment of an implant having a distal retention spring in accordance with embodiments disclosed herein.
Figures 8A-8C illustrate top, perspective, and cross sectional views of a proximal end of an alternative embodiment of an implant having a distal retention spring in accordance with embodiments disclosed herein.
Figures 9A-9D illustrate top, perspective, and cross sectional views of a proximal end of an alternative embodiment of an implant having a distal retention spring in accordance with embodiments disclosed herein.
Figures 10A-10B illustrate top and cross sectional views of a proximal end of an alternative embodiment of an implant having a distal retention spring in accordance with embodiments disclosed herein.
Figures 11A-11B illustrate top and cross sectional views of a proximal end of an alternative embodiment of an implant having a distal retention spring in accordance with embodiments disclosed herein.
Figures 12A-12B illustrate top and cross sectional views of a proximal end of an alternative embodiment of an implant having a distal retention spring in accordance with embodiments disclosed herein.
Figures 13A-13B illustrate top and cross sectional views of a proximal end of an alternative embodiment of an implant having a distal retention spring in accordance with embodiments disclosed herein.
Figures 14A-14C illustrate cross-sectional views of an embodiment of an elution control member in accordance with embodiments disclosed herein.
Figures 15A-15C illustrate cross-sectional views of an alternative embodiment of an elution control member in accordance with embodiments disclosed herein.
Figure 16 illustrates an exploded view of a kit for the insertion of a punctal plug.
Figure 17A illustrates a perspective view of an insertion device for inserting a punctal plug in accordance with embodiments disclosed herein.
Figure 17B illustrates an exploded view of the insertion device of Figure 17A.
Figures 17C-17D illustrates a side and top view of the insertion device of Figure 17A.
Figure 17E illustrates a perspective view of an embodiment of an inserter tip configured to engage with the insertion device of Figure 17A.
Figure 17F illustrates a perspective view of the inserter tip of Figure 17E with a removable loader tube attached wherein the loader tube is configured to load a punctal plug into the insertion device of Figure 17A.
Figures 18A-18B illustrate perspective and cross-sectional views of the insertion device of Figure 14A and the inserter tip with removable loader tube of Figure 17F.
Figures 19A-19U illustrate perspective views of the delivery of an implant into a target site of an eye using the insertion device of Figure 17A and the inserter tip with removable loader tube of Figure 17F.
Figure 20 illustrates a schematic view of an enlarged view of the anatomy of a patient's eye and the insertion of a drug elution device in the punctum of a patient's eye.
Figure 21A illustrates a perspective view of another embodiment of an insertion device for inserting a punctal plug in accordance with embodiments disclosed herein.
Figure 21B illustrates an exploded view of the insertion device of Figure 21A.
Figures 21C-21D illustrates a side and top view of the insertion device of Figure 21A.
Figure 21E illustrates a perspective view of an embodiment of an inserter tip configured to engage with the insertion device of Figure 21A.
Figure 21F illustrates a perspective view of the inserter tip of Figure 21E with a pusher tube, wherein the pusher tube is configured to allow a punctal plug to be loaded into the insertion device of Figure 21A.
Figures 22A-22U illustrate perspective views of the delivery of an implant into a target site of an eye using the retractable inserter of Figure 12A and inserter tip of Figure 21F.
Figure 23 illustrates a graph measuring elution from a silicone tube over time wherein the silicone tube is loaded with a gel formulation.
Figure 24 illustrates a graph measuring elution from a simple tube over time wherein a chamber in the tube is loaded with a paste formulation.

### DETAILED DESCRIPTION

Achieving local ocular administration of a drug may require direct injection or application, but could also include the use of a drug eluting implant, a portion of which, could be positioned in close proximity to the target site of action within the eye or within the chamber of the eye where the target site is located, such as the anterior chamber, posterior chamber, or both simultaneously. Use of a drug eluting implant could also allow the targeted delivery of a drug to a specific ocular tissue, such as, for example, the macula, the retina, the ciliary body, the optic nerve, or the vascular supply to certain regions of the eye. Use of a drug eluting implant could also provide the opportunity to administer a controlled amount of drug for a desired amount of time, depending on the pathology.

### Drug Eluting Implant

Achieving local ocular administration of a drug may require direct injection or application, but could also include the use of a drug eluting implant, a portion of which, could be positioned in close proximity to the target site of action within the eye or within the chamber of the eye where the target site is located, such as the anterior chamber, posterior chamber, or both simultaneously. Use of a drug eluting implant could also allow the targeted delivery of a drug to a specific ocular tissue, such as, for example, the macula, the retina, the ciliary body, the optic nerve, or the vascular supply to certain regions of the eye. Use of a drug eluting implant could also provide the opportunity to administer a controlled amount of drug for a desired amount of time, depending on the pathology. For instance, some pathologies may require drugs to be released at a constant rate for just a few days, others may require drug release at a constant rate for up to several months, still others may need periodic or varied release rates over time, and even others may require periods of no release, such as a "drug holiday." Further, implants may serve additional functions once the delivery of the drug is complete. Implants may maintain the patency of a fluid flow passageway within an ocular cavity, they may function as a reservoir for future administration of the same or a different therapeutic agent, or may also function to maintain the patency of a fluid flow pathway or passageway from a first location to a second location, functioning as a stent or shunt. **In** some embodiments, an implant may also be made partially or completely biodegradable.

**In** several embodiments, the implants advantageously obviate the need for additional topical agents, such as ointments, artificial tears, and the like. **In** several embodiments, however, the implants are configured, having a particular drug release profile, to work synergistically with one or more of such agents. For example, in several embodiments, the implant is configured to deliver a constant dosage of a therapeutic agent over time to treat a damaged or diseased eye.

**In** several embodiments, the agents delivered from the implant are used for treatment of another ocular disorder, such as glaucoma, ocular hypertension, and/or elevated intraocular pressure. Drugs that are commonly delivered in the form of eye drops may be delivered by a punctal implant. Advantageously, as discussed herein, several embodiments of the implants configured for punctal placement allows metered delivery of one or more therapeutic agents; that is, delivery at a constant rate, thereby reducing the peaks and valleys of therapeutic agent concentration as occurs with topical administration, such as via eye drop.

Implants according to the embodiments disclosed herein preferably do not require an osmotic or ionic gradient to release the drug(s), are implanted with a device that minimizes trauma to the healthy tissues of the eye which thereby reduces ocular morbidity, and/or may be used to deliver one or more drugs in a targeted and controlled release fashion to treat multiple ocular pathologies or a single pathology and its symptoms. However, in certain embodiments, an osmotic or ionic gradient is used to initiate, control (in whole or in part), or adjust the release of a drug (or drugs) from an implant. In some embodiments, osmotic pressure is balanced between the interior portion(s) of the implant and the ocular fluid, resulting in no appreciable gradient (either osmotic or ionic). In such embodiments, variable amounts of solute are added to the drug within the device in order to balance the pressures.

As used herein, "drug" refers generally to one or more drugs that may be administered alone, in combination and/or compounded with one or more pharmaceutically acceptable excipients, such as binders, disintegrants, fillers, diluents, lubricants, drug release control polymers or other agents, or the like, auxiliary agents or compounds as may be housed within the implants as described herein. The term "drug" is a broad term that may be used interchangeably with "therapeutic agent" and "pharmaceutical" or "pharmacological agent" and includes not only so-called small molecule drugs, but also macromolecular drugs, and biologics, including but not limited to proteins, nucleic acids, antibodies and the like, regardless of whether such drug is natural, synthetic, or recombinant. Drug may refer to the drug alone or in combination with the excipients described above. "Drug" may also refer to an active drug or a prodrug or salt of an active drug. When the drug is in the implant, it may be referred to as a "drug load" or "drug" and it is to be understood that these terms are interchangeable.

In some embodiments, the drug diffuses through the implant itself and into the intraocular environment. In several embodiments, the outer material of the implant is permeable or semi-permeable to the drug (or drugs) positioned within an interior lumen or cavity, and therefore, at least some portion of the total elution of the drug occurs through the shell itself. In other embodiments, however, the shell of the implant is impermeable to the drug (or drugs) in the interior lumen, and the implant comprises one or more specific regions of drug release. The term "permeable" and related terms, such as "impermeable" or "semi permeable," are used herein to refer to a material being permeable to some degree (or not permeable) to one or more drugs or therapeutic agents and/or ocular fluids. The term "impermeable" does not necessarily mean that there is no elution or transmission of a drug through a material, instead such elution or other transmission is negligible or very slight, such as less than about 3% of the total amount, including less than about 2% and less than about 1%. However, in some embodiments, an impermeable outer shell permits no elution of drug through the shell.

As used herein, "patient" shall be given its ordinary meaning and shall also refer to mammals generally. The term "mammal", in turn, includes, but is not limited to, humans, dogs, cats, rabbits, rodents, swine, ovine, and primates, among others. Additionally, throughout the specification ranges of values are given along with lists of values for a particular parameter. In these instances, it should be noted that such disclosure includes not only the values listed, but also ranges of values that include whole and fractional values between any two of the listed values.

In some examples, the drug eluting implant, such as a lacrimal insert or a punctal plug, can be designed for comfort and retention. Figures 1A-1C, 2A-2C, 3A-3C, 4A-4D, 5A-5B, 6A-6C, 7A-7C, 8A-8C, 9A-9D, 10A-10B, 11A-11B, 12A-12B, 13A-13B, 14A-14C, and 15A-15C illustrate a plurality of embodiments of a drug eluting implant. As will be discussed in more detail below, the embodiments of the drug eluting implant disclosed herein are configured to be retained in the punctum of the patient while providing increased patient comfort.

In some examples the disclosed drug eluting implant can include a body comprising a variety of materials. In some embodiments, the body is made from a material that is soft, flexible, compliant, elastic and/or compressible. It may be impermeable to a drug housed within (< 1% elution is through the body), substantially impermeable (< 5% elution) or permeable (>5% elution) to a drug. The lumen contains one or more drugs, together with any excipients, stabilizers, agents to control delivery, and the like as discussed herein below. A drug(s) in the lumen may be in any form including, but not limited to, a solid (tightly or loosely packed), liquid, oil, nanospheres, liposomes, emulsion, gel, paste, micropellets, or a slurry of solid particles in a liquid.

In several embodiments, the implant is molded of a polymeric material, such as silicone, polyurethane, hydrogel, or a copolymer. In most embodiments, preferred polymeric materials are soft and flexible and allow the plug to conform to the punctum of a patient, thereby increasing the comfort of the implant over the life of implantation. In several embodiments, these materials (or combinations thereof) also facilitate the consistent manufacture of the implants. In several embodiments the size of the implant may optionally vary depending on the patient. In other embodiments, the implants are designed as a "one size fits all patients" implant that may more readily conform to various sized puncta. In several embodiments, an implants is designed to fit in either the left or right eye. In alternative embodiments, however other materials disclosed herein may be used to construct the implant (either in whole or in part). In certain embodiments, the body is biodegradable or bioerodible, while in others, it is not.

In some embodiments, the plug body itself comprises a polymer with drug distributed or dispersed throughout. Detailed methods for incorporating drugs or prodrugs into polymer matrices are disclosed in US 8,628,792, WO 2009/035565, and US 2013/0172268, the disclosures of which are incorporated herein by reference in their entireties. The distribution of drug within the polymer could be homogeneous, such as would be obtained by stirring or agitating powdered or liquid drug with a soft or flowable form of a thermoplastic polymer (such as polyurethane); or by stirring or agitating powdered or liquid drug with a pre-polymer form of a thermoset polymer (such as polydimethylsiloxane or other silicones) or hydrogel (such as polyacrylamide). Alternatively, the distribution of drug within the polymer could be heterogeneous, such as would be obtained by layering different formulations. Examples of drug core materials suitable for placing in the implants are discussed herein below in the Drugs section.

Figures 1A-1C illustrate an embodiment of a punctal plug 100. In some embodiments, the punctal plug 100 can include a proximal end 102 and a distal end 104. In some examples, the proximal end 102 of the punctal plug 100 can include a flange 110, a neck 120, and a retention portion 130. In some embodiments, the distal end 104 of the punctal plug 100 can include a punctum dilator 150, a body 160, and a distal retention portion 170. In some examples, the punctal plug 100 is configured to form a "L-shaped" configuration, wherein a proximal end 102 of the punctal plug 100 is connected to the distal end 104 of the punctal plug 100 with an angled connector. As illustrated in Figure 1A, in some embodiments, the proximal end 102 of the punctal plug 100 and the distal end 104 of the punctal plug 100 are connected by a corner 140.

In some embodiments, the punctal plug 100 can include a flange 110. In some examples, the flange 110 can have a low-profile and can be configured to have a variety of sizes. In some examples, the flange 110 can have a proximal end 102 and a distal end 104. In some embodiments, the flange 110 can have a pointed portion at the proximal end 102 of the flange 110 and a rounded portion at the distal end 104 of the flange 110. However, this configuration is not intended to be limiting as the flange 110 can have any number of shapes and configurations. A number of potential configurations of the flange 110 are described in more detail below. In some examples, the flange 110 can include a top surface 111 that can be configured to lie flush against a target tissue for treatment. Figure 1B illustrates a top view of the flange 110 of the punctal plug 100. In some embodiments, the top surface 111 can include an opening 116 that is fluidly connected to the channel 118. As will be discussed in more detail below, in some embodiments, the channel 118 is configured to provide a fluid connection between the interior of the punctal plug 100 and the opening 116 in the top surface 111.

In some examples, the punctal plug 100 can include a neck 120 and a retention portion 130. As illustrated in Figures 1A and 1C, the neck 120 can be attached to the flange 110 and the retention portion 130. In some embodiments, the diameter of the neck 120 can be less than either the diameter of the flange 110 or the retention portion 130. In some examples, the narrowed diameter of the neck 120 can help to seat the proximal end 102 of the punctal plug 100 in the punctum of the patient such that the flange 110 is retained securely against a surface of the eye. As discussed above, in some examples, the channel 118 can extend at least through a portion of the neck 120. According to the present invention, the retention portion 130 has an elliptical cross-section. The elliptical configuration of the retention portion 130 provides for strong retention of the punctal plug 100 using a smaller equivalent diameter. This can help to reduce tissue stretch, such as in the punctum, which can reduce patient discomfort. As noted above, the differing diameters of the flange 110, neck 120, and the retention portion 130 can help to retain the punctal plug 100 in the punctum of the patient upon insertion.

In some embodiments, the punctal plug 100 can include a punctum dilator 150 and a body 160. As shown in Figures 1A-1C, the punctum dilator 150 can be positioned at an angle from the proximal end 102 of the punctal plug 100. As noted above, in some embodiments, the punctum dilator 150 can be attached to the retention portion 130 through a corner 140. In some embodiments, the corner 140 can form an angle of 90°. In some examples, the punctum dilator 150 is round. In some embodiments, the diameter of the punctum dilator 150 is similar in diameter with the corner 140 such that the tissue is stretched to the required size to allow the corner 140 to be passed when the punctal plug 100 is inserted. In some embodiments, the punctum dilator 150 can be tapered such that the diameter of the punctum dilator 150 increases in a proximal direction. In some embodiments, the increasing diameter of the punctum dilator 150 can facilitate the insertion of the punctal plug 100. As the punctal plug 100 is inserted in a distal to proximal direction, the gradual increase in diameter of the punctum dilator 150 can help to stretch and widen the punctum to allow for the retention portion 130 to be inserted. In some embodiments, the body 160 can also be tapered such that the diameter of the body 160 increases in a proximal direction. In some examples, like the punctum dilator 150, the body 160 can facilitate in the insertion of the punctal plug 100 by gradually widening the diameter of the punctum of the patient.

In some examples, the distal end 104 of the punctal plug 100 can include a retention portion 170. In some embodiments, the retention portion 170 has a spring design and is configured to provide a push-pull interaction with the punctum. In some examples, the retention portion 170 is made of silicone. During insertion of the punctal plug 100 into the punctum of the patient, the canaliculus tissue attempts to straighten the distal retention portion 170. In some examples, the silicone material is configured to push back on the tissue. This then results in a push-pull event that is configured to cause the proximal end 102 of the punctal plug 100 to be pulled into the punctum during insertion. In some embodiments, the retention portion 170 is configured to help retain the punctal plug 100 in the punctum of the patient to prevent it from being dislodged. In some embodiments, to provide for the retention portion 170 to have the push-pull design described above, the distal end 104 of the punctal plug 100 can include a bend 162 that is configured to angle the retention portion 170 away from the body 160. In some examples, the retention portion 170 can include a tip 180. In some embodiments, the tip 180 can be a self-dilating tip.

In some examples, the punctal plug 100 can have distinct "right" and "left" designs so as to increase the comfort for a patient with an inserted punctal plug 100 - particularly in patients with an ectopic punctum. In some embodiments, the flange 110 of the punctal plug 100 can be asymmetrical

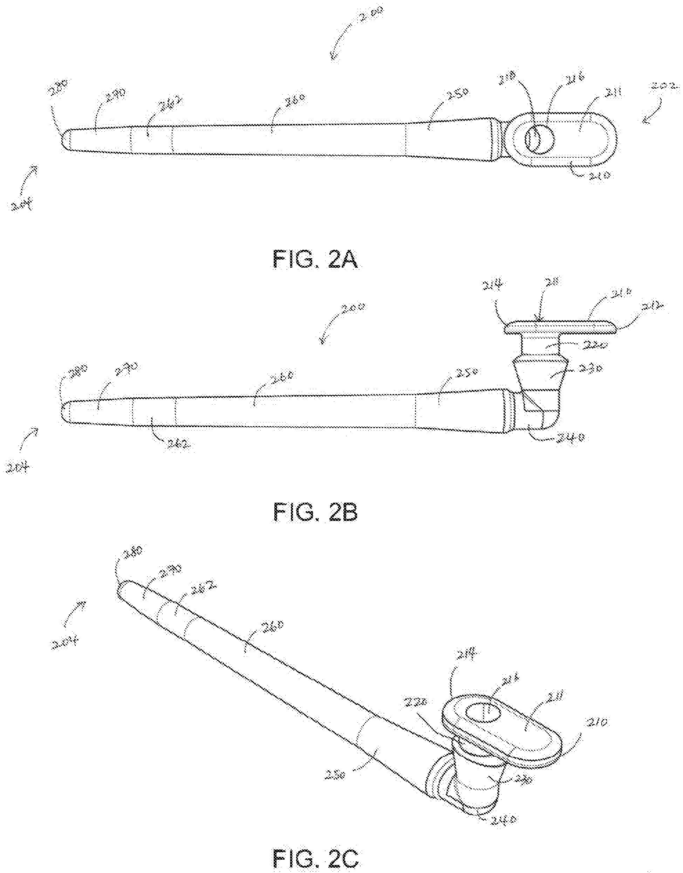

In some embodiments, the punctal plug can be configured to have flanges having different shapes and configurations. For example, Figures 2A-2C illustrate another embodiment of the punctal plug 200. Like the punctal plug 100, the punctal plug 200 can include a proximal end 202 and a distal end 204. In some embodiments, the proximal end 202 of the punctal plug 200 can include a flange 210, a neck 220, and a retention portion 230. In some embodiments, the distal end 204 of the punctal plug 200 can include a punctum dilator 250, a body 260, and retention portion 270. Like the punctal plug 100, the punctal plug 200 can be configured to form a "L-shaped" configuration, wherein a proximal end 202 of the punctal plug 200 is connected to the distal end 204 of the punctal plug 200 with an angled connector. Figures 2B-2C illustrate examples of the corner 240 that are configured to connect the proximal end 202 of the punctal plug 200 and the distal end 204 of the punctal plug 200.

As discussed with regard to the flange 110 of the punctal plug 100, the flange 210 of the punctal plug 200 can have a low-profile and can be configured to have a variety of sizes. The flange 210 can have a proximal end 202 and a distal end 204. **In** the embodiment illustrated in Figures 2A-2C, the flange 110 can have a profile that is curved at the proximal end 102 and the distal end 104 to form an oval. Again, the configuration of the flange 210 is not intended to be limiting, as will be illustrated in more detail below.

**In** some examples, the punctal plug 200 can include a neck 220 and a retention portion 230. As shown in Figures 2B and 2C, the neck 220 can be attached to the flange 210 and the retention portion 230. In some embodiments, the diameter of the neck 220 can be less than the diameter of either the flange 210 or the retention portion 230. In some examples, the narrowed diameter of the neck 220 can help to seat the proximal end 202 of the punctal plug 200 in the punctum of the patient such that the flange 210 is retained securely against a surface of the eye. As discussed above, in some examples, the channel 218 can extend at least through a portion of the neck 220. Like the retention portion 130, the retention portion 230 can have an elliptical cross-section. The elliptical configuration of the retention portion 230 can provide for strong retention of the punctal plug 200 using a smaller equivalent diameter. This can help to reduce tissue stretch, such as in the punctum, which can help to reduce patient discomfort. As noted above, the different diameters of the flange 210, neck 220, and the retention portion 230 can help to retain the punctal plug 200 in the punctum of the patient upon insertion.

In some embodiments, the punctal plug 200 can include a punctum dilator 250 and a body 260. As shown in Figures 2A-2C, the punctum dilator 250 can be positioned at an angle from the proximal end 202 of the punctal plug 200. As discussed above, in some embodiments, the punctum dilator 250 can be attached to the retention portion 230 through a corner 240. In some embodiments, the corner 240 can form a 90° angle. In some examples, the punctum dilator 250 is round. As discussed in relation to the punctum dilator 150 above, the diameter of the punctum dilator 250 can be similar in diameter with the corner 240 such that the tissue is stretched to the required size to allow the corner 240 to be passed when the punctal plug 200 is inserted. In some embodiments, the punctum dilator 250 can be tapered such that the diameter of the punctum dilator 250 increases in a proximal direction. In some embodiments, the increasing diameter of the punctum dilator 250 can facilitate the insertion of the punctal plug 200. As the punctal plug 200 is inserted in a distal to proximal direction, the gradual increase in diameter of the punctum dilator 250 can help to stretch and widen the punctum to allow for the retention portion 230 to be inserted. In some embodiments, any of the body 260, the bend 262, the retention portion 270, and the tip 280 can also be tapered such that the diameter of the distal end 204 increases in a proximal direction. In some examples, like the punctum dilator 250, any of the body 260, the bend 262, the retention portion 270, and the tip 280 can facilitate in the insertion of the punctal plug 200 by gradually widening the diameter of the punctum of the patient.

As discussed above, the embodiments of the punctal plugs disclosed herein, such as punctal plug 100 and punctal plug 200, can be configured to be drug eluting. By positioning the punctal plug in close proximity to a target site within the eye or within the chamber of the eye, the punctal plug can be configured to provide targeted delivery of a drug to a specific ocular tissue. Examples of drugs that can be delivered by the punctal plugs disclosed herein are described in additional detail below.

In some embodiments, the disclosed drug load can comprise a matrix of drug and polymer. In some examples, the drug load comprises a solid that slowly elutes drug at a controller rate into the target ocular tissue of the patient.

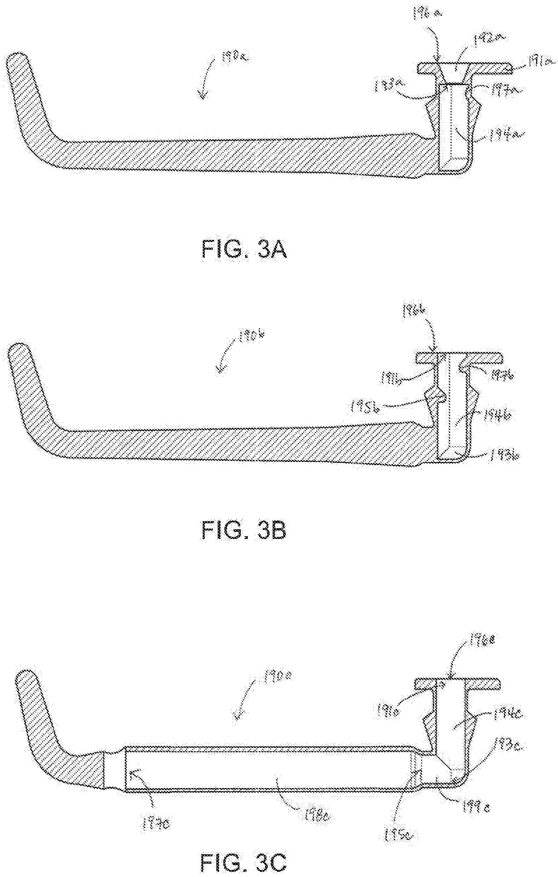

Figures 3A-3C illustrate an example cross-sections of the disclosed punctal plugs and where drug is stored for delivery. The drug loads disclosed below are not intended to be limited to any one configuration of punctal plug, and can be used in combination with any number of punctal plug configurations. Figure 3A illustrates a cross-sectional view of a punctal plug with a drug load 190a. As shown in Figure 3A, in some examples the drug load 190a comprises a vertical section. The vertical section of the drug load 190a can include a top portion 192a and a first body portion 194a. In some embodiments, the top portion 192a can be located in the flange 110 and the neck 120 of the punctal plug 100. In some examples, the top portion 192a can include a proximal end 191a and a distal end 193a. As illustrated in Figure 3A, the top portion 192a can form a taper, such that the diameter of the top portion 192a decreases from the proximal end 191a to the distal end 193a. In some embodiments, the proximal end 191a of the top portion 192a is located in the flange 110 of the punctal plug 100 and the distal end 193a of the top portion 192a can extend into a portion of the neck 120. In some embodiments, the proximal end 191a of the top portion 192a can include a contact surface 196a. In some examples, the contact surface 196a can be configured to lay flush with the top surface 111 of the flange 110. The contact surface 196a can be configured such that drug from the drug load 190a can directly contact the target portion of the eye to deliver the drug. In some embodiments, first body portion 194a can extend at a proximal end from a portion of the neck 120 to the corner 140 at a distal end. In some embodiments, the first body portion 194a can include a cut out 197a. In some examples, the drug load 190a can receive a volume of 320 nL. In some embodiments, the drug load 190a can provide approximately 200 µg of API.

Figure 3B illustrates a cross-sectional view of a punctal plug with a drug load 190b. As shown in Figure 3B, in some examples, the drug load 190b comprises a vertical section. In contrast with the drug load 190a, in some examples, the drug load 190b can include a single continuous portion. As illustrated, in some examples, the vertical portion of the drug load 190b can comprise a first body portion 194b. In some embodiments, the first body portion 194b can include a proximal end 191b and a distal end 193b. As shown in Figure 3B, the proximal end 191b of the first body portion 194b can be located in the flange 110 of the punctal plug 100 while a distal end 193b of the first body portion 194b can be located in the corner 140 of the punctal plug 100. In some embodiments, the proximal end 191b of the first body portion 194b can include a contact surface 196b. In some examples, the contact surface 196b can be configured to lay flush with the top surface 111 of the flange 110. The contact surface 196b can be configured such that drug from the drug load 190b can directly contact the target location of the eye to deliver the drug from the drug load 190b. In some embodiments, the first body portion 194b can include a cut out 195b and a cut out 197b. In some examples, the cut out 195b is located on a first side of the first body portion 194b and is distal to the cut out 197b that is located on a second side of the first body portion 194b. In some embodiments, the drug load 190b can provide approximately 200 µg of API. In some examples, the drug load 190b can receive a volume of 320 nL, for example.

Figure 3C illustrates a cross-sectional view of a punctal plug with a drug load 190c. As shown in Figure 3C, in some examples, the drug load 190c comprises a vertical section - a first body portion 194c and a horizontal section - a second body portion 198c. In some examples, the drug load 190c can carry a greater volume of drug within the punctal plug 100 in comparison to the drug load 190a and the drug load 190b. In some examples, the first body portion 194c can include a 191c and a 193c. As shown in Figure 3C, the 191c of the first body portion 194c can be located in the flange 110 of the punctal plug 100. In some examples, the 193c of the first body portion 194c can be located in a portion of the corner 140 of the punctal plug 100. In some examples, the 191c of the 104c can include a contact surface 196c. In some embodiments, the contact surface 196c can be configured to lay flush with the top surface 111 of the flange 110. As discussed above, the contact surface 196c can be configured such that drug from the drug load 190c can directly contact the target location of the eye to deliver drug from the drug load 190c. In some embodiments, the second body portion 198c can include a proximal end 195c and a distal end 197c. As provided in Figure 3B, the proximal end 195c of the second body portion 198c can be located in a portion of the corner 140 of the punctal plug 100. In some examples, the distal end 197c of the second body portion 198c can be located adjacent to the bend 162 of the punctal plug 100. In some embodiments, the second body portion 198c extends through the punctum dilator 150 and body 160 of the punctal plug 100 described above. In some embodiments, the drug load 190c can provide approximately 1000 µg of API. In some examples, the drug load 190b can receive a volume of 2200 nL.

In some embodiments, the drug load 190a, drug load 190b, and the drug load 190c can provide an initial elution range of 1 µg per day, 2 µg per day, 3 µg per day, 4 µg per day, 5 µg per day, 6 µg per day, 7 µg per day, 8 µg per day, 9 µg per day, 10 µg per day. In some examples, the drug load 190a, drug load 190b, and drug load 190c can include drug elution rates from at least 1 µg to about 5 µg per day, from about 5 µg to about 10 µg per day, from about 2 µg to about 4 µg per day, from about 4 µg to about 6 µg per day, from about 6 µg to about 8 µg per day, from about 8 µg to about 10 µg per day, from about 3 µg to about 8 µg per day. In some embodiments, the drug load 190a, drug load 190b, and drug load 190c can include drug elution rates ranging from drug elution rates of 0.2 µg per day, 0.3 µg per day, 0.4 µg per day, 0.5 µg per day, 0.6 µg per day,0.7 µg per day, 0.8 µg per day, 1.0 ng per day, 1.1 µg per day, 1.2 µg per day, 1.3 µg per day, 1.4 µg per day, 1.5 µg per day, 1.6 µg per day, 1.7 µg per day, 1.8 µg per day, 1.9 µg per day, 2.0 µg per day, 2.1 µg per day, 2.2 µg per day, 2.3 µg per day, 2.4 µg per day, 2.5 µg per day, 2.6 µg per day, 2.7 µg per day, 2.8 µg per day, 2.9 µg per day, 3.0 µg per day, 3.1 µg per day, 3.2 µg per day, 3.3 ng per day, 3.4 µg per day, 3.5 µg per day, 3.6 µg per day, 3.7 µg per day, 3.8 µg per day, 3.9 µg per day, 4.0 µg per day, 4.1 µg per day, 4.2 µg per day, 4.3 µg per day, 4.4 µg per day, 4.5 µg per day, and from about 0.2 µg per day to about 0.8 µg per day, from about 0.8 µg per day to about 1.0 µg per day to about 1.5 µg per day, from about 1.5 µg per day to about 2.0 µg per day, from about 2.0 µg per day to about 2.5 µg per day, from about 2.5 µg per day to about 3.0 µg per day, from about 3.0 µg per day to about 3.5 µg per day, from about 3.5 µg per day to about 4.0 µg per day, from about 4.0 µg per day to about 4.5 µg per day.

In some embodiments, the drug loads described above can become one with size variations intended to retain filaments. IN some embodiments, filaments are placed in both the vertical and horizontal compartments by cutting a hole in a separation wall. In some examples, the separation wall can act as a retention method for the filament.

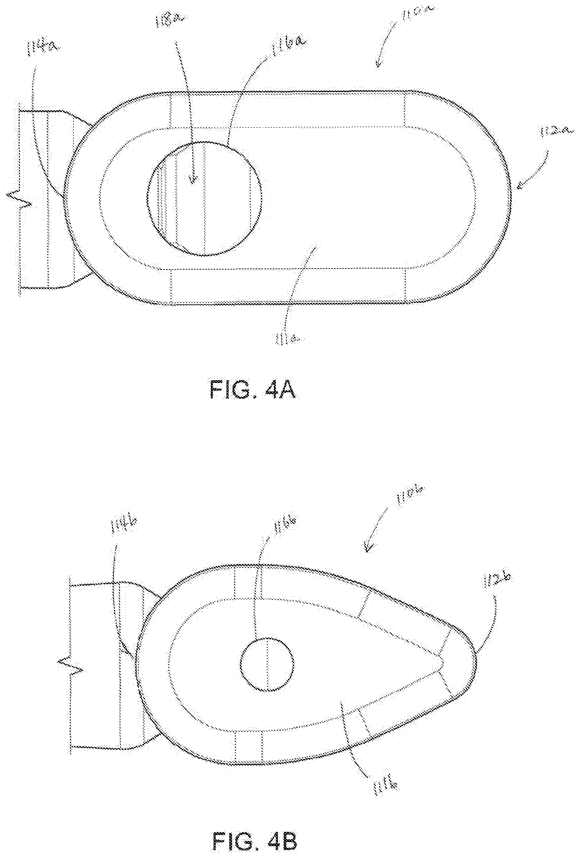

As discussed above, the disclosed punctal plugs can be configured to have flanges having a variety of shapes and sizes. For example, Figures 4A-4C illustrates a plurality of embodiments of flanges. Each of the flanges discussed below are similar to the flange 110 and flange 210 discussed above and can be used in combination with any of the punctal plug 100 and punctal plug 200 described above. Figure 4A illustrates an embodiment of a flange 110a having a proximal end 112a and a distal end 114a. In some embodiments, the flange 110a includes a top surface 111a that includes an opening 116a. In some examples, the opening 116a is fluidly connected to a channel 118a. As noted above, the channel 118a is configured to extend at least through a portion of the proximal end of a punctal plug so as to allow drug from the drug load to elute through the opening 116a. As illustrated, in some examples, the flange 110a has an elongated oval shape. In some embodiments, the flange 110a has a curved proximal end 112a and a curved distal end 114a and straight sides extending between the proximal end 112a and the distal end 114a. In some embodiments, the flange 110a is symmetrical along an axis that bisects the proximal end 112a and distal end 114a of the flange 110a. In some examples, the distance between the distal end 114a of the flange 110a and a midpoint of the opening 116a can be approximately 0.025 inches. In some embodiments, the distance between the proximal end 112a of the flange 110a and the midpoint of the opening 116a can be approximately 0.056 inches.

Figure 4B illustrates another embodiment of the flange 110b having a proximal end 112b and a distal end 114b. In some embodiments, the flange 110b includes a top surface 111b that includes an opening 116b. In some examples, the opening 116b is fluidly connected to a drug load within the punctal plug. As illustrated, in some examples, the flange 110b has a teardrop shape. In some embodiments, the flange 110b has pointed proximal end 112b and a curved distal end 114b. In some embodiments, the flange 110b is symmetrical along an axis that bisects the proximal end 112b and distal end 114b of the flange 110b. In some examples, the distance between the distal end 114b of the flange 110b and a midpoint of the opening 116b can be approximately 0.025 inches. In some embodiments, the distance between the proximal end 112b of the flange 110b and the midpoint of the opening 116b can be approximately 0.040 inches.

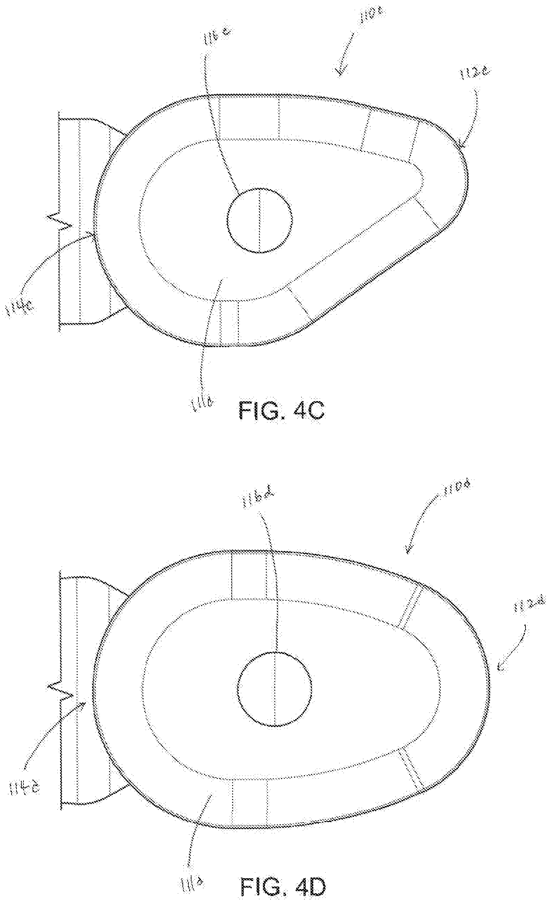

Figure 4C illustrates another embodiment of the flange 110c having a proximal end 112c and a distal end 114c. In some embodiments, the flange 110c includes a top surface 111c that includes an opening 116c. In some examples, the opening 116c is fluidly connected to a drug load within the punctal plug. As illustrated, in some examples, the flange 110c can have an asymmetrical teardrop shape. In some embodiments, the flange 110c has a pointed proximal end 112c and a curved distal end 114c. In some examples, the flange 110c has a straight portion extending along one side of the flange 110c between the proximal end 112c and the distal end 114c. In some embodiments, the flange 110c is asymmetrical along an axis that bisects the proximal end 112c and the distal end 114c of the flange 110c. In some examples, the distance between the distal end 114c of the flange 110c and a midpoint of the opening 116c can be approximately 0.025 inches. In some embodiments, the distance between the proximal end 112c of the flange 110c and the midpoint of the opening 116c can be approximately 0.032 inches.

Figure 4D illustrates an embodiment of a flange 110d having a proximal end 112d and a distal end 114d. In some embodiments, the flange 110d includes a top surface 111d that includes an opening 116d. In some examples, the opening 116d is fluidly connected to a drug load within the punctal plug. As illustrated, in some examples, the flange 110d has an oval or egg shape. In some examples, the flange 110d has a curved proximal end 112d and a curved distal end 114d. In some embodiments, the curved distal end 114d is wider than the curved proximal end 112d. In some embodiments, the sides of the flange 110d is curved on either side of the flange 110d. In some embodiments, the flange 110d is symmetrical along an axis that bisects the proximal end 112d and distal end 114d of the flange 110d. In some examples, the distance between the distal end 114d of the flange 110d and a midpoint of the opening 116d can be approximately 0.025 inches. In some embodiments, the distance between the proximal end 112d of the flange 110d and the midpoint of the opening 116d can be approximately 0.030 inches.

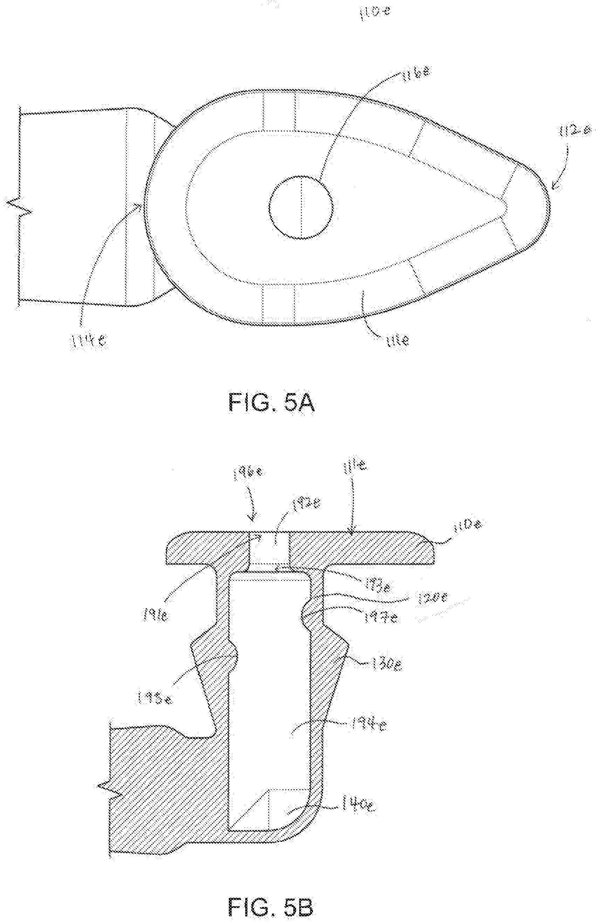

Figures 5A-5B, 6A-6C, 7A-7C, 8A-8C, 9A-9D, 10A-10B, 11A-11B, 12A-12B, 13A-13B, 14A-14C, and 15A-15C illustrate a plurality of embodiments of the proximal end of the punctal plug disclosed herein. The disclosed embodiments are not intended to be limiting, and any of the features, such as the flanges and the configuration of the drug load, of any of the punctal plugs can be combined with any other feature of the punctal plug.

Figures 5A-5B illustrate an embodiment of a flange 110e and a drug load 190e of a proximal end of another embodiment of a punctal plug. Figure 5A illustrates an embodiment of a flange 110e having a proximal end 112e and a distal end 114e. In some embodiments, the flange 110e includes a top surface 111e that includes an opening 116e. In some examples, the opening 116e is fluidly connected to the first body portion 194e illustrated in Figure 5B. In some examples, the flange 110e has a teardrop shape. As shown, the flange 110e can have a pointed proximal end 112e and a curved distal end 114e. In some embodiments, the flange 110e is symmetrical along an axis that bisects the proximal end 112e and the distal end 114e of the flange 110e. In some examples, the distance between the distal end 114e of the flange 110e and a midpoint of the opening 116e can be approximately 0.025 inches.

Figure 5B illustrates a cross-sectional view of the punctal plug with a drug load 190e. As shown in Figure 5B, in some examples, the drug load 190e comprises a vertical section. The vertical section of the drug load 190e can include a top portion 192e and a first body portion 194e. In some embodiments, the top portion 192e can be located in the flange 110e and a portion of the neck 120e of the punctal plug. In some examples, the top portion 192e can include a proximal end 191e and a distal end 193e. As illustrated in Figure 5B, the top portion 192e can form a cylinder, such that the diameter of the proximal end 191e and the distal end 193e are approximately the same. In some embodiments, the proximal end 191e of the top portion 192e can include a contact surface 196e. In some examples, the contact surface 196e can be configured to lay flush with the top surface 111e of the flange 110e. The contact surface 196e can be configured such that drug from the drug load 190e can elute from the contact surface 196e to contact the target location, such as the eye, to deliver the drug. In some embodiments, the first body portion 194e can extend at a proximal end from a portion of the neck 120e to the corner 140e at a distal end. In some examples, the diameter of the first body portion 194e can be greater than the diameter of the distal end 193e. In some embodiments, the first body portion 194b can include a cut out 195e and a cut out 197e. In some examples, the cut out 195e is located on a first side of the first body portion 194e and is distal to the cut out 197e that is located on a second side of the first body portion 194e. In some embodiments, the drug load 190e can provide approximately 200 µg of API. In some examples, the drug load 190e can receive a volume of 320 nL.

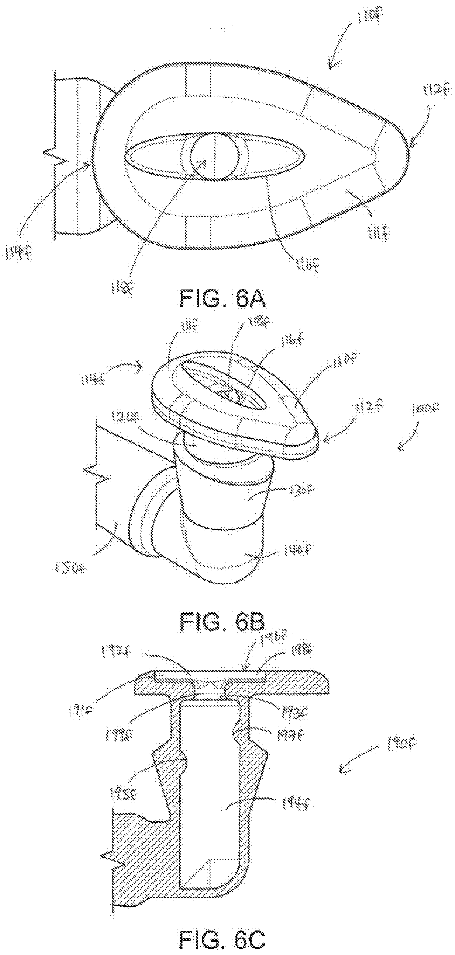

Figures 6A-6C illustrate an embodiment of a flange 110f and a drug load 190e of a proximal end of another embodiment of a punctal plug 100f. Figures 6A-6B illustrate a proximal end of the punctal plug 100f. Figure 6A illustrates an embodiment of a flange 110f having a proximal end 112f and a distal end 114f. Figure 6B illustrates the proximal end of the punctal plug 100f having the flange 110f, neck 120f, retention portion 130f, corner 140f, and the punctum dilator 150f discussed in more detail above. Turning first to the flange 110f, in some embodiments, the flange 110f includes a top surface 111f that includes an opening 116f. In some examples, the opening 116f is fluidly connected to a channel 118f. As noted above, the channel 118f is configured to extend at least through a portion of the proximal end of the flange 110f so as to allow drug from the drug load 190f to elute through the opening 116f. In some embodiments, the opening 116f is in the shape of a narrowed oval wherein the opening 116f extends between the distal end 114f and the proximal end 112f of the flange 110f. In some embodiments, the opening 116f is closer to the distal end 114f. In some examples, the opening 116f has a greater diameter along a first axis that bisects the proximal end 112f and the distal end 114f than along a second perpendicular axis. In some embodiments, the opening 116f is pointed at the proximal and distal ends of the opening 116f. In some embodiments, the opening of the channel 118f has a diameter that is approximately the same as the diameter of the opening 116f along the second axis. As illustrated, in some examples, the flange 110f has a teardrop shape. As shown, the flange 110f can have a pointed proximal end 112f and a curved distal end 114f. In some embodiments, the flange 110f is symmetrical along an axis that bisects the proximal end 112f and the distal end 114f of the flange 110f. In some examples, the distance between the distal end 114f of the flange 110f and a midpoint of the opening 116f can be approximately 0.025 inches.

Figure 6B illustrates a cross-sectional view of the flange 110f with a drug load 190f. As shown in Figure 6B, in some examples, the drug load 190f comprises a vertical section. The vertical section of the drug load 190f can include a top portion 192f having a proximal end 191f and a distal end 193f. As illustrated in Figure 6B, the top portion 192f can form a first portion 198f at the proximal end 191f that has a greater diameter than a second portion at the distal end 193f that has a smaller diameter. In some embodiments, the proximal end 191f of the top portion 192f can have a contact surface 196f. In some examples, the contact surface 196f can be configured to lay flush with the top surface 111f of the flange 110f. The contact surface 196f can be configured such that drug from the drug load 190f can elute from the contact surface 196f to contact the target location to deliver the drug. In some embodiments, the first body portion 194f can extend at a proximal end from a portion of the neck 120f to the corner 140f at a distal end. In some embodiments, the first body portion 194f can include a cut out 195f and a cut out 197f. In some examples, the cut out 197f is located on a first side of the first body portion 194f and is distal to the cut out 195f that is located on a second side of the first body portion 194f. In some embodiments, the drug load 190f can provide approximately 200 µg of API. In some examples, the drug load 190f can receive a volume of 320 nL.

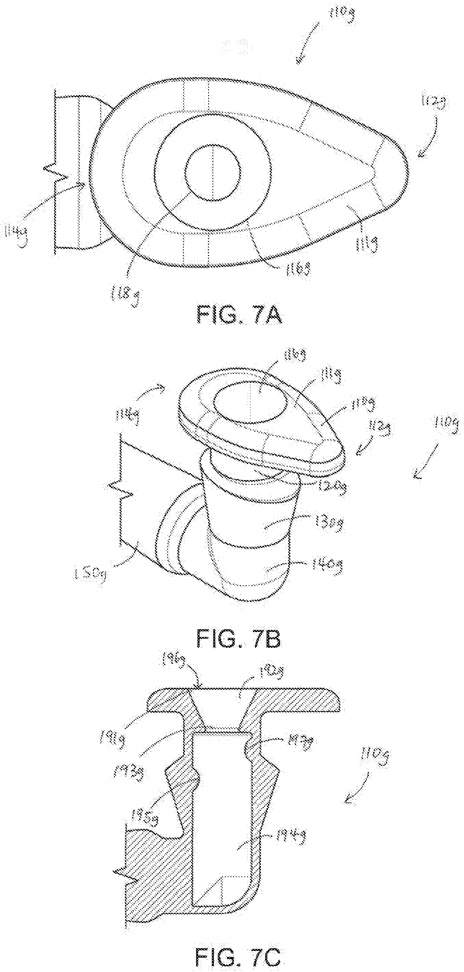

Figures 7A-7C illustrate an embodiment of a flange 110g and a drug load 190g of a proximal end of another embodiment of a flange 110g. Figures 7A-7B illustrate a proximal end of the punctal plug 100g. Figure 7A illustrates an embodiment of a flange 110g having a proximal end 112g and a distal end 114g. Figure 7B illustrates the proximal end of the punctal plug 100g having the flange 110g, neck 120g, retention portion 130g, corner 140g, and the punctum dilator 150g discussed previously. Turning first to the flange 110g, in some embodiments, the flange 110g includes a top surface 111g that includes an opening 116g. In some embodiments, the opening 116g is fluidly connected to a channel 118g. As noted above, the channel 118g is configured to extend at least through a portion of the proximal end of the flange 110g so as to allow drug from the drug load 190g to elute to the opening 116g. In some embodiments, the opening 116g is circular. In some embodiments, the opening 116g has a diameter that is greater than the diameter of the channel 118g. In some examples, the flange 110g has a teardrop shape. As shown, the flange 110g can have a pointed proximal end 112g and a curved distal end 114g. In some embodiments, the flange 110g is symmetrical along an axis that bisects the proximal end 112g and the distal end 114g of the flange 110g. In some examples, the distance between the distal end 114g of the flange 110g and a midpoint of the opening 116g can be approximately 0.025 inches.

Figure 7B illustrates a cross-sectional view of the flange 110g with a drug load 190g. As shown in Figure 7B, in some examples, the drug load 190g comprises a vertical section. The vertical section of the drug load 190g can include a top portion 192g having a proximal end 191g and a distal end 193g. As illustrated in Figure 7B, the top portion 192g can form a taper such that the diameter at the proximal end 191g is greater than the diameter at the distal end 193g. In some embodiments, the proximal end 191g of the top portion 192g can have a contact surface 196g. In some examples, the contact surface 196g can be configured to lay flush with the top surface 111g of the flange 110g. The contact surface 196g can be configured such that drug from the drug load 190g can elute from the contact surface 196g to contact the target location to deliver the drug. In some embodiments, the first body portion 194g can extend at a proximal end from a portion of the neck 120g to the corner 140g at a distal end. In some embodiments, the first body portion 194g can include a cut out 195g and a cut out 197g. In some examples, the cut out 197g is located on a first side of the first body portion 194g and is distal to the cut out 195g that is located on a second side of the first body portion 194g. In some embodiments, the drug load 190g can provide approximately 200 µg of API. In some examples, the drug load 190g can receive a volume of 320 nL.

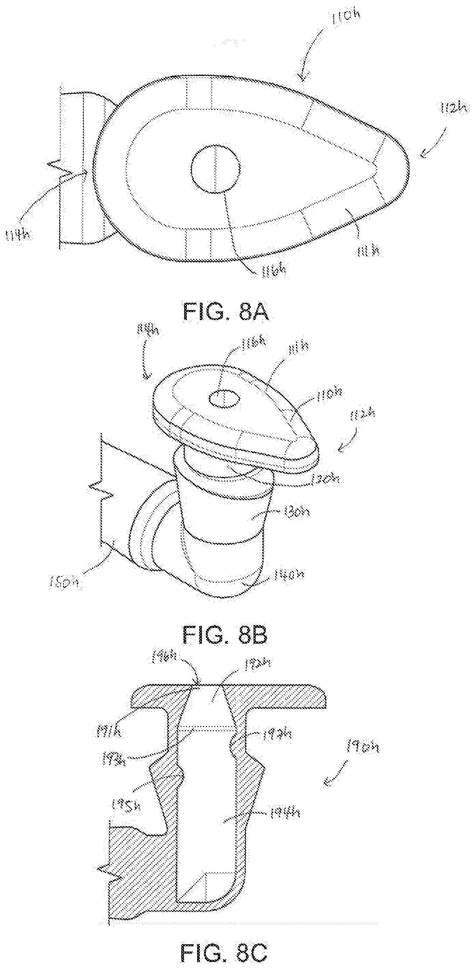

Figures 8A-8C illustrate an embodiment of a flange 110h and a drug load 190h of a proximal end of another embodiment of a punctal plug. Figures 8A-8B illustrate a proximal end of the punctal plug 100h. Figure 8A illustrates an embodiment of a flange 110h having a proximal end 112h and a distal end 114h. Figure 8B illustrates the proximal end of the punctal plug 100h having the flange 110h, neck 1120h, retention portion 130h, and the punctum dilator 150h discussed previously. Turning first to the flange 110h, in some embodiments the flange 110h includes a top surface 111h that includes an opening 116h. In some embodiments, as will be discussed in more detail below, the opening 116h is fluidly connected to a drug load within the punctal plug. In some embodiments, the opening 116h is circular. In some examples, the flange 110h has a teardrop shape. As shown, the flange 110h can have a pointed proximal end 112h and a curved distal end 114h. In some examples, the flange 110h is symmetrical along an axis that bisects the proximal end 112h and the distal end 114h. In some examples, the distance between the distal end 114h of the flange 110h and the midpoint of the opening 116h can be approximately 0.025 inches.

Figure 8B illustrates a cross-sectional view of the flange 110h with a drug load 190h. As shown in Figure 8B, in some examples, the drug load 190h comprises a vertical section. The vertical section of the drug load 190h can include a top portion 192h having a proximal end 191h and a distal end 193h. As illustrated, the distal end 193h can form a reverse taper such that the diameter at the proximal end 191h is less than the diameter at the distal end 193h. In some embodiments, the proximal end 191h of the top portion 192h can have a contact surface 196h. In some examples, the contact surface 196h can be configured to lay flush with the top surface 111h of the flange 110h. The contact surface 196h can be configured such that drug from the drug load 190h can elute from the contact surface 196h to contact the target location to deliver the drug. In some embodiments, the first body portion 194h can extend at a proximal end from a portion of the neck 120h to the corner 140h at a distal end. In some embodiments, the first body portion 194h can include a cut out 195h and a cut out 197h. In some examples, the cut out 197h is located on a first side of the first body portion 194h and is distal to the cut out 195h that is located on a second side of the first body portion 194h. In some embodiments, the drug load 190h can provide approximately 200 µg of API. In some examples, the drug load 190g can receive a volume of 320 nL.

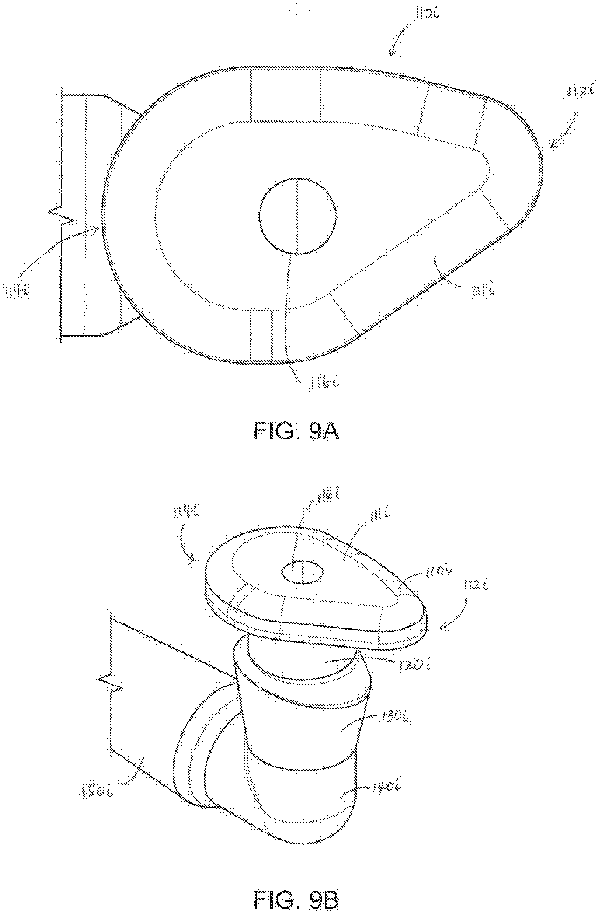

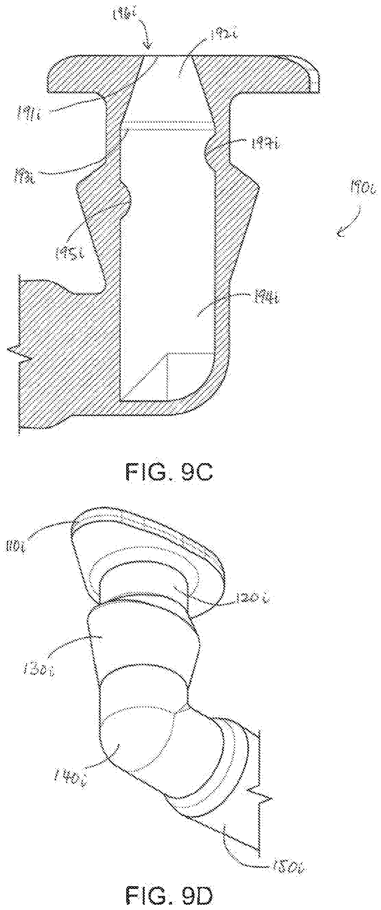

Figures 9A-9D illustrate an embodiment of a flange 110i and a drug load 190i of a proximal end of another embodiment of a punctal plug. Figures 9A-9C illustrate a proximal end of the punctal plug 100i. Figure 9A illustrates an embodiment of a flange 110i having a proximal end 112i and a distal end 114i. Figure 9B and 9D illustrate the proximal end of the punctal plug 100i having the flange 110i, neck 120i, retention portion 130i, corner 140i, and the punctum dilator 150i discussed previously. Turning first to the flange 110i, in some embodiments, the flange 110i includes a top surface 111i that includes an opening 116i. In some embodiments, the opening 116i is fluidly connected to a drug load within the punctal plug 100i. In some embodiments, the opening 116i is circular. In some examples, the flange 110i can have an asymmetrical teardrop shape. In some embodiments, the flange 110i has a pointed proximal end 112i and a curved distal end 114i. In some examples, the flange 110i has a straight portion extending along one side of the flange 110i between the proximal end 112i and the distal end 114i. In some examples, the flange 110i is asymmetrical along an axis that bisects the proximal end 112i and the distal end 114i. In some examples, the distance between the distal end 114i of the flange 110i and the midpoint of the opening 116i can be approximately 0.025 inches.

Figure 9C illustrates a cross-sectional view of the flange 110i with a drug load 190i. As shown in Figure 9C, in some examples, the drug load 190i comprises a vertical section. The vertical section of the drug load 190i can include a top portion 192i having a proximal end 191i and a distal end 193i. As illustrated, the distal end 193i can form a reverse taper such that the diameter at the proximal end 191i is less than the diameter at the distal end 193i. In some embodiments, the proximal end 191i of the top portion 192i can have a contact surface 196i. In some examples, the contact surface 196i can be configured to lay flush with the top surface 111i of the flange 110i. The contact surface 196i can be configured such that drug from the drug load 190i can elute from the contact surface 196i to contact the target location to deliver the drug. In some embodiments, the first body portion 194i can extend at a proximal end from a portion of the neck 120i to the corner 140i at a distal end. In some examples, the cut out 197i is located on a first side of the first body portion 194i and is distal to the cut out 195i that is located on a second side of the first body portion 194i. In some embodiments, the drug load 190h can provide approximately 200 µg of API. In some examples, the drug load 190g can receive a volume of 320 nL.

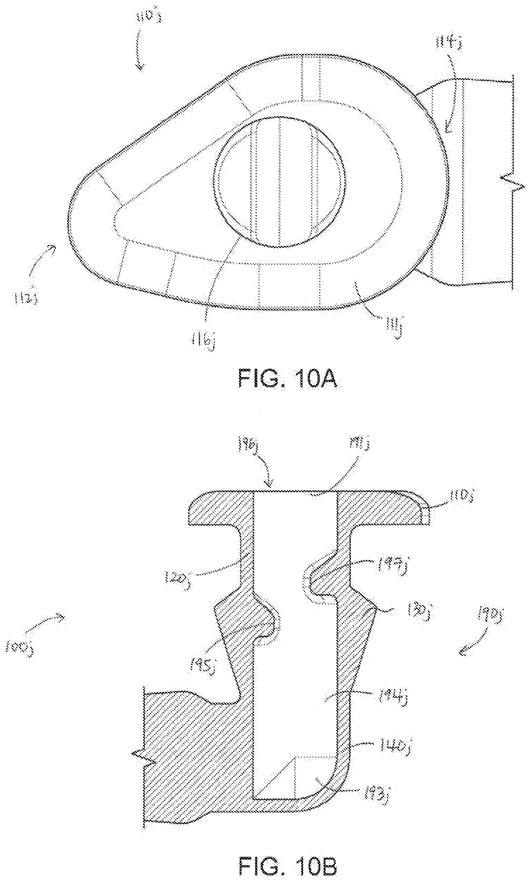

Figures 10A-10B illustrate an embodiment of a flange 110j and a drug load 190j of a proximal end of another embodiment of a punctal plug. Figure 10A illustrates an embodiment of a flange 110j having a proximal end 112j and a distal end 114j. In some embodiments, the flange 110j includes a top surface 111j that includes an opening 116j. In some embodiments, the opening 116j is fluidly connected to a drug load within the punctal plug. In some embodiments, the opening 116j is circular. In some examples, the flange 110j can have an asymmetrical teardrop shape. In some embodiments, the flange 110j has a pointed proximal end 112j and a curved distal end 114j. In some examples, the flange 110j has a straight portion extending along one side of the flange 110j between the proximal end 112j and the distal end 114j. In some examples, the flange 110j is asymmetrical along an axis that bisects the proximal end 112j and the distal end 114j. In some examples, the distance between the distal end 114j of the flange 110j and the midpoint of the opening 116j can be approximately 0.025 inches.

Figure 10B illustrates a cross-sectional view of the flange 110j with a drug load 190j. As shown in Figure 10B, in some examples, the drug load 190j comprises a vertical section. In some embodiments, the vertical section of the drug load 190j can comprise a first body portion 194j. In some examples, the first body portion 194j can include a proximal end 191j and a distal end 193j. As shown in Figure 10B, the proximal end 191j of the first body portion 194j can be located in the flange 110j of the punctal plug 100j while a distal end 193j of the first body portion 194j can be located in a portion of the corner 140j of the punctal plug 100j. In some embodiments, the proximal end 191j of the first body portion 194j can include a contact surface 196j. In some examples, the contact surface 196j can be configured to lay flush with the top surface 111j of the flange 110j. The contact surface 196j can be configured such that drug from the drug load 190j can directly contact the target location of the eye to deliver the drug from the drug load 190j. In some embodiments, the first body portion 194j can include a cut out 195j and a cut out 197j. In some examples, the cut out 195j is located on a first side of the first body portion 194j and is distal to the cut out 197j that is located on a second side of the first body portion 194j. In some embodiments, the drug load 190j can provide approximately 200 µg of API. In some examples, the drug load 190b can receive a volume of 320 nL.

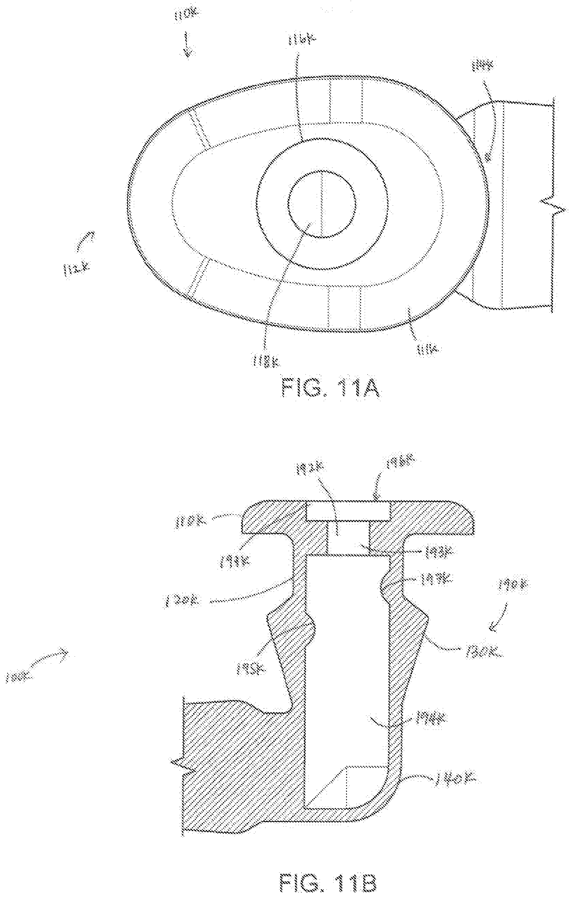

Figures 11A-11B illustrate an embodiment of a flange 110k and a drug load 190k of a proximal end of another embodiment of a punctal plug. Figure 11A illustrates an embodiment of a flange 110k having a proximal end 112k and a distal end 114k. Figure 11B illustrates the proximal end of the punctal plug 100k having the flange 110k, neck 120k, retention portion 130k, 140k, and the 150k discussed in more detail above. Turning first to the flange 110k, in some embodiments, the flange 110k includes a top surface 111k that includes an opening 116k. In some examples, the flange 110k is fluidly connected to a channel 118k. As noted above, the channel 118k is configured to extend at least through a portion of the proximal end of the flange 110k so as to allow drug from the drug load 190k to elute through the opening 116k. In some embodiments, the opening 116k is in circular. As illustrated in some embodiments, the flange 110k can have an oval or egg shape. In some examples, the flange 110k has a curved proximal end 112k and a curved distal end 114k. In some embodiments, the curved distal end 114k is wider than the curved proximal end 112k. In some embodiments, the sides of the flange 110k is curved on either side of the flange 110k. In some embodiments, the flange 110k is symmetrical along an axis that bisects the proximal end 112k and the distal end 114k of the flange 110k. In some embodiments, the distance between the distal end 114k of the flange 110k and a midpoint of the opening 116k can be approximately 0.025 inches.

Figure 11B illustrates a cross-sectional view of the flange 110k with a drug load 190k. As shown in Figure 11B, in some examples, the drug load 190k comprises a vertical section. The vertical section of the drug load 190k can include a top portion 192k having a proximal end 191k and a distal end 193k. As illustrated in Figure 11B, the top portion top portion 192k can form a first portion 198k at the proximal end 191k that has a greater diameter than a second portion at the distal end 193k that has a smaller diameter. In some embodiments, the proximal end 191k of the top portion 192k can have a contact surface 196k. In some examples, the contact surface 196k can be configured to lay flush with the top surface 111k of the flange 110k. The contact surface 196k can be configured such that drug from the drug load 190k can elute from the contact surface 196k to contact the target location to deliver the drug. In some embodiments, the first body portion 194k can extend at a proximal end from a portion of the neck 120k to the corner 140k at a distal end. In some embodiments, the first body portion 194k can include a cut out 195k and a cut out 197k. In some examples, the cut out 197k is located on a first side of the first body portion 194k and is distal to the cut out 195k that is located on a second side of the first body portion 194k. In some embodiments, the drug load 190k can provide approximately 200 µg of API. In some examples, the drug load 190k can receive a volume of 320 nL.

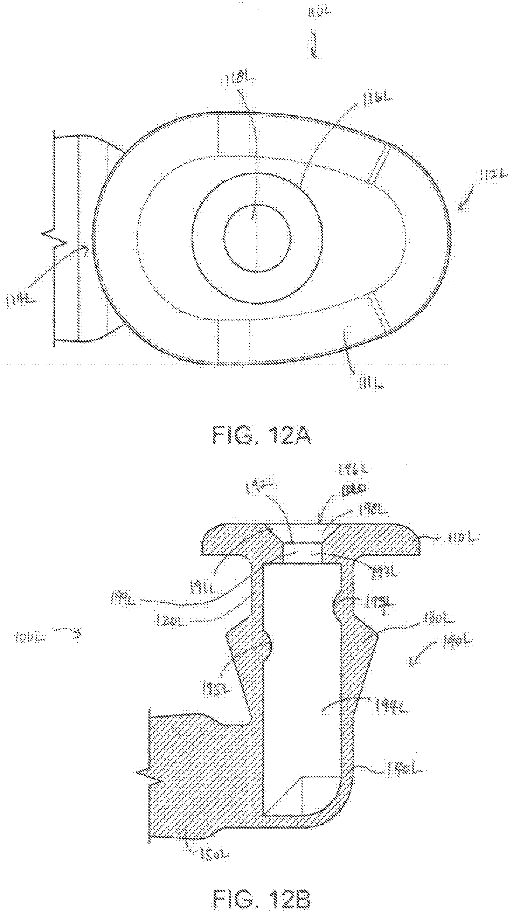

Figures 12A-12B illustrate an embodiment of a flange 110L and a drug load 190L of a proximal end of another embodiment of a punctal plug. Figure 12A illustrates an embodiment of a flange 110L having a proximal end 112L and a distal end 114L. Figure 12B illustrates the proximal end of the punctal plug 100L having the flange 110L, neck 120L, retention portion 130L, corner 140L, and the punctum dilator 150L discussed in more detail above. Turning first to the flange 110L, in some embodiments, the flange 110L includes a top surface 111L that includes an opening 116L. In some examples, the flange 110L is fluidly connected to a channel 118L. As noted above, the channel 118L is configured to extend at least through a portion of the proximal end of the flange 110L so as to allow drug from the drug load 190L to elute through the opening 116L. In some embodiments, the opening 116L is in circular. As illustrated in some embodiments, the flange 110L can have an oval or egg shape. In some examples, the flange 110L has a curved proximal end 112L and a curved distal end 114L. In some embodiments, the curved distal end 114L is wider than the curved proximal end 112L. In some embodiments, the sides of the flange 110L is curved on either side of the flange 110L. In some embodiments, the flange 110L is symmetrical along an axis that bisects the proximal end 112L and the distal end 114L of the flange 110L. In some embodiments, the distance between the distal end 114L of the flange 110L and a midpoint of the opening 116L can be approximately 0.025 inches.

Figure 12B illustrates a cross-sectional view of the flange 110L with a drug load 190L. As shown in Figure 12B, in some examples, the drug load 190L comprises a vertical section. The vertical section of the drug load 190L can include a top portion 192L having a proximal end 191L and a distal end 193L. As illustrated in Figure 12B, the top portion top portion 192L can form a first portion 198L at the proximal end 191L. In some embodiments, the first portion 198L at the proximal end 191L can form a taper wherein the diameter of the first portion 198L decreases in diameter as the first portion 198L at the proximal end 191L extends from the top surface 111L toward the neck 120L. In some examples, the top portion 192L can have a second portion at the distal end 193L. In some embodiments, the second portion at the distal end 193L of the top portion 192L has a diameter that is approximately the same as the smallest diameter of the first portion 198L at the proximal end 191L of the top portion 192L. In some embodiments, the proximal end 191L of the top portion 192L can have a contact surface 196L. In some examples, the contact surface 196L can be configured to lay flush with the top surface 111L of the flange 110L. The contact surface 196L can be configured such that drug from the drug load 190L can elute from the contact surface 196L to contact the target location to deliver the drug. In some embodiments, the first body portion 194L can extend at a proximal end from a portion of the neck 120L to the corner 140L at a distal end. In some embodiments, the first body portion 194L can include a cut out 195L and a cut out 197L. In some examples, the cut out 197L is located on a first side of the first body portion 194L and is distal to the cut out 195L that is located on a second side of the first body portion 194L. In some embodiments, the drug load 190L can provide approximately 200 µg of API. In some examples, the drug load 190L can receive a volume of 320 nL.

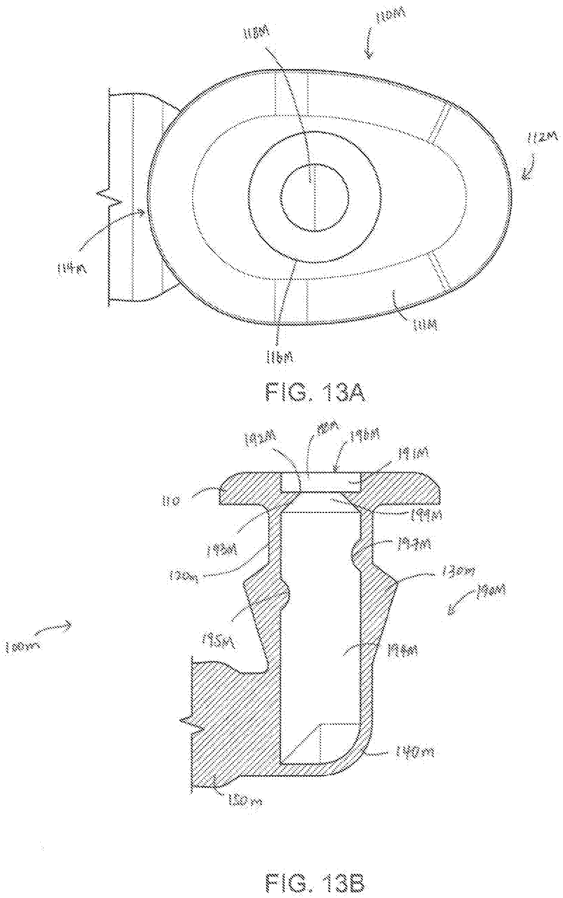

Figures 13A-13B illustrate an embodiment of a flange 110m and a drug load 190m of a proximal end of another embodiment of a punctal plug. Figure 13A illustrates an embodiment of a flange 110m having a proximal end 113m and a distal end 114m. Figure 13B illustrates the proximal end of the punctal plug 100m having the flange 110m, neck 120m, retention portion 130m, corner 140m, and the punctum dilator 150m discussed in more detail above. Turning first to the flange 110k, in some embodiments, the flange 110m includes a top surface 111m that includes an opening 116m. In some examples, the flange 110m is fluidly connected to a channel 118m. As noted above, the channel 118m is configured to extend at least through a portion of the proximal end of the flange 110m so as to allow drug from the drug load 190m to elute through the opening 116m. In some embodiments, the opening 116m is in circular. As illustrated in some embodiments, the flange 110m can have an oval or egg shape. In some examples, the flange 110m has a curved proximal end 113m and a curved distal end 114m. In some embodiments, the curved distal end 114m is wider than the curved proximal end 113m. In some embodiments, the sides of the flange 110m is curved on either side of the flange 110m. In some embodiments, the flange 110m is symmetrical along an axis that bisects the proximal end 113m and the distal end 114m of the flange 110m. In some embodiments, the distance between the distal end 114m of the flange 110m and a midpoint of the opening 116m can be approximately 0.025 inches.

Figure 13B illustrates a cross-sectional view of the flange 110m with a drug load 190m. As shown in Figure 13B, in some examples, the drug load 190m comprises a vertical section. The vertical section of the drug load 190m can include a top portion 192m having a proximal end 191m and a distal end 193m. As illustrated in Figure 13B, the top portion top portion 192m can form a first portion 198m at the proximal end 191m. In some embodiments, the first portion 198m at the proximal end 191m can form a cylinder in the flange 110. In some examples, the top portion 192m can have a second portion 199m at the distal end 193m. In some embodiments, the second portion 199m at the distal end 193m of the top portion 192m can form a reverse taper, wherein the diameter of the second portion 199m of the top portion 192m increases as the second portion 199m extends from the flange 110m towards the neck 120m. In some examples, the proximal most diameter of the second portion 199m is less than the diameter of the first portion 198m at the proximal end 191m of the top portion 192m. In some embodiments, the distal most diameter of the second portion 199m is approximately the same as the diameter of the first portion 198m at the proximal end proximal end 191m of the top portion 192m.

In some embodiments, the proximal end 191m of the top portion 192m can have a contact surface 196m. In some examples, the contact surface 196m can be configured to lay flush with the top surface 111m of the flange 110m. The contact surface 196m can be configured such that drug from the drug load 190m can elute from the contact surface 196m to contact the target location to deliver the drug. In some embodiments, the first body portion 194m can extend at a proximal end from a portion of the neck 130m to the corner 140m at a distal end. In some embodiments, the first body portion 194m can include a cut out 195m and a cut out 197m. In some examples, the cut out 197m is located on a first side of the first body portion 194m and is distal to the cut out 195m that is located on a second side of the first body portion 194m. In some embodiments, the drug load 190m can provide approximately 200 µg of API. In some examples, the drug load 190m can receive a volume of 320 nL.

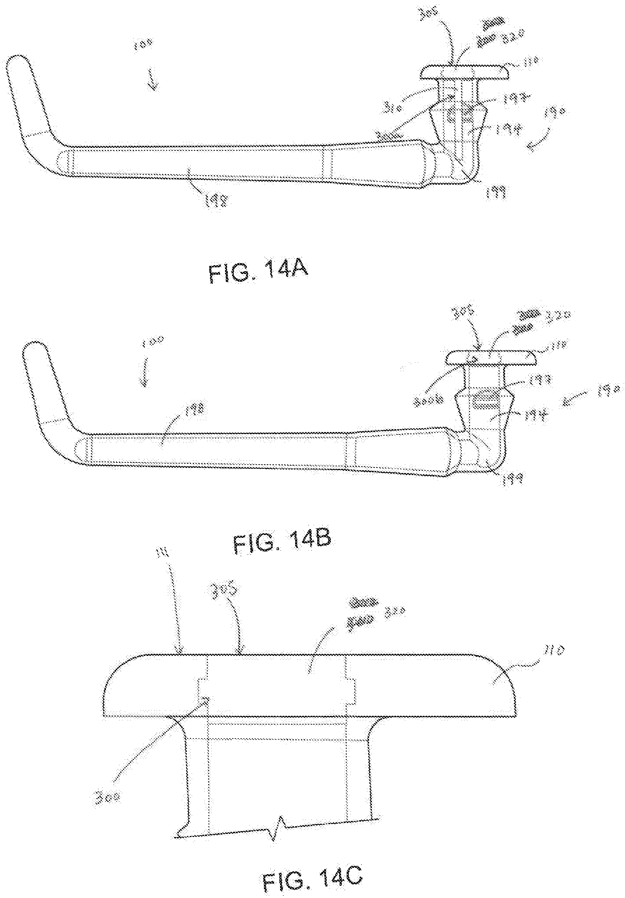

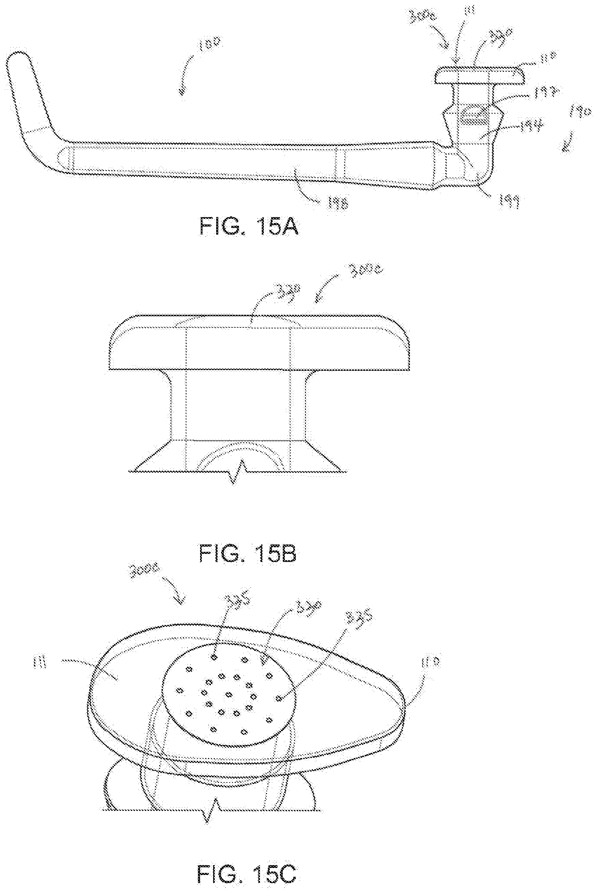

As discussed above, in some embodiments, the implant, such as the punctal plug, can be configured to provide drug delivery using an elution control member. Figures 14A-14C and 15A-15C illustrate a plurality of embodiments of elution control members configured to provide drug delivery to a target location using a punctal plug. Figures 14A illustrates an embodiment of an elution control member 300a. In some examples, the elution control member 300a can include a body 320 and an arm 310. In some embodiments, the body 320 is configured to be located in the flange 110 of the punctal plug 100. As illustrated in Figure 13C, in some embodiments the body 320 is positioned in the flange 110 such that the contact surface 305 is flush with the top surface 111 of the flange 110. In some examples, the body 320 can have features to promote retention of the body 320 in the flange 110 of the punctal plug 100. This can allow the contact surface 305 to contact the target ocular tissue. In some embodiments, the body 320 can be retained in the flange 110 of the punctal plug 100 in a variety of ways. For example, the body 320 can be molded into the flange 110, placed into molded geometry, glued in the opening 116 of the flange 110, etc. The arm 310 of the elution control member 300a can be configured to extend through the first body portion 194 of the drug load 190. In some examples, the arm 310 can be configured to improve the retention of the body 320 and the drug/polymer blend located in the drug load 190. As illustrated in Figure 14B, in some examples the elution control member 300b can include a body 320 without arm 310.

Figures 15A-15C illustrates another embodiment of the elution control member 300c. In some examples, the elution control member 300c can include a membrane 330. In some embodiments, the membrane 330 is located on the flange 110. In some examples, the membrane 330 is located on a top surface 111 of the flange 110. The membrane 330 can be thin and configured to contact the target ocular tissue. In some embodiments, the membrane 330 can include a plurality of holes 335. As shown in Figure 15C, the plurality of holes 335 can be in concentric circles, however this is not intended to be limiting. In some examples, the plurality of holes 335 can be in any configuration and each of the plurality of holes 335 can have any shape or size. In some embodiments, the membrane 330 can be retained on the flange 110 of the punctal plug 100 in a variety of ways. For example, the 333 can be glued, over molded, heat attached, etc. to the punctal plug 100.

### Drug Eluting Implant Having a Flexible Portion

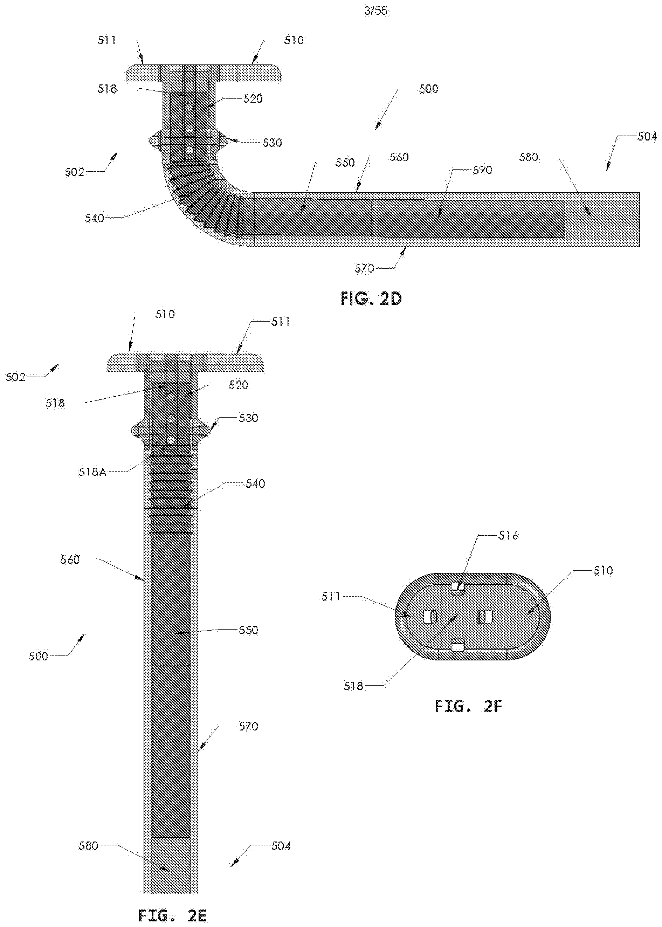

Figures 2D-2F illustrate an embodiment of a punctal plug 500 having a flexible region. In some embodiments, the punctal plug 500 can include a proximal end 502 and a distal end 504. In some examples, the proximal end 502 of the punctal plug 500 can include a flange 510, a neck 520, and a retention portion 530. In some examples, the punctal plug 500 can include a flex region 540 that is configured to allow the punctal plug 500 to form a "L-shaped" configuration.

As described above, in some embodiments, the punctal plug 500 can include a flange 510. In some examples, the flange 510 can have a low-profile and can be configured to have a variety of sizes. In some examples, the flange 510 can have a proximal end 502 and a distal end 504. As illustrated in Figure 2F, in some embodiments, the flange 510 can have a rounded portion at the proximal end 502 of the flange 510 and a rounded portion at the distal end 504 of the flange 510. However, this configuration is not intended to be limiting as the flange 510 can have any number of shapes and configurations. A number of potential configurations of the flange 510 are described in more detail below. In some embodiments, the flange 510 comprise a thermoplastic polymer or injection molded silicone. In some embodiments, the flange 510 comprises a non-permeable elastomer with perforations along fluted channels that allow the active ingredient, such as a pharmaceutical agent, of a drug matrix 590 to communicate with the tear film in the eye.

In some examples, the flange 510 can include a top surface 511 that can be configured to lie flush against a target tissue for treatment. Figure 2F illustrates a top view of the flange 510 of the punctal plug 500. In some embodiments, the top surface 511 can include an opening 516 that is fluidly connected to a channel 518 to provide a fluid connection between the interior of the punctal plug 500 and the opening 516 in the top surface 511. As illustrated in Figures 2D and 2E, the channel 518 can be configured to conform to the shape of the punctal plug 500. In some examples, the flange 510 of the punctal plug 500 can have a soft elastomeric feel to provide patient comfort while being designed to fit into the punctum region of the lacrimal duct and communicate chemically with the tear film of the eye.

In some examples, the punctal plug 500 can include a neck 520 and a retention portion 530. As illustrated in Figures 2D and 2E, the neck 520 can be attached to the flange 510 and the retention portion 530. In some embodiments, the diameter of the neck 520 can be less than either the diameter of the flange 510 or the retention portion 530. In some examples, the narrowed neck 520 can help to seat the proximal end 502 of the punctal plug 500 in the punctum of the patient such that the flange 510 is retained securely against a surface of the eye. In some embodiments, the shape of the retention portion 530 can help to reduce tissue stretch, such as in the punctum, which can reduce patient discomfort. As noted above, the differing diameters of the flange 510, neck 520, and the retention portion 530 can help to retain the punctal plug 500 in the punctum of the patient upon insertion

In some embodiments, the punctal plug 500 can include an interior element 550. In some examples, the interior element 550 can extend through the flange 510 and is configured to form a lumen having a hollow interior. In some embodiments, the interior element 550 can be axially flexible but strong radially. In some embodiments, element 550 has perforations or holes 518a which allows the API to transfer from the interior lumen via fluted channels 518 and out through the flange openings 516 to deliver API to the tear film. This can allow the interior element 550 to be non-collapsible and provide for structural robustness. In some examples, as illustrated in Figures 2D and 2E, the interior element 550 can be positioned in either a straight or bent manner. In some embodiments, the interior element 550 of the punctal plug 500 can be manufactured according to, but not limited to, the following techniques: extruded polymer tube with pleated section for kink-proof bending, cut nitinol tube with interlinking struts that form a mesh-like structure, or an electroform with bellows and or helices.

In some embodiments, the punctal plug 500 can include a fluted channel 518 that extends from the flange 510 to a location more distal than the perforations 518a in element 550. In some embodiments, the body 560 can contain a drug matrix 590 that includes one or more active ingredients, such as pharmaceutical agents, that are configured to treat ocular disease. In some embodiments, the drug matrix 590 is configured to treat dry eye and/or allergic conjunctivitis. In some examples, as illustrated in Figure 2D, the drug matrix 590 can be contained in the lumen of 550, extending into lumen of structure 570, and held in place by a structure 580. In some embodiments the structure 580 is located at the distal end 504, such as the tip, of the punctal plug 500. In some examples, the structure 580 is configured to be cured to seal the active ingredient, such as a pharmaceutical agent, of the drug matrix 590 within the punctal plug 500 and to prevent extrusion or leaking of the active ingredient of the drug matrix 590.

In some examples, the drug matrix 590 of the interior element 550 contains one or multiple pharmaceutical agents. In some embodiments, the pharmaceutical agents may treat any ocular disease, including but not limited to, dry eye and allergic conjunctivitis. In some examples, pharmaceutical agents may be currently known or unknown whether in structure or function or both. In some embodiments, the pharmaceutical agents may be incorporated within the punctal plug 500 as a homogeneous formulation or in combination with other features of the punctal plug 500. See section "Drugs" herein for additional detail.

In some examples, the punctal plug 500 can include an exterior 570. In some embodiments, the exterior 570 can include a polymer with permselective properties. In some embodiments, only the distal portion of the punctal plug 500 has the permselective exterior 570 disposed about the punctal plug 500. In some examples, the permselective exterior 570 is disposed about the drug matrix 590 located within the punctal plug 500. In some embodiments, the permselective polymer of the exterior 570 can be configured to allow for water to permeate through the polymer but prevent the component within the drug matrix 590, such as active and/or pharmaceutical ingredients, to permeate and be transported through the polymer exterior 570. This composition of the polymer exterior 570 can draw water into the formulation of the drug matrix 590 to create a concentrated solution thereof. In accordance with mass transport principles, in some embodiments, the active ingredients of the drug matrix 590 will move from the high-concentration distal region of the punctal plug 500 to the less-concentrated tear film of the eye via the holes 528a in element 550, the channel 518, and the flange holes 516. The polymer portions of the punctal plug 500 may be obtained using, but not limited to, one of the following processes: injection molding, extrusion, thermosetting, and monomer curing.

In some embodiments, the drug matrix 590 is configured to sustain delivery for a sufficient therapeutic interval. In some examples, this can be 6 months for some diseases. In some embodiments, the drug matrix 590 can include 1 mg or more of active ingredient.

In some examples, the punctal plug 500 can be processed using a number of different techniques. For example, the processing techniques may be, but not limited to the following: particle size manipulation, tableting, hot melt extrusion, encapsulation, phase or ionic change, liquid or paste homogenization, lyophilizing, solid dispersion, and/or emulsification.

In some embodiments, the punctal plug 500 can contain a plurality of active pharmaceutical ingredients ("API"). **In** some examples, the punctal plug 500 can be configured to contain two pharmaceutical agents providing an initial release of corticosteroid, such as loteprednol etabonate and sustained release of an immunosuppressant, such as cyclosporine A. In some embodiments, the flange 510 can include a plurality of holes 516. The plurality of holes can be formed in various ways including, for example, laser-cutting or molding. In some examples, the dual therapy described above can be accomplished using the plurality of holes 516 in the flange 510 in fluidic contact with a formulated corticosteroid tablet residing in the lumen of 550, communicating with the tear film through holes 518a in in element 550, the fluted channels 518, and the holes 516 in the flange. In some embodiments, the perm-selective exterior 570 provides a high surface area for the water from surrounding tissues to penetrate the tablet(s) and provide for solvation of the active ingredients. In some examples, solvation occurs for a duration of time suitable to prevent inflammation by the immunosuppressant, such as one to two weeks in duration. In some embodiments, water from surrounding tissues permeate exterior 570, causing a phase change of the formulation from liquid to solid. This could provide for a sustained release of the immunosuppressant for an extended time. In some embodiments, the immunosuppressant could be released for a duration of three to six months. In some examples, the phase change would preserve the physical structure of the device and help to prevent the tubing from collapse or the drug matrix from extruding.

In some examples, the corticosteroid can be formulated as a tablet and the immunosuppressant as a liquid to prevent unwanted mixing of active substances in the same phase. In some embodiments, the tablet(s) can be formulated with a solubilizing agent, such as hydroxypropyl beta-cyclodextrin, to an optimal elution rate, including about 5 - 10 µg/day. In some examples, the tablet(s) can contain no less than 50% active substance by weight. The immunosuppressant can be dissolved in a media, such as propylene glycol, at elevated temperatures and maintained as a true solution liquid formulation at room temperature. In some embodiments, the liquid formulation can be optimal for ease of filling the remaining open volume of the device, maximizing the drug load necessary for a sustained therapy or elution rate including at about 0.2 - 7.0 µg/day. In some examples, the immunosuppressant would contain no less than 65% active substance by weight.

### Kit for Drug Eluting Implant and Inserter

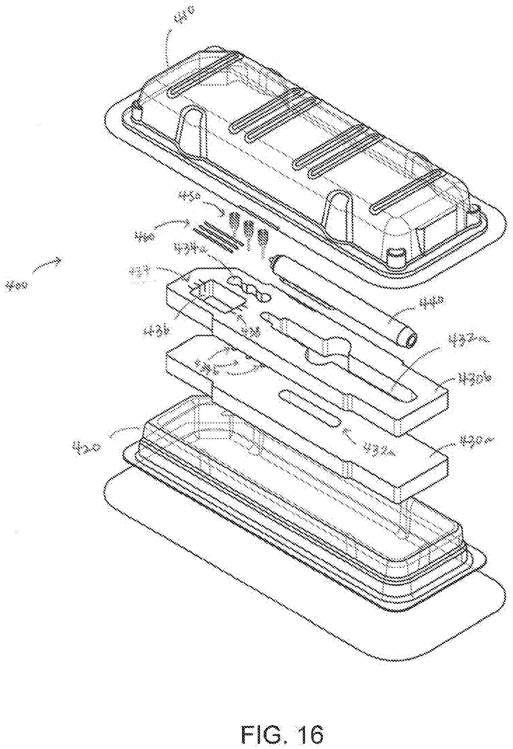

In some examples, the above described drug eluting implant, such as the punctal plug, can be easily inserted into a patient in a quick procedure. In some embodiments, the procedure can be conducted by the user, such as the physician, in an outpatient procedure. In order to provide the physician with easy and flexibility to insert the punctal plug, the punctal plug and the associated insertion device (to be described in more detail below) can be packaged and sent to the physician in a kit. An example embodiment of the kit 400 including at least one punctal plug and at least one insertion device is illustrated in Figure 16. In some embodiments, the kit 400 can be configured to include an inserter 440, at least one removable inserter tip 450, and at least one loader tube 460.

As will be described in more detail below, a punctal plug can be inserted into the patient using an inserter 440. In some embodiments, the inserter 440 of the kit 400 can be disposable. For example, the inserter 440 is a one-use device or a limited-use device. In some embodiments, a separate inserter 440 can be included in every kit 400. In other embodiments, the inserter 440 is reusable. In some examples, when the inserter 440 is reusable, a physician can have the option to purchase kits 400 that include an inserter 440 or do not include an inserter 440.

In some embodiments, the inserter 440 can be configured to engage with a removable inserter tip 450. In some embodiments, the removable inserter tip 450 is configured to load and position the punctal plug into a proximal end of the inserter 440. In some examples, the removable inserter tip 450 is disposable. In some embodiments, the removable inserter tip 450 is reusable.

The kit 400 can also include at least one loader tube 460. As will be described in more detail below, in some examples, the loader tube 460 is each loaded with a punctal plug. This can allow for easy loading of the punctal plug into the inserter 440 as well as ensuring the sterility of the punctal plug before insertion into the patient. In some examples, in order to insert a punctal plug into the punctum of the patient, the physician first attaches the removable inserter tip 450 to the proximal end of the inserter 440. The physician can then insert the loader tube 460 into the distal end of the removable inserter tip 450 so as to load the punctal plug into the inserter 440 and prepare the punctal plug for delivery. In some embodiments, the loader tube 460 is single-use such that each time an additional punctal plug is to be inserted, the physician must load the inserter 440 with an additional loader tube 460.

As illustrated in Figure 16, the kit 400 can include a hard case and a plurality of foam inserts to secure and protect the contents of the kit 400 as well as provide for easy shipping and transportation. In some embodiments, the kit 400 can include a lid 410 and a base 420. The lid 410 and the base 420 can be configured such that the lid 410 can be secured to the base 420. In some embodiments, the lid 410 and the base 420 can be sealable.

In some embodiments, the kit 400 can include a plurality of foam inserts - for example, foam insert 430a and a foam insert 430b. In some examples, the base 420 can receive the foam insert 430a and the foam insert 430b. In some embodiments, the foam insert 430a and the foam insert 430b is configured to receive and secure the inserter 440, removable inserter tip 450, and loader tube 460. In some examples, the foam insert 430b can include an opening 432a, an opening 434a, and an opening 436. In some embodiments, the opening 432a can be configured to receive the inserter 440. The opening 432a can include widened portions to allow for easy insertion and removal of the inserter 440 from the foam insert 430b. In some embodiments, the opening 434a can be configured to receive the at least one removable inserter tip 450. The opening 434a can be configured to provide for easy insertion and removal of the at least one removable inserter tip 450 from the foam insert 430b. In some embodiments, the opening 436 can be configured to include at least one pair of slots 437. In some examples, the at least one pair of slots 437 is configured to receive the at least one loader tube 460. As shown in Figure 16, each of the pair of slots 437 is configured to receive and secure a first and second end of the at least one loader tube 460. In some examples, each of the pair of slots 437 are sufficiently spaced apart in the opening 436 to allow for easy insertion and removal of the at least one loader tube 460 into the foam insert 430b.

As discussed above, in some embodiments, the base 420 is configured to receive the foam insert 430a and the foam insert 430b. In some embodiments, the foam insert 430a can also include a plurality of openings to receive portions of the components of the kit 400 that extend past the foam insert 430b. For example, as shown in Figure 16, the foam insert 430a can include an opening 432a and at least one opening 434b. In some embodiments, the opening 432a is configured to receive a portion of the inserter 440. In some examples, the at least one opening 434b is configured to receive a portion of the at least one removable inserter tip 450.

The configuration of the case for the kit 400 described above is not intended to be limiting. In particular, the number of and thickness of the foam inserts to be secured within the kit 400 can vary so long as the foam inserts help to secure and protect the contents of the kit 400.

### Insertion Device Overview

As described above, the embodiments of the drug eluting implants described above can be inserted into the patient by a physician by using an insertion device. In some embodiments, the insertion device can be designed for comfort and ease of use using a simple modular design. Two example insertion devices are described below. Various features of the below insertion device are provided to allow the punctal plug to be easily and comfortably inserted into the patient's punctum.

For example, in some embodiments, the punctal plug is loaded into a tip which can be configured to easily snap onto the body of the insertion device. In some examples, the loaded punctal plug has a distal end that can indicate insertion orientation. In some embodiments, the tip of the insertion device can be configured to be transparent to allow for easy visualization of the punctal plug location during insertion. In some examples, the insertion tip is configured to be 360° rotatable to allow for the most ergonomic orientation. This can allow for right or left handed insertion. In some embodiments, the trigger of the insertion device is configured to allow the physician to hold the insertion device near the insertion tip to enhance control over the procedure and delivery of the punctal plug into the patient's punctum.

### Insertion Device with a Push Mechanism

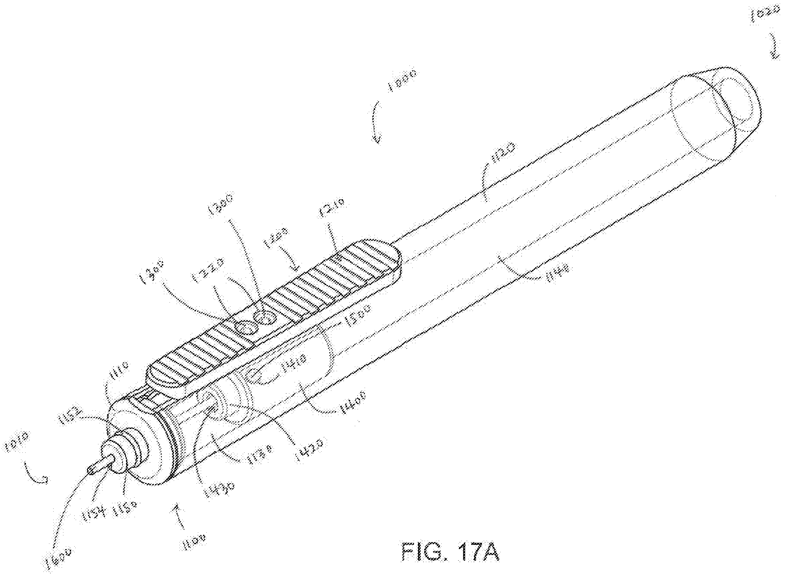

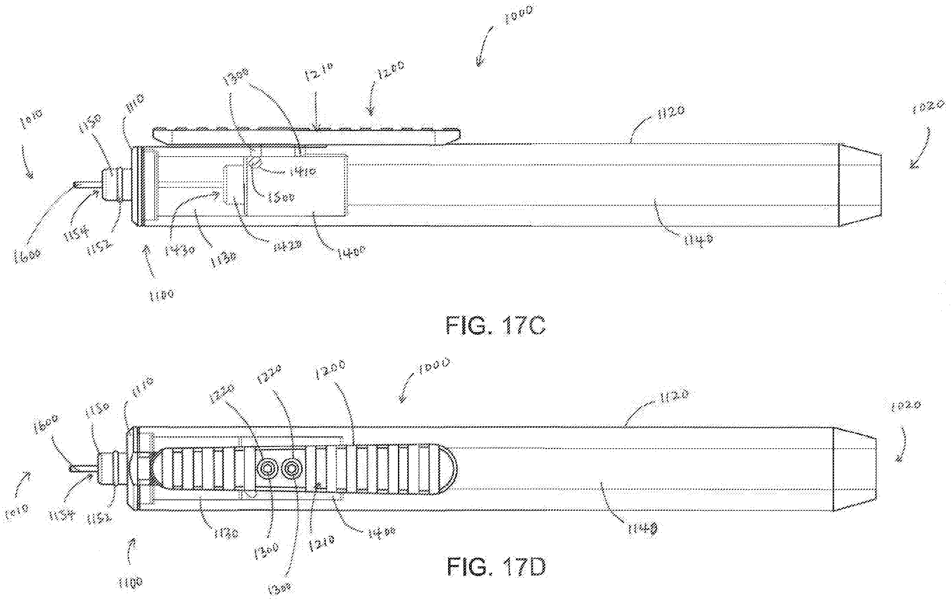

Figures 17A-17F illustrates an embodiment of an insertion device 1000. As will be described in more detail below, the insertion device 1000 including a push mechanism that allows for easy and quick delivery of the punctal plug using a simple push mechanism.

Figures 17A-17D illustrate a plurality of views of the insertion device 1000. The insertion device 1000 can have a proximal end 1010 and a distal end 1020. In some embodiments, the insertion device 1000 can include housing 1100, a trigger 1200, a slider 1400, and a pusher 1600.

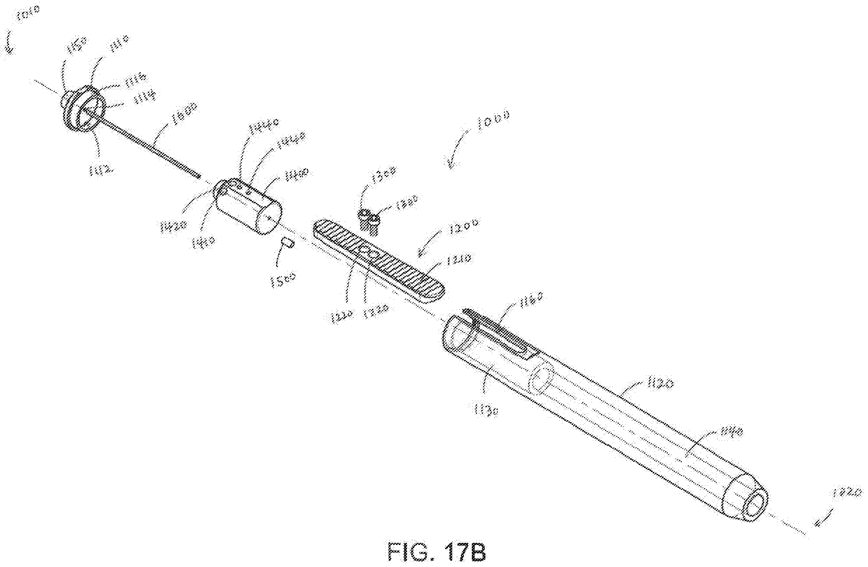

In some examples, the insertion device 1000 includes a housing 1100 that extends between the proximal end 1010 and the distal end 1020 of the insertion device 1000. In some embodiments, the housing 1100 can include a front portion 1110 and a rear portion 1120. In some examples, the front portion 1110 is configured to be secured within the proximal end of the rear portion 1120. As illustrated in Figure 17B, the front portion 1110 can include a shoulder 1116 that engages with the rear portion 1120. In some embodiments, the shoulder 1116 is configured to form a receiving portion 1112. As will be discussed in more detail below, the receiving portion 1112 is configured to receive a proximal end of the slider 1400. In some examples, the front portion 1110 includes an opening 1114 that is configured to allow at least a portion of the pusher 1600 to pass through the front portion 1110. As will be described in more detail below, the pusher 1600 is configured to push the punctal plug 100 out of the insertion device 1000. In some examples the front portion 1110 can include an engagement portion 1150. The engagement portion 1150 can include a snap ring 1152 disposed about a center portion of the engagement portion 1150. As will be discussed in more detail below, the snap ring 1152 of the engagement portion 1150 is configured to secure a removable inserter tip. In some embodiments, the engagement portion 1150 can include an opening 1154 that is aligned with the opening 1114 of the front portion 1110. Like the opening 1114, the opening 1154 is configured to allow at least a portion of the pusher 1600 to pass through the front portion 1110.

The rear portion 1120 of the front portion 1110 can include a proximal channel 1130 and a distal channel 1140 that extends through the body of the rear portion 1120. As will be discussed in more detail below, the slider 1400 can be disposed within the proximal channel 1130 and configured to move in a proximal-distal direction. In some embodiments, the diameter of the distal channel 1140 is less than the body of the proximal channel 1130. This can, for example, limit the distal movement of the slider 1400 within the proximal channel 1130. As illustrated in Figure 17B, in some examples, the rear portion 1120 can include an opening 1160 that extends along a proximal end of the rear portion 1120. In some embodiments, the length of the opening 1160 is less than the length of the proximal channel 1130 extending through the rear portion 1120. As will be discussed in more detail below, in some embodiments, the opening 1160 is configured to allow the trigger 1200 to move in a proximal-distal direction relative to the trigger 1200.

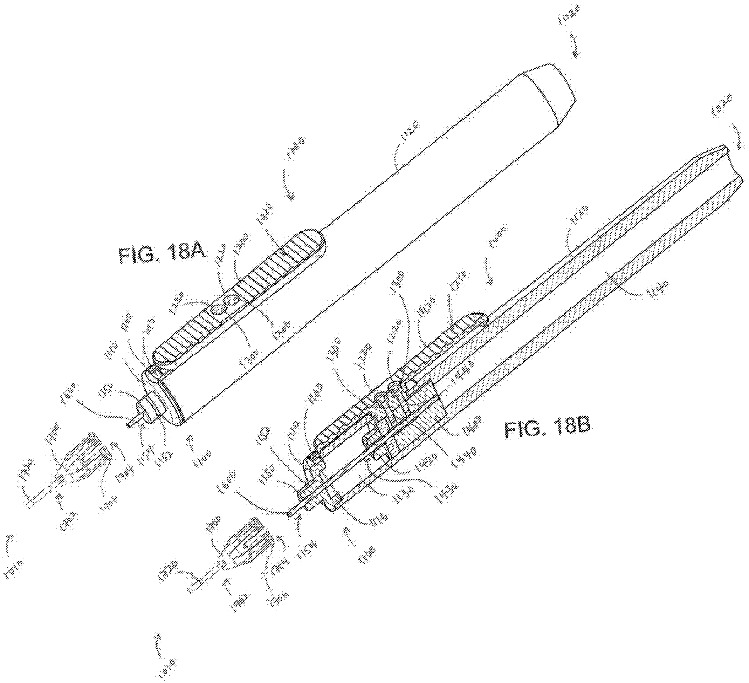

In some embodiments, the insertion device 1000 can include a slider 1400. The slider 1400 is configured to control movement of the pusher 1600 such that the punctal plug 100 can be delivered to the punctum of the patient in a controlled manner. As will be discussed in more detail below, the slider 1400 can be configured to be attached to a trigger 1200 that allows a user to control movement of the slider 1400 within the rear portion 1120. As discussed above, in some examples, the slider 1400 can be configured to move in a proximal-distal direction in the proximal channel 1130 of the rear portion 1120. As illustrated in Figure 17B, the slider 1400 can include a front portion 1420 on a proximal end of the slider 1400. In some embodiments, the front portion 1420 is cylindrical and can be configured to fit into the receiving portion 1112 of the front portion 1110. The front portion 1420 and the slider 1400 can include an opening 1430 that extends through the front portion 1420 and the entire length of the slider 1400 (illustrated in subsequent figures, like Figure 18B). In some embodiments, the pusher 1600 is secured within the opening 1430. In some examples (illustrated in subsequent figures, like Figure 18B), the distal end of the pusher 1600 does not extend through the distal end of the slider 1400. In some embodiments, the slider 1400 can include a plurality of openings 1440 on a top surface of the 1400. As will be discussed in more detail below, the openings 1440 can each be configure to receive a fastener to secure the trigger 1200 to the slider 1400. In some embodiments, as illustrated in Figure 17A and 17C, the slider 1400 can include a side opening 1410 that is configured to receive a friction bar 1500. In some embodiments, the friction bar 1500 comprises a compressible material and is configured to engage with an inner surface of the proximal channel 1130. In In some examples, the friction bar 1500 provides a small amount of friction such that the slider 1400 can move through the proximal channel 1130 in a controlled manner.

As shown in Figures 17A-17D, the insertion device 1000 can include a trigger 1200. As discussed above, the trigger 1200 is configured to allow the user to move the slider 1400 and the attached pusher 1600 in a proximal-distal direction through the proximal channel 1130. In some embodiments, the trigger 1200 can have a textured top surface 1210 that can provide the user with a better grip of the trigger 1200. In some examples, the trigger 1200 can include a plurality of openings 1220. In some embodiments, the plurality of openings 1220 can be located at a center portion of the trigger 1200. In some embodiments, each of the plurality of openings 1220 are configured to each receive one of a plurality of screws 1300. As illustrated in Figure 17A and 17C, the screws 1300 can extend through the openings 1220 of the trigger 1200 and engage the corresponding openings 1440 in the slider 1400. In some examples, the screws 1300 are configured to secure the trigger 1200 with the slider 1400. In some embodiments, the distal end of the screws 1300 can include external threading that are configured to engage the internal threading of each of the openings 1440.

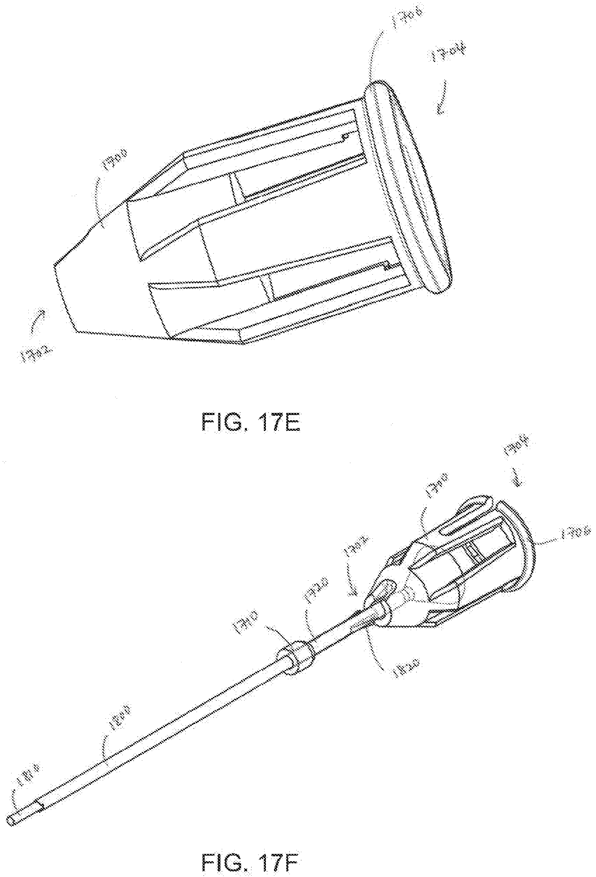

As described generally above, the insertion device 1000 can be configured to engage a removable inserter tip 1700. Figure 17E illustrates an embodiment of the inserter tip 1700. As shown, the inserter tip 1700 can include a proximal end 1702 and a distal end 1704. In some embodiments, the inserter tip 1700 includes a lip 1706 located at the distal end 1704 of the inserter tip 1700. As will be discussed below, the lip 1706 of the inserter tip 1700 is configured to allow the inserter tip 1700 to be removably attached to the proximal end 1010 of the insertion device 1000.

As discussed, the inserter tip 1700 is configured to allow the loading of the punctal plug into the insertion device 1000. Figure 17F illustrates an embodiment of the inserter tip 1700 engaged with the loader tube 1800 that can be pre-loaded with the punctal plug. As shown in Figure 17F, in some embodiments, the inserter tip 1700 can include an inserter tube 1720 that extends from the proximal end 1702 of the inserter tip 1700. In some examples, the inserter tube 1720 can include a cushion cup 1710 at the proximal end of the inserter tube 1720. As will be discussed in more detail below, the cushion cup 1710 is configured to provide the physician with a larger surface area to rest against the punctum to position the tip of the insertion device 1000 as the physician prepares to insert the punctal plug into the punctum. Figure 17F also illustrates the loader tube 1800 inserted through the inserter tube 1720 and into the proximal end 1702 of the inserter tip 1700. The loader tube 1800 can include a plurality of grabbers 1820 on a distal end of the loader tube 1800. The plurality of grabbers 1820 can be positioned such that it is adjacent to and closest to the proximal end 1702 of the inserter tip 1700. In some examples, the loader tube 1800 can include a loader pin 1810. The loader pin 1810 can be configured to extend through the loader tube 1800. Additional details regarding the loader pin 1810 and the loader tube 1800 will be provided in more detail below.

Figures 18A-18B illustrates a perspective and cross-sectional view of the insertion device 1000 that is configured to engage with the inserter tip 1700 and the attached inserter tube 1720. As shown, in some embodiments, the distal end 1704 of the inserter tip 1700 is configured to be removably attached to the proximal end 1010 of the insertion device 1000. In some embodiments, the distal end 1704 of the inserter tip 1700 includes an opening that is configured to engage with the engagement portion 1150 of the insertion device 1000. As discussed above, in some examples, the engagement portion 1150 includes a snap ring 1152 that is configured to engage with the lip 1706 of the distal end 1704 of the inserter tip 1700. In some embodiments, the pusher 1600 extending through the opening 1154 of the engagement portion 1150 is configured to extend through the inserter tip 1700 and the proximal end 1010 of the inserter tube 1720.

### Punctal Plug Delivery Using an Insertion Device with a Push Mechanism

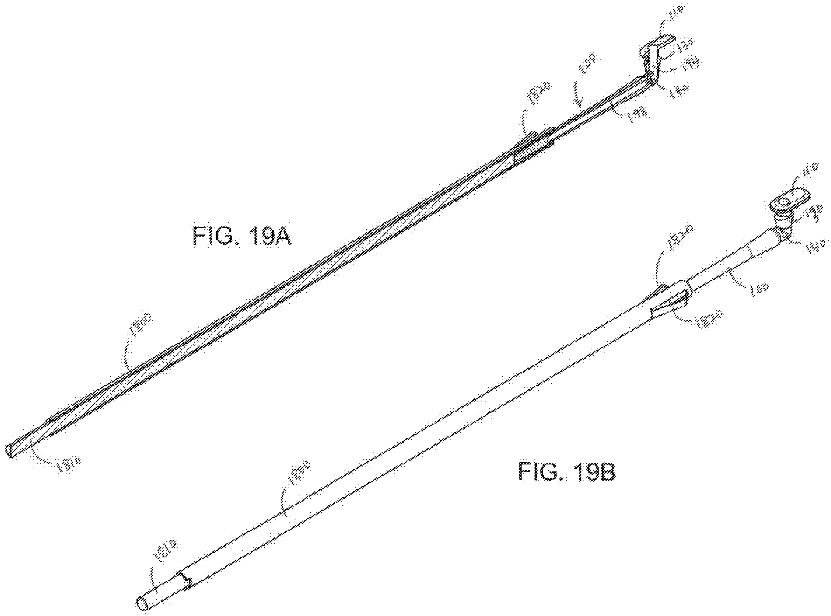

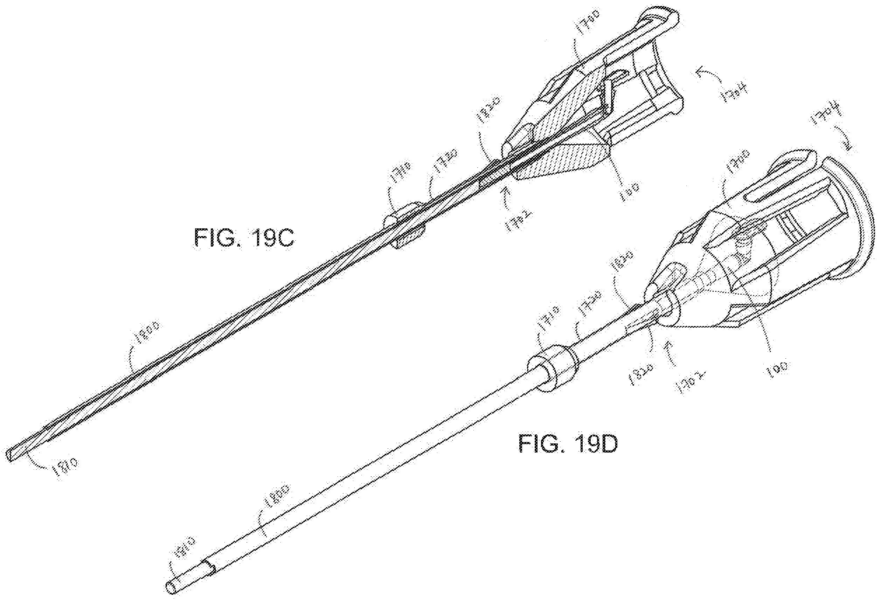

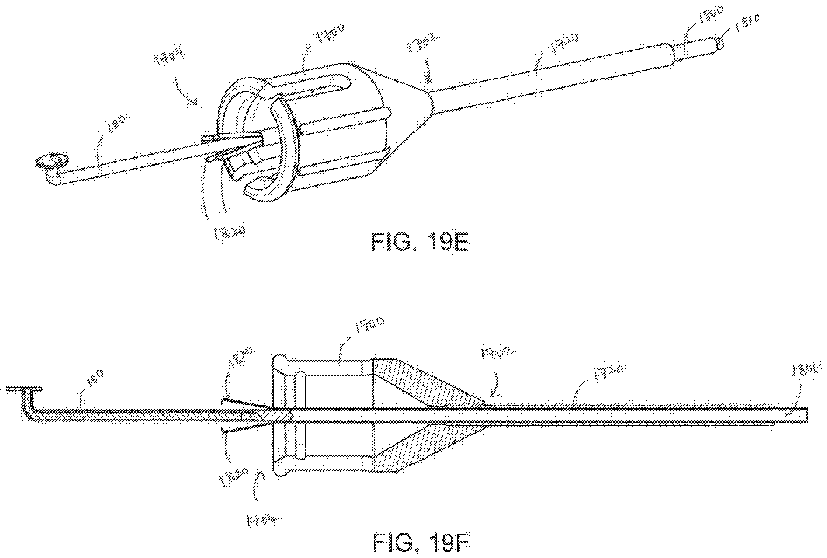

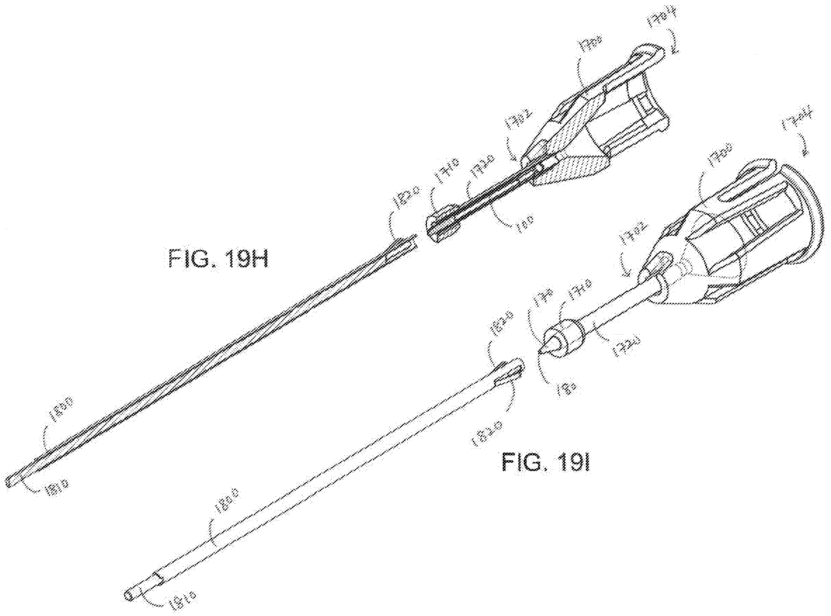

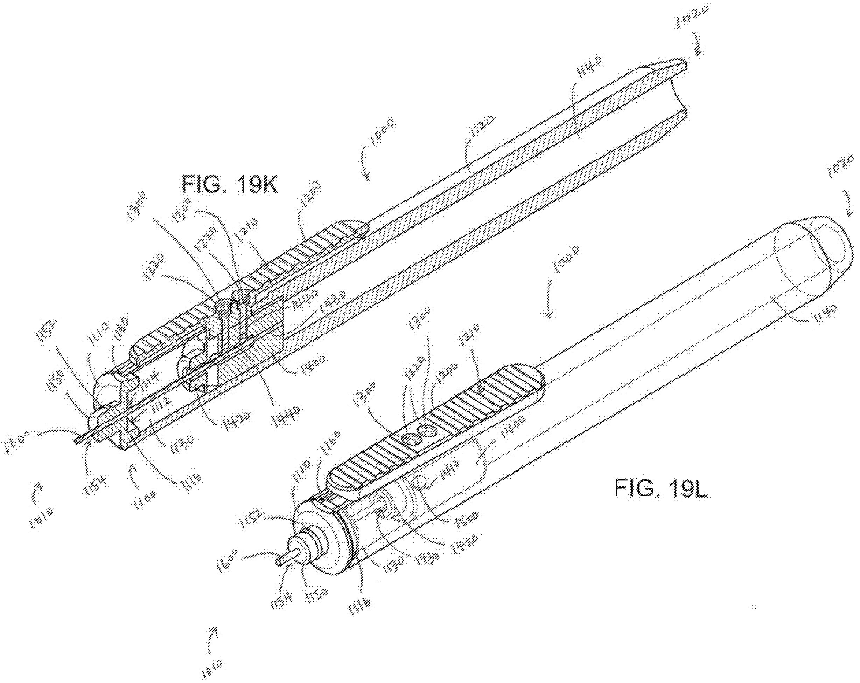

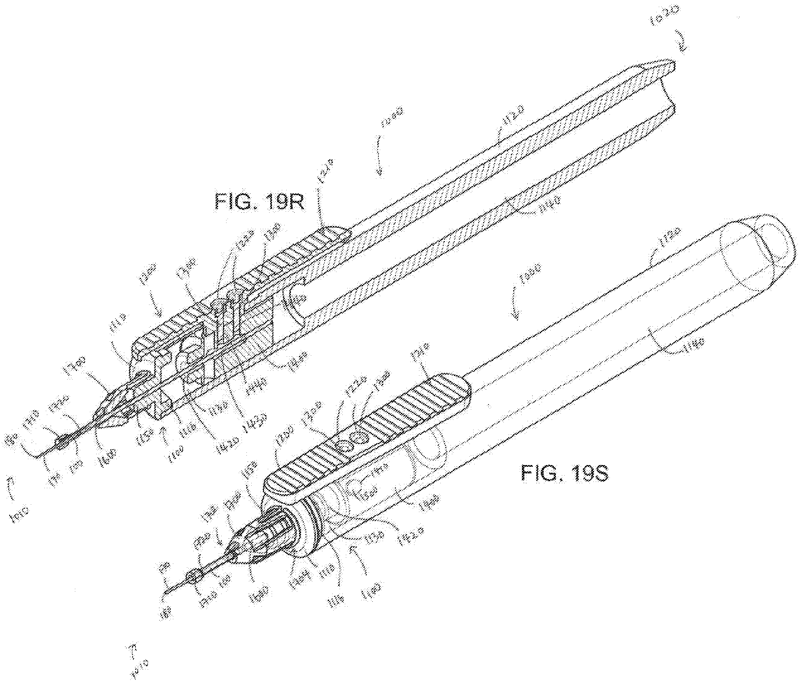

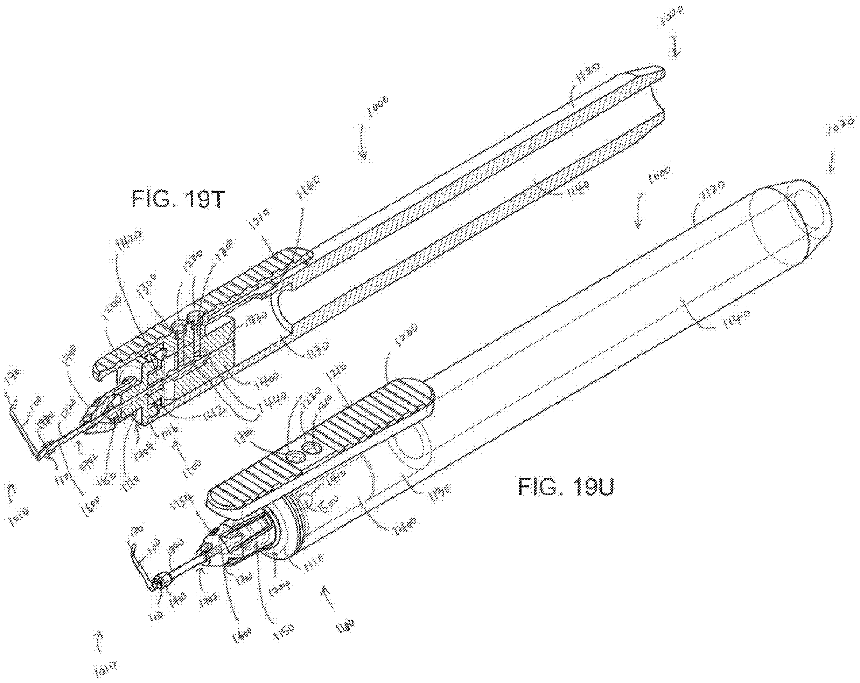

Figures 19A-19U illustrate a step-by-step schematic illustration of the loading and delivery of the punctal plug 100 into a punctum of a patient. Although the present method discusses the insertion device 1000 with regard to the delivery of a punctal plug 100 into the punctum, the above-referenced insertion device 1000 can be used to load and deliver a device into a patient, such as in other locations of the eye.

Figures 19A-19B illustrate a plurality of views of the punctal plug 100 being loaded into the distal end of the loader tube 1800. As shown in Figure 19A-19B, a distal end of the punctal plug 100, such as the retention portion 170 and the tip 180, are retained in a distal end of the loader tube 1800. As illustrated in Figure 19A, in some examples, the distal end of the punctal plug 100 is proximate to the grabbers 1820 of the loader tube 1800. In some embodiments, the loader pin 1810 extends through the loader tube 1800 such that a distal end of the loader pin 1810 is adjacent to the distal tip 180 of the punctal plug 100. In some examples, a proximal end of the loader pin 1810 extends out of a proximal end of the loader tube 1800. In some examples, the punctal plug 100 is loaded onto the distal end of the grabbers 1820 and shipped to the user in the kit 400 illustrated in Figure 16. As will be discussed below, each of the loader tubes 1800 can be inserted into a proximal end of the inserter tip 1700 and loaded into the insertion device 1000.

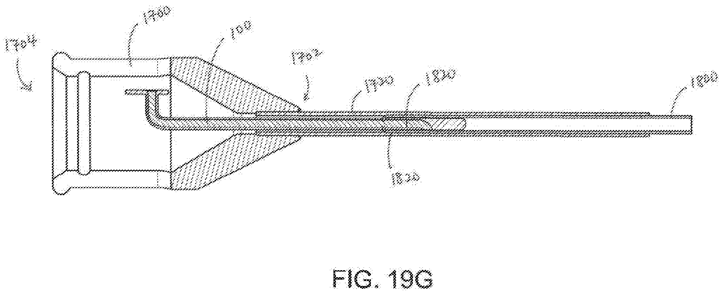

Figures 19C-19G illustrate a plurality of views of the punctal plug 100 loaded into the loader tube 1800 inserted into the proximal end 1010 of the inserter tip 1700. Figures 19C-19D illustrate cross-sectional and perspective views of the loader tube 1800 and the punctal plug 100 inserted into the proximal end of the inserter tip 1700. Figures 19E-19G illustrate a plurality of views of the position of the loader tube 1800 and the punctal plug 100 relative to the inserter tip 1700 and the inserter tube 1720 as the loader tube 1800 and the punctal plug 100 is pulled in a proximal direction through the inserter tip 1700. As shown in Figure 19E-19F, each of the plurality of grabbers 1820 on the distal end of the loader tube 1800 can have an expanded configuration when the grabbers 1820 of the loader tube 1800 is distal to the inserter tube 1720 within the inserter tip 1700. As the loader tube 1800 is pulled in a proximal direction through the inserter tube 1720, the distance between each of the plurality of grabbers 1820 is reduced as the grabbers 1820 is pulled through the inserter tube 1720. As illustrated in Figure 19G, the pronged ends of each of the grabbers 1820 grip onto the distal end of the punctal plug 100 and pull the punctal plug 100 into the inserter tube 1720 as the 1800.

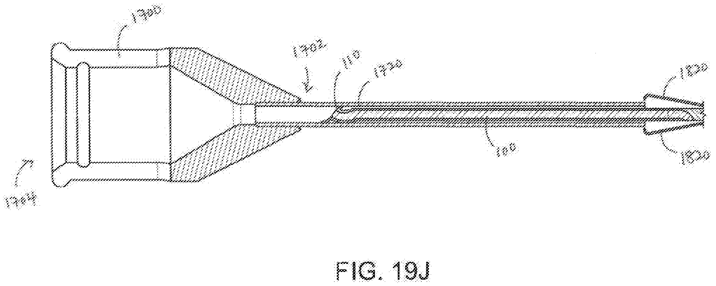

Figures 19H-19J illustrate a plurality of views of the punctal plug 100 as it is pulled into the inserter tube 1720 located at a proximal end 1702 of the inserter tip 1700. Figures 19H-19I illustrate the position of the loader tube 1800 after the punctal plug 100 is pulled through and removed from the distal end of the inserter tube 1720. As discussed above, each of the grabbers 1820 on the loader tube 1800 securely grips onto the distal end of the punctal plug 100 and pulls the punctal plug 100 in a proximal direction into and through the inserter tube 1720. As shown, when the loader tube 1800 is entirely removed from the inserter tube 1720, the retention portion 170 and tip 180 of the punctal plug 100 protrude from the cushion cup 1710 on the proximal end of the inserter tube 1720. Figure 19H illustrates a cross-sectional view of the punctal plug 100 in the inserter tube 1720. As shown, the "L-shaped" configuration of the punctal plug 100 can be flattened to fit through the inserter tube 1720. The straightening of the punctal plug 100 is illustrated in Figure 19J. Figure 19J illustrates a cross-sectional view of the inserter tip 1700 and inserter tube 1720 with the punctal plug 100 positioned within the inserter tube 1720 after it is loaded by the loader tube 1800. As shown, the flange 110 of the punctal plug 100 can straightened out to be in-line with the remainder of the body of the punctal plug 100.

Figures 19K-19L illustrate cross-sectional and perspective views of the insertion device 1000 prior to the engagement of the inserter tip 1700 to the proximal end of the punctal plug 100. As illustrated in Figures 19K-19L, the slider 1400 and the attached trigger 1200 are located at the distal-most position within the housing 1100 of the insertion device 1000. In some embodiments the distal-most surface of the slider 1400 abuts against a shoulder formed by the difference in diameter between the proximal channel 1130 and the distal channel 1140. The cross-section of Figure 19K illustrates the pusher 1600 extending through the slider 1400 and a proximal end of the housing 1100. In some examples, the distal end of the pusher 1600 is secured in the slider 1400. In some embodiments, in the configuration illustrated in Figures 19K-19L, a small portion of the proximal end of the pusher 1600 extends from the proximal end of the engagement portion 1150 through the opening 1154.

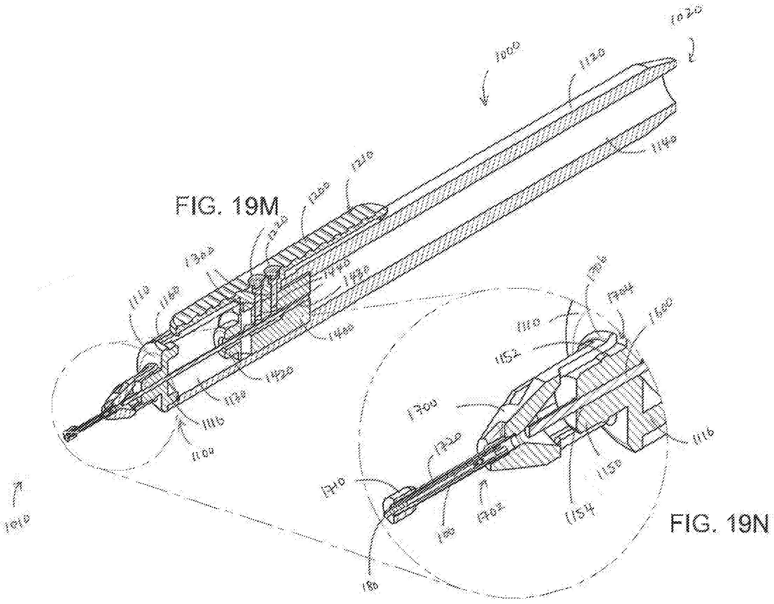

Figure 19M illustrates a cross-sectional view of the insertion device 1000 once the inserter tip 1700 is attached to the engagement portion 1150 on the proximal end 1010 of the insertion device 1000. Figure 19N illustrates an enlarged view of the proximal end 1010 of the insertion device 1000 with the inserter tip 1700 engaged to the engagement portion 1150 on the proximal end 1010 of the insertion device 1000. As shown in Figure 19N, the punctal plug 100 is positioned within the inserter tube 1720 and a proximal end of the pusher 1600 is located adjacent to a distal end of the punctal plug 100, such as adjacent to the flange 110 of the punctal plug 100. In some embodiments, the inserter tip 1700 includes a lip 1706 at a distal end 1704 of the inserter tip 1700. The lip 1706 can be configured to engage the snap ring 1152 of the engagement portion 1150 such that the lip 1706 is distal to the engagement portion 1150. In some embodiments, the inserter tip 1700 is removably attached to the engagement portion 1150 of the housing 1100. As discussed above, the inserter tip 1700 can be included in the kit 400 illustrated in Figure 16A. In some examples, the kit 400 can include a plurality of inserter tips 1700 such that the insertion device 1000 can be reused with each of the inserter tips 1700. In some embodiments, the inserter tip 1700 is engaged with the engagement portion 1150 of the insertion device 1000 to allow for 360° rotation of the inserter tip 1700. This can allow the physician to use the insertion device 1000 with the most ergonomic orientation as the insertion device 1000 is configured for either right or left hand insertion.

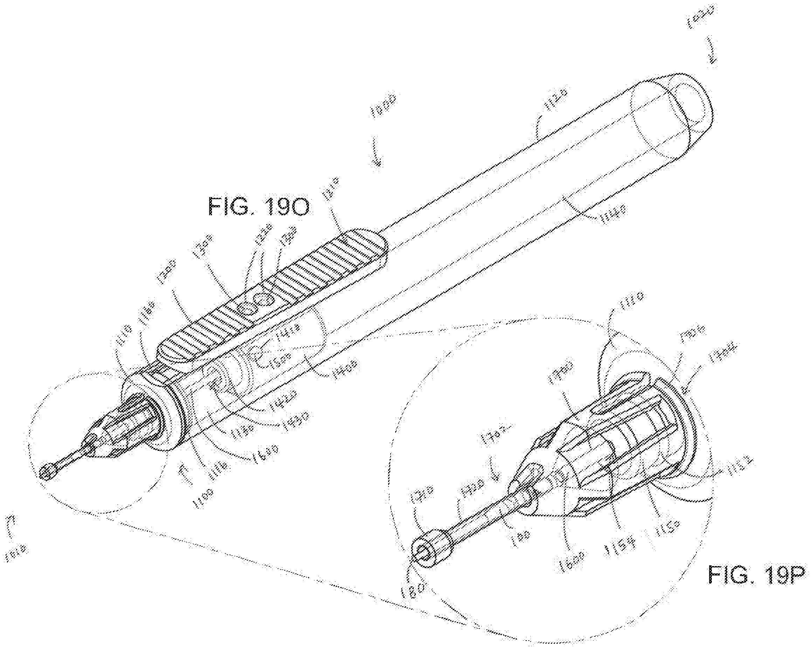

Figures 19O-19P illustrates additional perspective views similar to Figures 19M-19N. Figure 19O illustrates a perspective view of the insertion device 1000 with the inserter tip 1700 attached to the engagement portion 1150 on the proximal end 1010 of the insertion device 1000. Figure 19P illustrates an enlarged view of the proximal end 1010 of the insertion device 1000 with the inserter tip 1700 engaged to the engagement portion 1150 on the proximal end 1010 of the insertion device 1000. As shown, the lip 1706 of the inserter tip 1700 is configured to push past the snap ring 1152 of the engagement portion 1150 and secure the inserter tip 1700 to the engagement portion 1150 of the insertion device 1000.

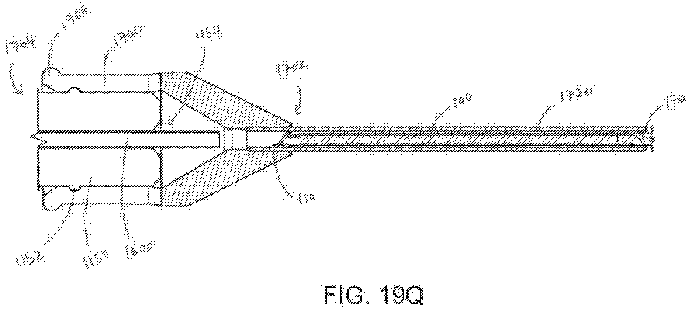

Figure 19Q illustrates a cross-sectional view of the inserter tip 1700 and the inserter tube 1720 with the punctal plug 100 positioned within the inserter tube 1720 after the inserter tip 1700 is engaged to the engagement portion 1150 on the proximal end 1010 of the insertion device 1000. In comparison with the cross-sectional view of the inserter tip 1700 in Figure 19O, the inserter tip 1700 is positioned over the engagement portion 1150. As shown, the pusher 1600 extends through the opening 1154 of the engagement portion 1150 such that the proximal end of the pusher 1600 is positioned adjacent to the distal end, such as the flange 110, of the punctal plug 100. As discussed above, the lip 1706 of the inserter tip 1700 can be configured to engage the snap ring 1152 of the engagement portion 1150 such that the lip 1706 is distal to the engagement portion 1150. In some embodiments, the inserter tip 1700 is configured to be removably attached to the engagement portion 1150 of the housing 1100.

Figures 19R-19S illustrates a cross-sectional and perspective view of the insertion device 1000 as the trigger 1200 is advanced forward within the proximal channel 1130 of the insertion device 1000. In some embodiments, as the trigger 1200 is advanced in a proximal direction towards the proximal end 1010 of the insertion device 1000, the attached slider 1400 is also moved in a proximal direction in the proximal channel 1130. As the slider 1400 is advanced, the proximal end of the pusher 1600 can be moved into the inserter tube 1720 and push the punctal plug 100 in a proximal direction. In Figures 19R-19S, the punctal plug 100 further extends from the proximal end 1010 of the inserter tube 1720. As illustrated, as the trigger 1200 advances the slider 1400 to a mid-point of the proximal channel 1130, the retention portion 170 and tip 180 of the punctal plug 100 is pushed proximally to protrude from the inserter tube 1720. In some examples, during insertion, the cushion cup 1710 is placed adjacent to the punctum of the patient and the tip 180 and retention portion 170 are advanced into the punctum of the patient.

Figures 19T-19U illustrates a cross-sectional and perspective view of the insertion device 1000 as the trigger 1200 is advanced forward until the slider 1400 within the proximal channel 1130 is moved into the proximal-most position such that the punctal plug 100 is delivered. In some examples, as the trigger 1200 is continually advanced in a proximal direction relative to the housing 1100, the attached slider 1400 can be advanced until the front portion 1420 of the slider 1400 is received in the receiving portion 1112 of the front portion 1110. In some embodiments, the trigger 1200 can no longer be advanced in a proximal direction when the proximal end of the front portion 1420 engages the inner surface of the receiving portion 1112. When the proximal end of the front portion 1420 is fully advanced into the receiving portion 1112, the proximal end of the pusher 1600 will have moved through the inserter tube 1720 to fully push the punctal plug 100 out of the inserter tube 1720. In some examples, during insertion, when the user has advanced the trigger 1200 to the proximal-most position, the punctal plug 100 will be delivered into the punctum of the patient.

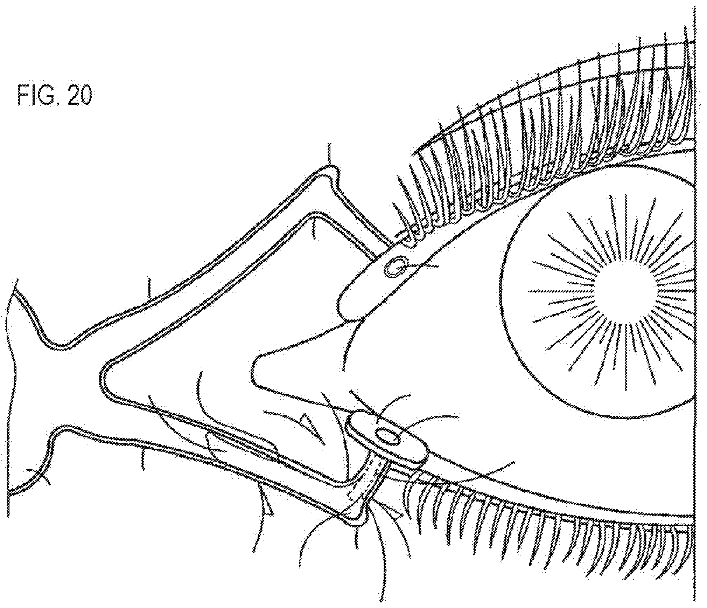

Figure 20 illustrates a schematic view of an enlarged view of the anatomy of a patient's eye and the insertion of the punctal plug 100 in the punctum of the patient's eye. As discussed above, in some embodiments, the flange 110 of the punctal plug 100 is adjacent to the target ocular tissue such that drug from the punctal plug 100 can contact and treat the target tissue.

### Insertion Device with a Retractable Mechanism

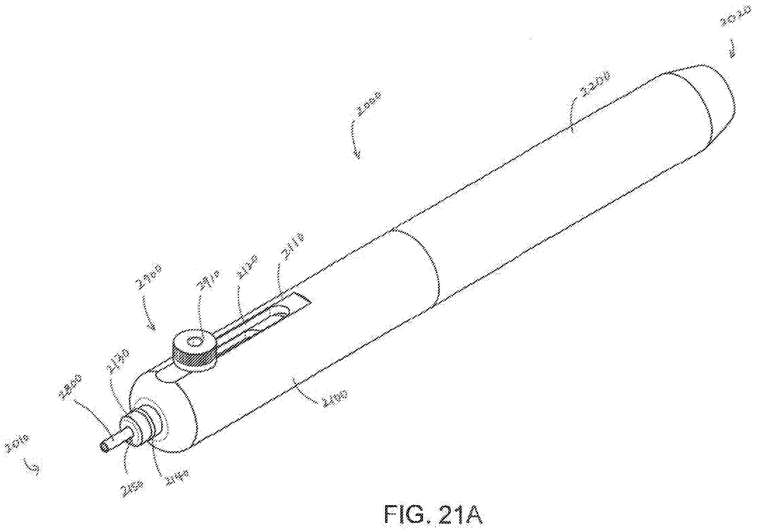

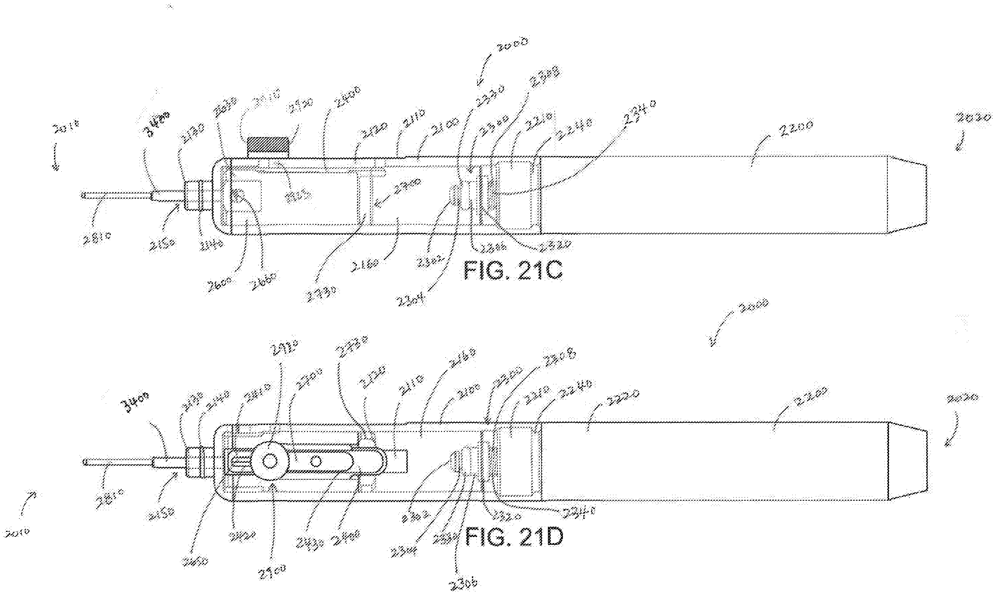

Figures 21A-21F illustrates an embodiment of an insertion device 2000. As will be described in more detail below, the insertion device 2000 including a retractable mechanism that allows for easy and quick delivery of the punctal plug. In addition to advancing the punctal plug 100 in a forward direction, the insertion device 2000 is configured to advance the punctal plug 100 in a proximal and distal direction.

Figures 21A-21D illustrate a plurality of views of the insertion device 2000. In some embodiments, the insertion device 2000 can have a proximal end 2010 and a distal end 2020. In some embodiments, the insertion device 2000 can include a housing 2100, a stem 2200, a slide stop pin 2300, an insert 2400, a mover 2500, shoulder screw 2900, and a pusher pin 2810.

In some examples, the insertion device 2000 includes a housing 2100 at a proximal end 2010 of the insertion device 2000 and a stem 2200 at a distal end 2020 of the insertion device 2000. As illustrated in Figures 21C-21D, in some examples, the distal end of the housing 2100 can be configured to engage a proximal end of the stem 2200. In some embodiments, the stem 2200 includes a waist 2240 formed between the proximal portion 2210 and the distal portion 2220 that is configured to receive an engagement lip 2170 of the housing 2100.

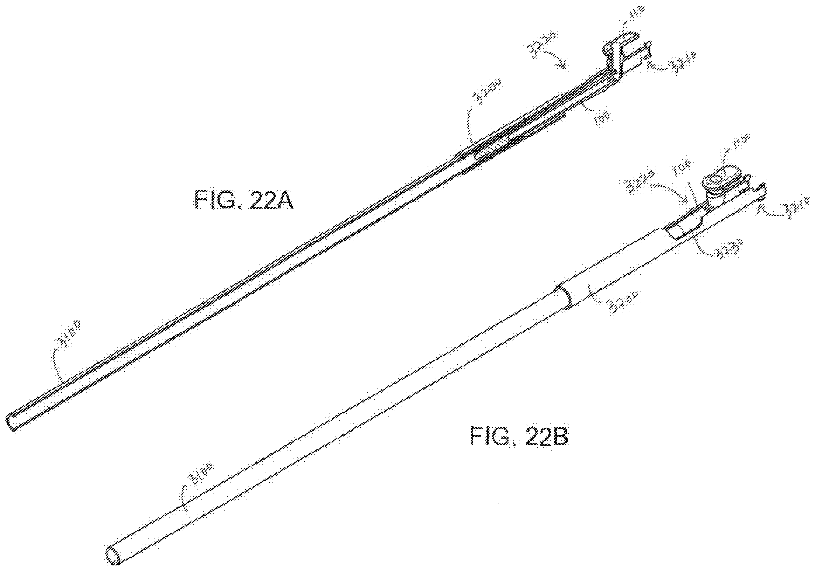

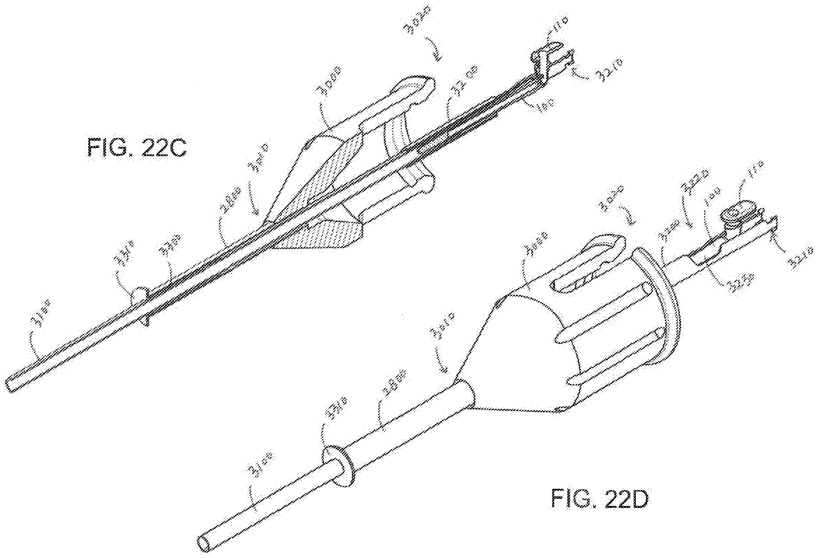

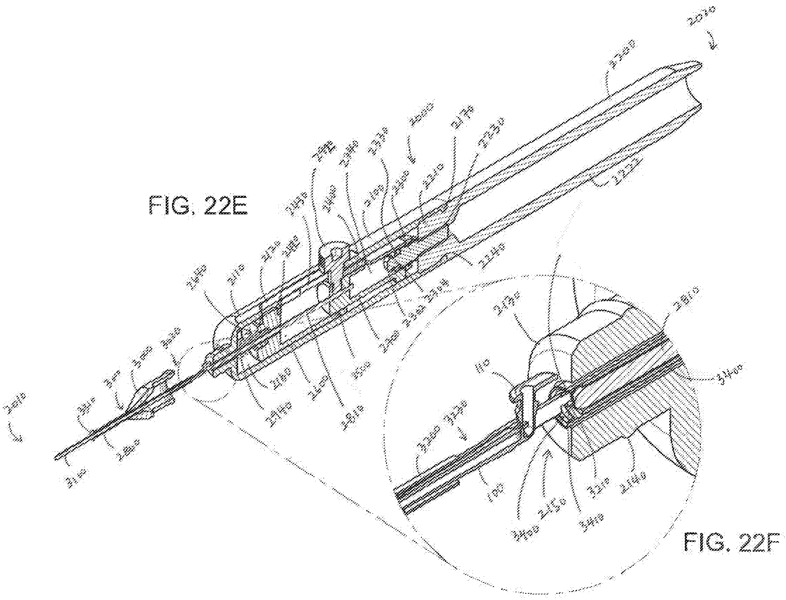

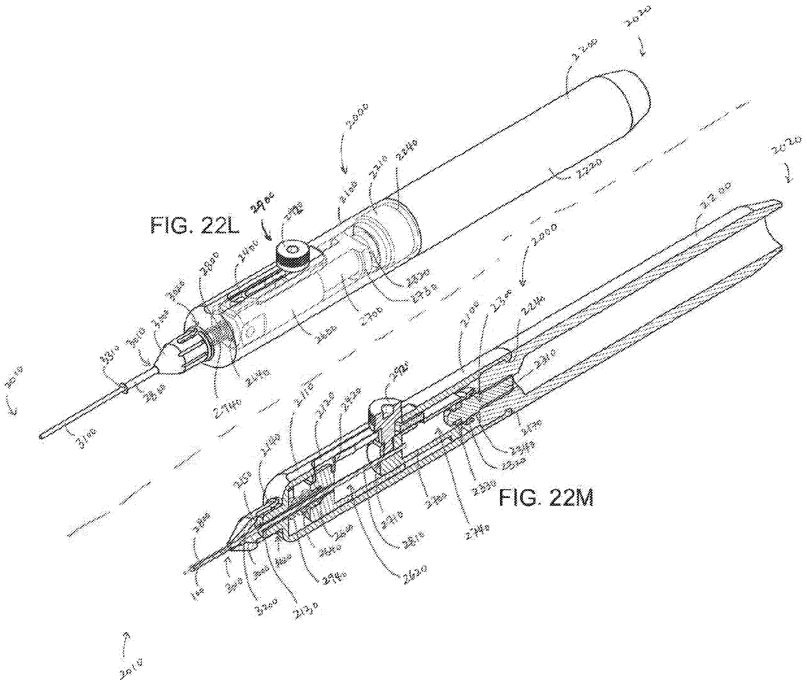

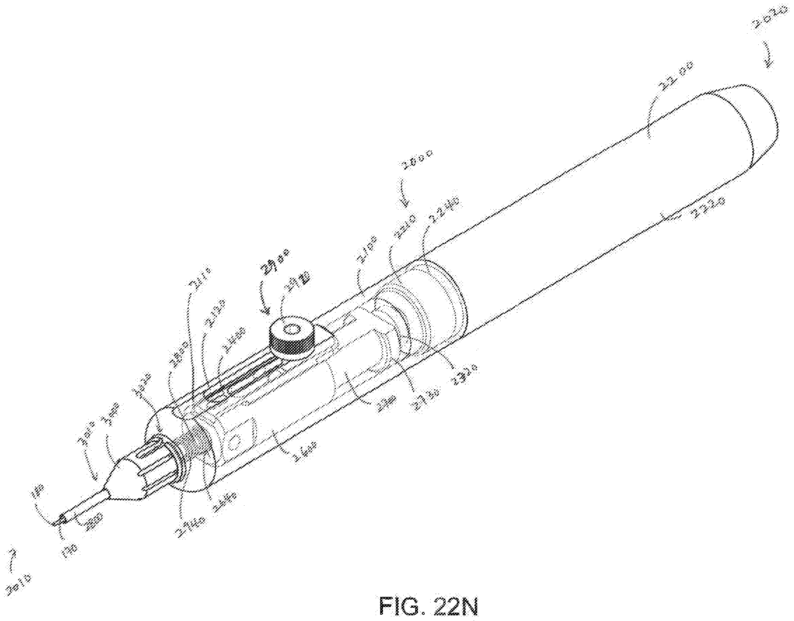

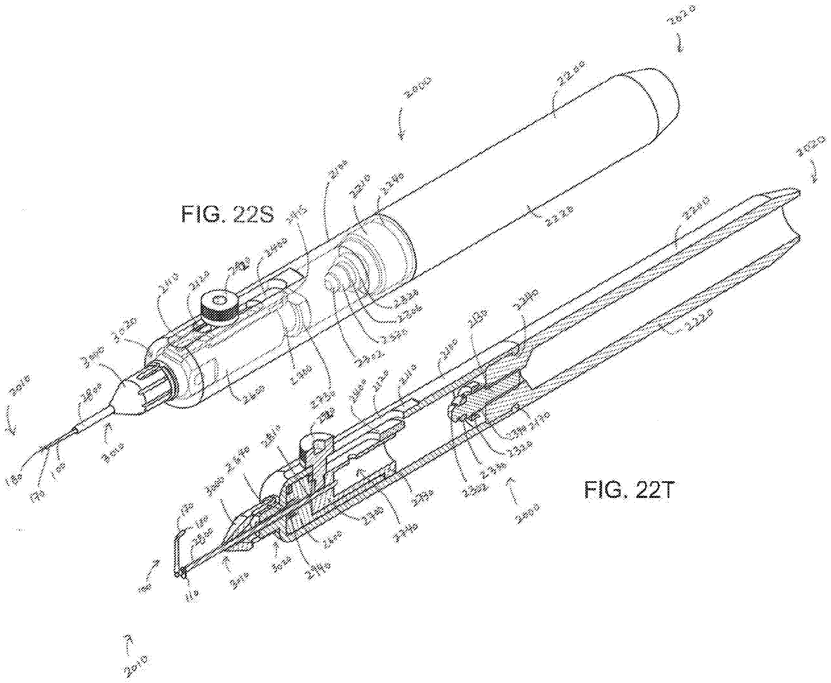

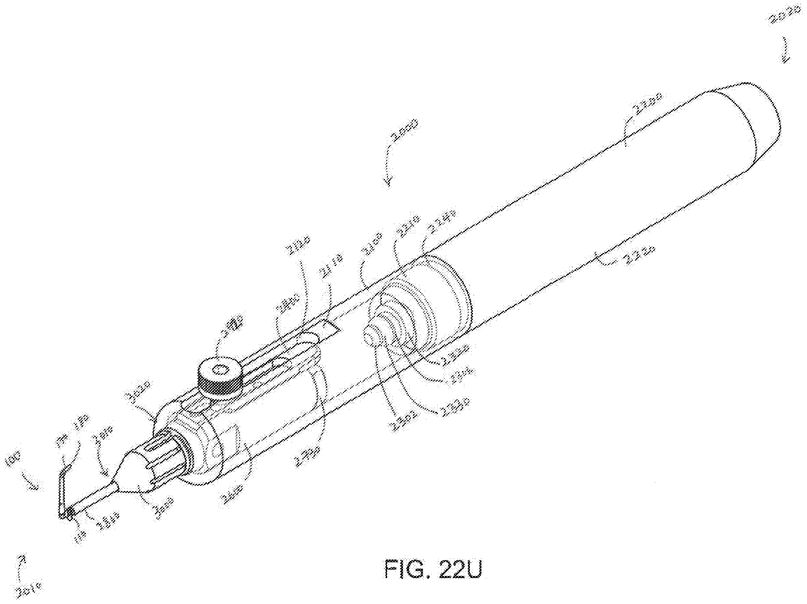

Turning first to the housing 2100, in some embodiments, the housing 2100 includes a top surface that includes an opening 2120. In some examples, the opening 2120 is configured to receive a distal portion of the slider 2900 such that the slider 2900 can move in a proximal-distal direction along the opening 2120. In some examples, the top surface of the housing 2100 can include an indentation 2110 that is formed around the opening 2120. In some embodiments, the opening 2120 is configured to accommodate a proximal portion 2920 of the slider 2900 that can have a larger profile than a distal portion of the slider 2900. In some embodiments, the housing 2100 can have an engagement portion 2130 on a proximal end of the housing 2100. In some embodiments, the engagement portion 2130 has a profile that can fit into an inserter tip 3000 described below. In some examples, the engagement portion 2130 can have a snap ring 2140 that is disposed about a mid-section of the engagement portion 2130. As will be discussed in more detail below, the inserter tip 3000 can have a lip 3005 at a distal end 3020 that is configured to fit over the snap ring 2140 of the engagement portion 2130. The snap ring 2140 can allow for a removable attachment of the inserter tip 3000 while also allowing the inserter tip 3000 to have a 360° rotation about the engagement portion 2130. In some embodiments, the engagement portion 2130 can include an opening 2150. In some examples, as illustrated in the cross-sectional view of Figure 22E below, the opening 2150 can extend through the engagement portion 2130 and into a channel 2160 of the housing 2100. As will be discussed in more detail below, the opening 2150 allows for the pusher pin 2810 to extend through the housing 2100 and into the inserter tip 3000 in a proximal-distal direction. In some examples, the channel 2160 extends through the housing 2100 and houses the mover 2500 as it moves in a proximal-distal direction within the housing 2100.

Turning next to the stem 2200, in some embodiments, the stem 2200 includes a proximal portion 2210, a distal portion 2220, and a waist 2240 formed there between. As discussed above, in some embodiments, the engagement lip 2170 of the housing 2100 is configured to engage with the waist 2240 of the stem 2200 such that the housing 2100 and stem 2200 are secured together. In some embodiments, the stem 2200 includes an opening 2230 that extends through a proximal end of the proximal portion 2210. In some examples, the opening 2230 is configured to receive a distal end of the slide stop pin 2300.

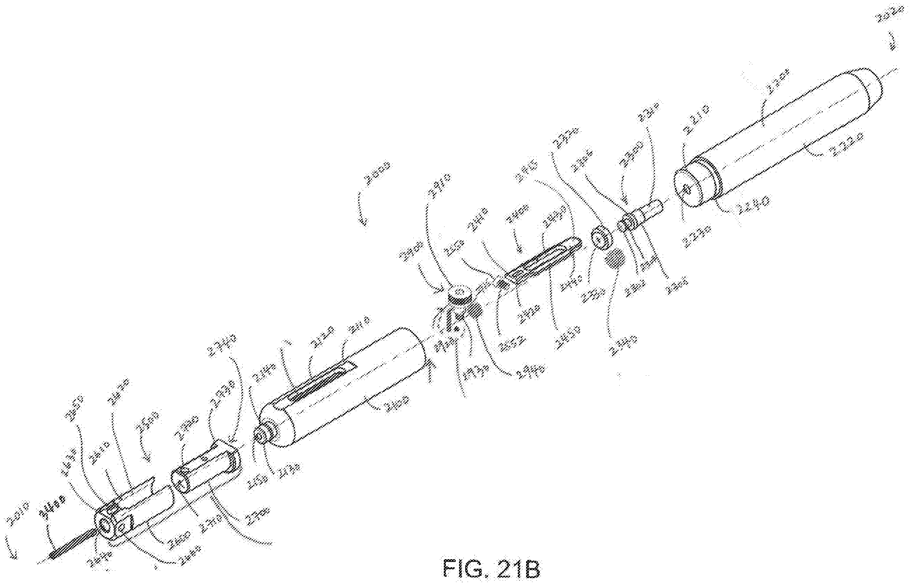

In some examples, the insertion device 2000 includes a slide stop pin 2300. In some embodiments, the slide stop pin 2300 is configured to provide minimal resistance to a distal end of a portion of the mover 2500 such that the user can advance the pusher pin 2810 in a controlled proximal-distal movement within the housing 2100. As illustrated in Figure 21B, the slide stop pin 2300 can include a protrusion 2302 at a proximal end and a cylindrical distal portion 2310 at a distal end. In some embodiments, the cylindrical distal portion 2310 is configured to be inserted into the opening 2230 of the stem 2200. The slide stop pin 2300 can include a neck portion 2304, a shoulder 2306, and a body portion 2308 that extends between the protrusion 2302 and the cylindrical distal portion 2310. In some examples, the neck portion 2304 can have a diameter less than the adjacent protrusion 2302 and the shoulder 2306. The neck portion 2304 can be configured to retain an o-ring 2330 between the protrusion 2302 and the neck portion 2304. In some examples, the shoulder 2306 can have a diameter that is greater than the adjacent body portion 2308. In some embodiments, a washer 2320 is configured to be disposed about the body portion 2308. As the diameters of the shoulder 2306 of the slide stop pin 2300 and the proximal portion 2210 of the stem 2200 are greater than the body portion 2308, the washer 2320 can be retained on the slide stop pin 2300. In some embodiments, a spring 2340 can also be disposed about the body portion 2308. The washer 2320 can form an opening on a distal end such that the distal end of the washer 2320 is disposed over the spring 2340. As illustrated in the cross-sectional view of Figure 22E, the spring 2340 can be configured to be compressed between the washer 2320 and the proximal portion 2210. As will be discussed in more detail below, the o-ring 2330, the washer 2320, and the spring 2340 of the slide stop pin 2300 are configured to provide a small amount of resistance to a distal end of the mover 2500. As a portion of the mover 2500 is connected to the slider 2900 and the pusher pin 2810, this can allow the user to have greater control over the pusher pin 2810 to implant the punctal plug 100 in the punctum of the patient.

In some embodiments, the insertion device 2000 can include a mover 2500. In some embodiments, the mover 2500 can include a front portion 2600 and a rear portion 2700. The engagement of the front portion 2600 and the rear portion 2700 can allow the physician to control the position of the punctal plug 100 within the insertion device 2000. In some embodiments, the front portion 2600 can have a top portion that has a cutout 2610 to receive an insert 2400. As shown in Figure 21B, the insert 2400 can have a proximal portion 2410 and a distal portion 2415. In some embodiments, the proximal portion 2410 of the insert 2400 can include an opening 2420. In some examples, the insert 2400 includes a body portion 2440 that has a longitudinal opening 2430 that extends between the opening 2420 and the distal portion 2415. In some embodiments, the body portion 2440 forms a shoulder 2450 on either side of the body portion 2440. In some examples, the front portion 2600 can receive the insert 2400 in the top cutout 2610. In some embodiments, a protrusion 2650 of the front portion 2600 can be secured by the opening 2420 of the insert 2400. In some embodiments, the proximal portion 2410 of the insert 2400 is retained between the shoulder 2630 on either side of the top proximal end of the front portion 2600 to prevent the insert 2400 from rotating on the front portion 2600. In some embodiments, the shoulder 2450 on either side of the body portion 2440 of the insert 2400 can extend over either end of the cutout 2610. In some examples, the insert 2400 is aligned on the front portion 2600 such that the longitudinal opening 2430 is positioned above the body opening 2620 of the front portion 2600. In some embodiments, the front portion 2600 can include a side opening 2660 that is configured to receive a securement screw 2550. In some embodiments, the proximal end of the front portion 2600 can include an opening 2640. As illustrated in the cross-sectional view of Figure 22E, in addition to allowing the pusher pin 2810 to pass through the proximal end of the front portion 2600, the opening 2640 has a diameter large enough to receive a spring 2940. As will be discussed in more detail below, the spring 2940 provides the user with minimal resistance such that the user can advance the pusher pin 2810 in a controlled proximal direction. In some embodiments, the front portion 2600 includes a body opening 2620. The body opening 2620 can be configured to receive a proximal end of the rear portion 2700.

In some embodiments, the rear portion 2700 of the mover 2500 includes an opening 2710 in a proximal end of the mover 2500, a base 2730 at a distal end of the mover 2500, and a channel 2740 formed within the rear portion 2700. As illustrated in the cross-sectional view of Figure 22E, the opening 2710 in the proximal end of the rear portion 2700 can be configured to secure a distal end of the pusher pin 2810. In some embodiments, the rear portion 2700 is configured to control movement of the pusher pin 2810 in a proximal-distal direction. As will be discussed in more detail below, in some embodiments, the rear portion 2700 is configured to be received within the body opening 2620 of the front portion 2600. In some embodiments, the diameter of the proximal end of the rear portion 2700 is less than the inner diameter of the body opening 2620. In some examples, the base 2730 of the rear portion 2700 is located at a distal end of the rear portion 2700 and can limit the movement of the rear portion 2700 within the body opening 2620. In some embodiments, the base 2730 has a diameter that is greater than the outer diameter of the front portion 2600. In some embodiments the rear portion 2700 includes a channel 2740 that extends through the body of the rear portion 2700. As will be discussed below, the opening of the channel 2740 can be configured to receive a proximal portion of the slide stop pin 2300 and the o-ring 2330. In some examples, the rear portion 2700 can include a securement opening 2720 located on a top surface of the rear portion 2700. In some embodiments, the securement opening 2720 can be threaded to receive a threaded portion 2930 at the distal end of the slider 2900. As will be discussed in more detail below, in some embodiments the user is able to control movement of the rear portion 2700 by moving the slider 2900.

In some embodiments, the insertion device 2000 can include the slider 2900. As discussed above, the slider 2900 is configured to engage with the rear portion 2700. In some embodiments, the threaded portion 2930 is configured to engage with the securement opening 2720 on the rear portion 2700. In some examples, the distal portion 2925 of the slider 2900 is configured to move in a proximal-distal direction along the longitudinal opening 2430 of the insert 2400 and the opening 2120 of the housing 2100. The slider 2900 can include a proximal portion 2920 having an engagement portion 2910. In some embodiments, the engagement portion 2910 is disposed on an exterior of the engagement portion 2910. The engagement portion 2910 can be textured to provide a better gripping surface for the user to move the slider 2900.

In some embodiments, the insertion device 2000 includes a retainer tube 3400 and the pusher pin 2810. As illustrated in Figure 21C-21D, the retainer tube 3400 can extend through the opening 2150 of the engagement portion 2130. In some embodiments, the retainer tube 3400 can be disposed about the pusher pin 2810. As will be discussed in more detail below, in some examples, the retainer tube 3400 includes an undercut 3410 inside a proximal end of the retainer tube 3400. The undercut 3410 is configured to engage a portion of a retractor tube 3200 that is loaded with the punctal plug 100.

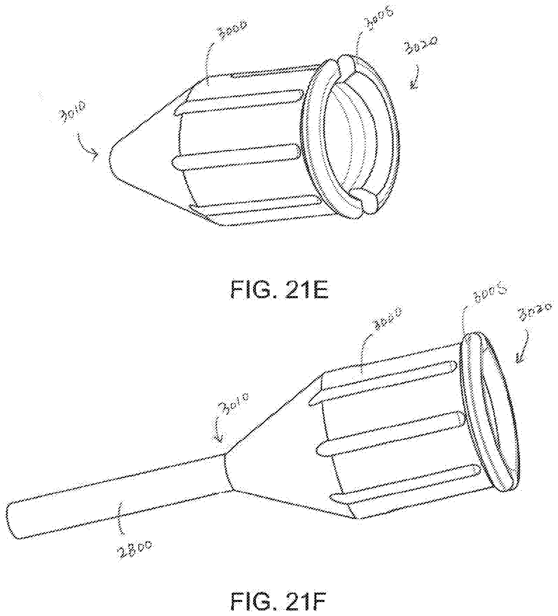

As described generally above, the insertion device 2000 can be configured to engage the inserter tip 3000. Figure 21E illustrates an embodiment of the inserter tip 3000. As shown, the inserter tip 3000 can include a proximal end 3010 and a distal end 3020. In some embodiments, the inserter tip 3000 includes a lip 3005 located at the distal end 3020 of the inserter tip 3000. As will be discussed below, the lip 3005 of the inserter tip 3000 is configured to allow the inserter tip 3000 to be removably attached to the proximal end 2010 of the insertion device 2000.

As discussed above, the inserter tip 3000 can be configured to provide for the loading of the punctal plug into the insertion device 2000. Figure 21F illustrates an embodiment of the inserter tip 3000 including the inserter tube 2800 that extends from the proximal end 3010 of the inserter tip 3000. Although not illustrated, in some examples, the inserter tube 2800 can include a cushion cup similar to the cushion cup 1710 disclosed above. In some examples, the cushion cup can be located at the proximal end of the inserter tube 2800. As discussed above, the cushion cup is configured to provide the physician with a larger surface area to rest against the punctum to position the tip of the insertion device 2000 as the physician prepares to insert the punctal plug into the punctum.

### Punctal Plug Delivery Using an Insertion Device with a Retractable Mechanism

Figures 22A-22U illustrate a step-by-step schematic illustration of the loading and delivery of the punctal plug 100 into a punctum of a patient. Although the present method discusses the insertion device 2000 with regard to the delivery of a punctal plug 100 into the punctum, the above-referenced insertion device 2000 can be used to load and deliver a device into a patient, such as in other locations of the eye.

Figures 22A-22B illustrate a plurality of view of the punctal plug 100 being loaded into the distal end of the opening 3220 of the retractor tube 3200. As illustrated, in some embodiments, the retractor tube 3200 is configured to engage with the loader tube 3100. As shown in Figures 22A-22B, in some embodiments, a distal end of the loader tube 3100 is configured to extend into the proximal end of the retractor tube 3200. In some embodiments, the insertion device 1000 is positioned such that the distal end of the punctal plug 100 is retained within the distal end of the loader tube 3100. As shown in the cross-sectional view of Figure 22A, the punctal plug 100 can extend in the retractor tube 3200 such that the flange 110 protrudes from an opening 3220 at the distal end of the retractor tube 3200. In some embodiments, the retractor tube 3200 can include a cutout 3230, the opening 3220 and a pair of engagement portions 3210. As will be discussed in more detail below, the distal pair of engagement portions 3210 can be configured to engage with a portion of the retainer tube 3400 to load the punctal plug 100 into the insertion device 2000. In some embodiments, the opening 3220 of the retractor tube 3200 allows for the punctal plug 100 to be loaded into the distal end of the retractor tube 3200. In some embodiments, the cutout 3230 is configured to engage with a portion of the flange 110 and facilitate the folding of the punctal plug 100 as it is pushed out of the inserter tip 3000. In some embodiments, the cutout 3230 prevents the punctal plug 100 from being damaged as it is pushed out of the inserter tip 3000. In some examples, the punctal plug 100 is loaded into the retractor tube 3200 and onto the distal end of the loader tube 3100 and shipped to the user in the kit 400 illustrated in Figure 16. As will be discussed below, each of the loader tube 3100 can be inserted into a proximal end of the inserter tip 3000 and loaded into the insertion device 2000.

Figures 22C-22D illustrate a plurality of views of the punctal plug 100 on the loader tube 3100 and the retractor tube 3200 and inserted into the proximal end 3010 of the inserter tip 3000. As illustrated in the cross-sectional view of Figure 22C, in some embodiments, the inserter tube 2800 is disposed about a spacer tube 3300. The spacer tube 3300 is configured to fill the volume within the inserter tube 2800. In some examples, the spacer tube 3300 can include a proximal cylinder 3310. As will be discussed in more detail below, the proximal cylinder 3310 allows the spacer tube 3300 to be removed from the inserter tube 2800. As illustrated in Figures 22C-22D, when the loader tube 3100 is inserted into the inserter tip 3000, the loader tube 3100 extends out of a proximal end of the inserter tube 2800 and a distal end of the retractor tube 3200 and the attached punctal plug 100 extends out of a distal end of the inserter tip 3000.

Figures 22E-22F illustrate a plurality of views of the punctal plug 100 as it is loaded into the proximal end 2010 of the engagement portion 2130 of the insertion device 2000. Figure 22F illustrates a cross-sectional view of the inserter tip 3000 and inserter tube 2800 with the punctal plug 100 positioned within the inserter tip 3000 as the punctal plug 100 is secured in the retractor tube 3200. As illustrated in Figure 22F, the pair of engagement portions 3210 of the retractor tube 3200 is configured to engage with the undercut 3410 located in the proximal end 2010 of the retainer tube 3400.

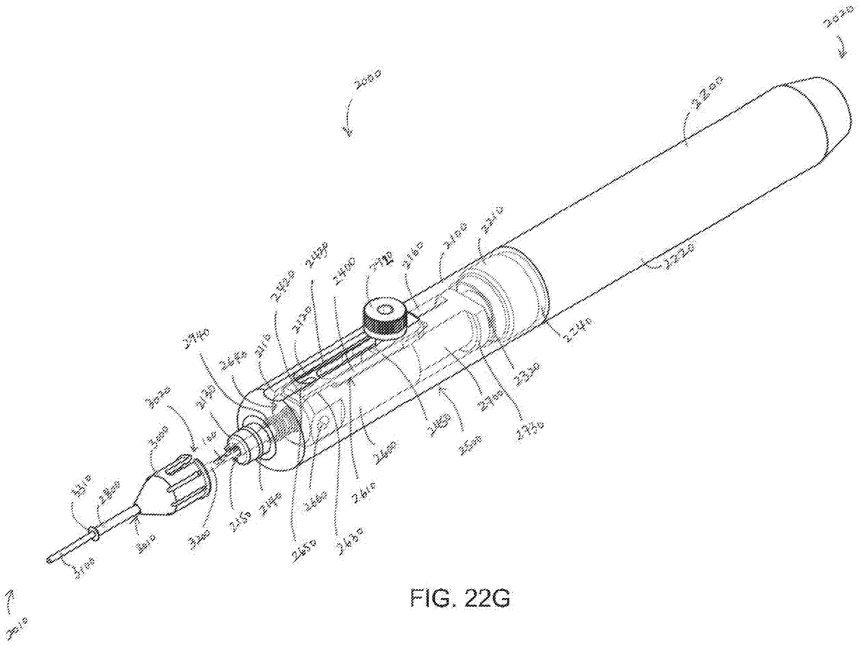

Figure 22G illustrates a perspective view of Figure 22E wherein the retractor tube 3200 is configured to load the punctal plug 100 into the engagement portion 2130 located at the proximal end 2010 of the insertion device 2000.

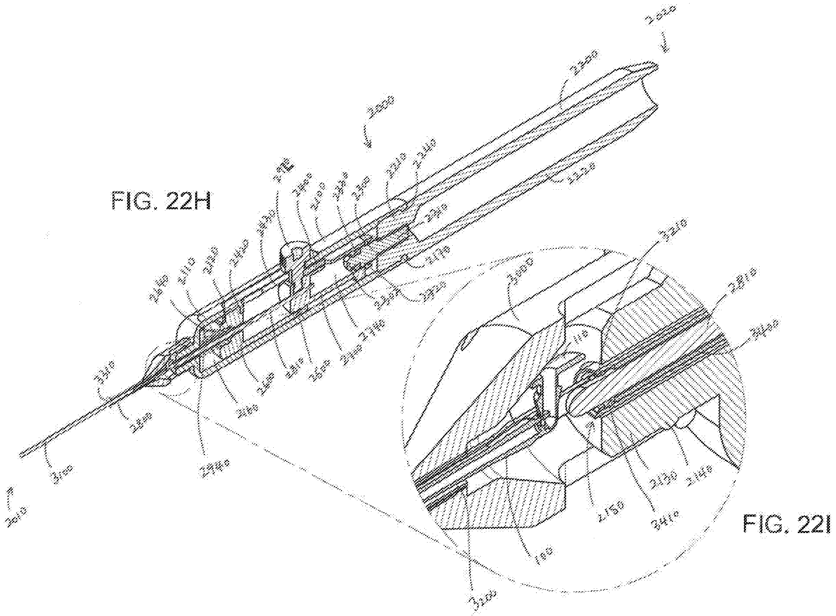

Figure 22H illustrates a cross-sectional view of the insertion device 2000 once the inserter tip 3000 is attached to the engagement portion 2130 on the 20110 of the insertion device 2000. As shown, the rear portion 2700 of the mover 2500 and the attached can be located in the distal-most position within the housing 2100 of the 2000. In some embodiments, the distal end of the rear portion 2700 of the mover 2500 abuts against the washer 2320 as the channel 2740 of the rear portion 2700 has an inner diameter smaller than the outer diameter of the washer 2320. In some embodiments, in this distal-most configuration, the channel 2740 is disposed over a proximal end of the slide stop pin 2300. In some examples, the channel 2740 is disposed over the o-ring 2330 which can include an outer diameter that is greater than the inner diameter of the channel 2740. In some embodiments, the o-ring 2330 can be made of a compressible material which allows the outer surface of the o-ring 2330 to engage with the inner surface of the channel 2740. The friction between the o-ring 2330 and the inner surface of the channel 2740 can provide for a controlled movement of the slider 2900 and the attached rear portion 2700 to advance the attached pusher pin 2810 forward.

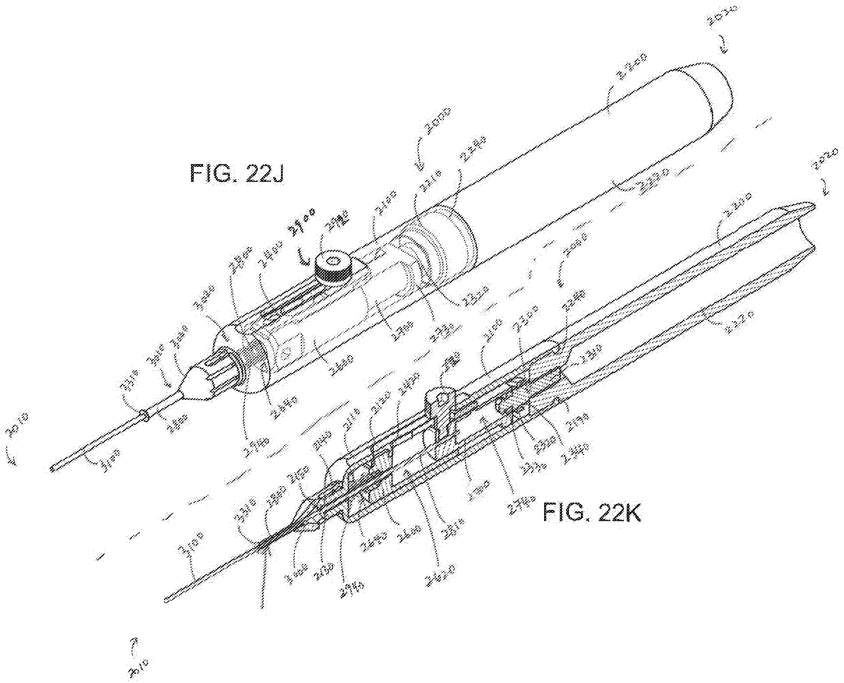

Figure 22I illustrates an enlarged view of the proximal end 2010 of the insertion device 2000 with the inserter tip 3000 engaged to the engagement portion 2130 on the proximal end 2010 of the insertion device 2000. As shown in Figure 22I, the punctal plug 100 is positioned within the distal end of the inserter tip 3000. As illustrated, a proximal end of the pusher pin 2810 is located adjacent to the distal end of the punctal plug 100. Figure 22J illustrates an additional view of a perspective view of the insertion device 2000 once the inserter tip 3000 is attached to the proximal end 2010 of the insertion device 2000.

Figures 22K-22L illustrate cross-sectional and perspective views of the punctal plug 100 pulled proximally into the inserter tube 2800 of the inserter tip 3000 as the loader tube 3100 is pulled in a proximal direction. In some embodiments the loader tube 3100 moves in a proximal direction relative to the spacer tube 3300 (wherein the proximal cylinder 3310 of the spacer tube 3300 is visible) and the inserter tube 2800. As discussed above, a distal end of the inserter tube 2800 engages the distal end of the punctal plug 100. Therefore, as the loader tube 3100 is pulled in a proximal direction out of the inserter tube 2800, the loader tube 3100 is configured to the punctal plug 100 into the inserter tube 2800. As discussed above, the cutout 3230 of the retractor tube 3200 allows for the folding and straightening out of the punctal plug 100 without damaging it. As illustrated in the cross-sectional view of the Figure 22K, the punctal plug 100 is straightened out in the inserter tube 2800.

Figures 22M-22N illustrate cross-sectional and perspective views of the position of the punctal plug 100 as the spacer tube 3300 is pulled in a proximal direction and removed from the inserter tube 2800. In some examples, the spacer tube 3300 is pulled proximally relative to the inserter tube 2800. In some embodiments, the spacer tube 3300 can be removed without disrupting the position of the punctal plug 100 in the retractor tube 3200. In some examples, the inner diameter and the outer diameter of the spacer tube 3300 can be the same as the retractor tube 3200. In some embodiments, the proximal cylinder 3310 of the spacer tube 3300 is configured to provide the user with a gripping portion to remove the spacer tube 3300 from the retractor tube 3200 and the inserter tube 2800. As illustrated in Figures 22M-22N, once the loader tube 3100 and the spacer tube 3300 are removed, the distal retention portion 170 and the tip 180 of the punctal plug 100 are exposed and extend from a proximal end of the inserter tube 2800.

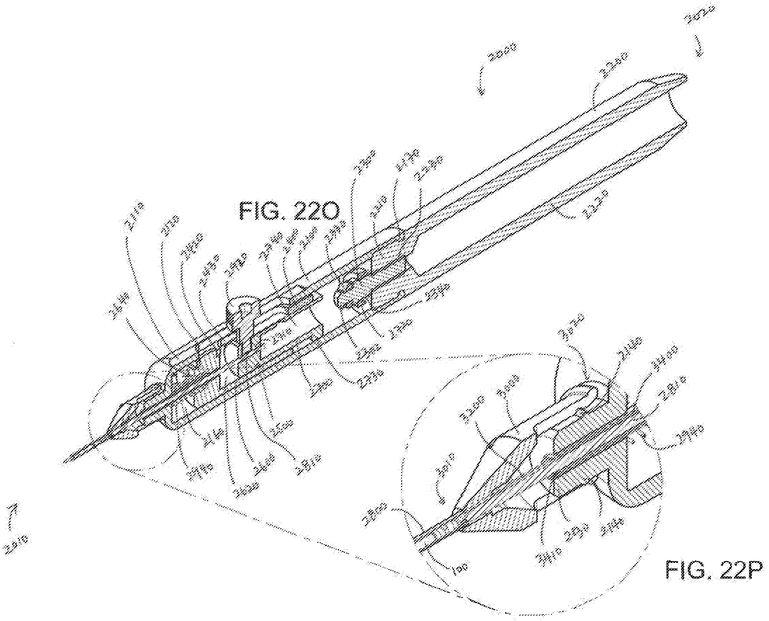

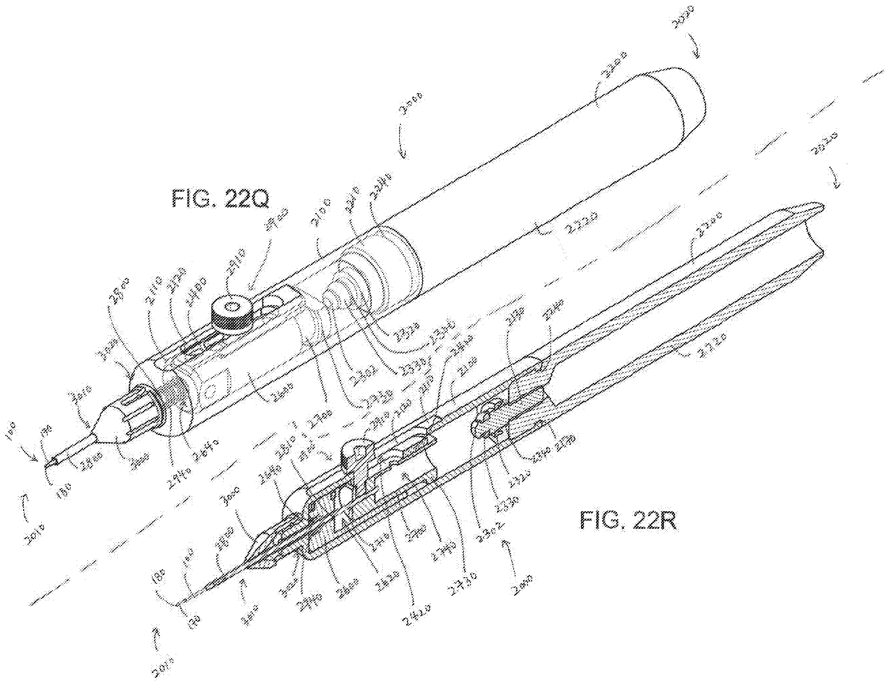

Figures 22O-22Q illustrate a cross-sectional and perspective views of the insertion device 2000 as the slider 2900 is advanced forward within the housing 2100. Figure 22P illustrates an enlarged view of the proximal end 2010 of the insertion device 2000 as the proximal end of the pusher pin 2810 is configured to engage with the punctal plug 100. In some embodiments, as illustrated in Figures 22O and 22Q, as the rear portion 2700 is advanced, the base 2730 engages with a distal end of the distal portion 2415 of the insert 2400. In some embodiments, this engagement is a friction engagement between the materials of the insert 2400 and the rear portion 2700. As discussed above, the insert 2400 is attached to the front portion 2600 by engaging the protrusion 2650 of the front portion 2600 with the opening 2420 of the insert 2400. Therefore, engagement of the distal portion 2415 of the insert 2400 and the base 2730 of the rear portion 2700 causes the front portion 2600 to also advance in a proximal direction through the housing 2100 as the slider 2900 is pushed in a proximal direction. As shown, the enlarged view of Figure 22P, as the slider 2900 is moved in a proximal direction, the proximal end of the pusher pin 2810 extends through the inserter tip 3000 and into the distal end of the inserter tube 2800.

Figures 22R-22S illustrate cross-sectional and perspective views of the insertion device 2000 as the slider 2900 is advanced forward within the housing 2100 such that the proximal end of the front portion 2600 contacts the inner surface of the channel 2160 in the housing 2100. As shown, this proximal movement of the slider 2900 further advances the pusher pin 2810 and further advances the punctal plug 100 through the inserter tube 2800. As shown in Figure 22R, at least half of the punctal plug 100 is exposed from the proximal end 2010 of the inserter tube 2800 of the insertion device 2000. In some embodiments, the position of the components of the insertion device 2000 illustrated in Figures 22R-22S is the "point of no return." Should the user further advance the slider 2900 in a proximal direction through the opening 2120 the punctal plug 100 will be fully delivered from the insertion device 2000. However, should the user retract the slider 2900 in a distal direction, the punctal plug 100 can still be fully retracted into the insertion device 2000. In some embodiments, at the configuration illustrated in Figures 22R-22S, the user receives a tactile feedback as the distal portion 2415 of the insert 2400 engages the base 2730 of the rear portion 2700. The resistance felt by the user can communicate that additional force in a proximal direction will deliver the punctal plug 100 into the punctum of the patient from the insertion device 2000. Similarly, the tactile feedback can also communicate to the user that the punctal plug 100 can still be withdrawn back into the insertion device 2000 by retracting the slider 2900 in a distal direction.

Figures 22T-22U illustrate cross-sectional and perspective views of the insertion device 2000 as the punctal plug 100 is pushed out of the proximal end 2010. In some examples, this delivers the punctal plug 100 into the target location on the patient. As discussed above, the slider 2900 can be pushed past the "point of no return" such that the proximal portion 2920 of the slider 2900 abuts against the proximal-most portion of the opening 2120. As shown in the cross-sectional view of Figure 22T, when the punctal plug 100 is pushed out of the insertion device 2000, the proximal end of the rear portion 2700 is configured to abut against an inner wall of the proximal end of the body opening 2620. As well, the base 2730 of the rear portion 2700 is configured to push past the distal portion 2415 of the 2400 as the rear portion 2700 moves proximally into the body opening 2620 of the front portion 2600. In some examples, the base 2730 of the rear portion 2700 is configured to engage with the distal end of the front portion 2600 to prevent the rear portion 2700 from proximally sliding further into the body opening 2620 of the front portion 2600.

Because the pusher pin 2810 is retained in the rear portion 2700, when the slider 2900 is slid into the proximal-most position within the opening 2120, the proximal end of the pusher pin 2810 extends through the inserter tube 2800 to push the punctal plug 100 out of the inserter tube 2800. In some embodiments, the pusher pin 2810 does not extend from the proximal end 2010 of the inserter tube 2800. In some examples, a portion of the pusher pin 2810 can extend from the proximal end 2010 of the inserter tube 2800.

### Drugs

As used herein, "drug" refers generally to one or more drugs that may be administered alone, in combination and/or compounded with one or more pharmaceutically acceptable excipients, such as binders, disintegrants, fillers, surfactants, osmogens, glidants, diluents, lubricants, polymers or other agents, and the like, auxiliary agents or compounds as may be housed within the implants as described herein. The term "drug" is a broad term that may be used interchangeably with "therapeutic agent" and "pharmaceutical" or "pharmacological agent" and includes not only so-called small molecule drugs, but also macromolecular drugs, and biologics, including but not limited to proteins, nucleic acids, antibodies and the like, regardless of whether such drug is natural, synthetic, or recombinant. Drug may refer to the drug alone or in combination with excipients, diluents, or other materials as described herein. "Drug" may also refer to an active drug or a prodrug or salt of an active drug. When the drug is in the implant, it may be referred to as a "drug load" or "drug" and it is to be understood that these terms are interchangeable.

Drugs may be formulated using excipients and/or processed using techniques to accomplish, but not limited to, the following: bioavailability enhancement, targeted delivery rate or location, incorporation with or within applicable device, improved processability or manufacturability and/or stability. Excipients and/or processing techniques may be currently known or unknown. Drug processing techniques that may be utilized in embodiments disclosed herein include, but are not limited to, particle size manipulation, tableting, hot melt extrusion, encapsulation, phase or ionic change, liquid or paste homogenization, lyophilizing, solid dispersion, and/or emulsification. As a result, the therapeutic agent may be in any form, including but not limited to compressed pellet, solid (tightly or loosely packed), capsule, multiple particles, liquid (including solution), oil, gel, paste, suspension, nanospheres, liposomes, slurry of solid particles in a liquid, emulsion, and the like. In certain embodiments, drug particles are in the form of micro-pellets, such as micro-tablets, fine powders, or slurries, each of which has fluid-like properties, allowing for charging or recharging by injection into the inner lumen(s). In other embodiments, the drug is compounded with one or more polymers that affect the rate at which a drug elutes from the implant.

It will be understood that embodiments as described herein may include a drug mixed or compounded with a biodegradable material, excipient, polymer, or other agent modifying the release characteristics of the drug to form a solid or soft solid, such as paste, gel, or the like, that may be referred to herein as a matrix or drug matrix or drug load. A drug matrix or other form of drug may be loaded into an implant directly or it may be placed into a sleeve, usually a polymer sleeve, either before or concurrent with placement into the implant. The sleeve may be permeable, semi-permeable or impermeable to one or more drugs placed therein and/or it may be permeable, semi-permeable or impermeable to water or ocular fluids such as tear fluid.

In several embodiments, such materials include biodegradable or bioerodible copolymers of lactic acid and glycolic acid, also known as poly (lactic-co-glycolic acid) or PLGA. It will be understood by one skilled in the art that although some disclosure herein specifically describes use of PLGA, other suitable biodegradable materials may be substituted for PLGA or used in combination with PLGA in such embodiments. It will also be understood that in certain embodiments as described herein, the drug positioned within the lumen or interior space of the implant is not compounded or mixed with any other compound or material, thereby maximizing the volume of drug that is positioned within the lumen.

It may be desirable, in some embodiments, to provide for a particular rate of release of drug from a PLGA copolymer, other polymeric material, or other excipient. As the release rate of a drug from a polymer correlates with the degradation rate of that polymer, control of the degradation rate provides a means for control of the delivery rate of the drug contained within the therapeutic agent. Variation of the average molecular weight of the polymer or copolymer chains, which make up the PLGA copolymer or other polymer may be used to control the degradation rate of the copolymer, thereby achieving a desired duration or other release profile of therapeutic agent delivery to the eye.

In certain other embodiments employing PLGA copolymers, rate of biodegradation of the PLGA copolymer may be controlled by varying the ratio of lactic acid to glycolic acid units in a copolymer.

Still other embodiments may utilize combinations of varying the average molecular weights of the constituents of the copolymer and varying the ratio of lactic acid to glycolic acid in the copolymer to achieve a desired biodegradation rate.

As described above, the outer shell of the implant comprises a polymer in some embodiments. Additionally, the shell may further comprise one or more polymeric coatings in various locations on or within the implant. The outer shell and any polymeric coatings are optionally biodegradable. The biodegradable outer shell and biodegradable polymer coating may be any suitable material including, but not limited to, poly(lactic acid), polyethylene-vinyl acetate, poly(lactic-co-glycolic acid), poly(D,L-lactide), poly(D,L-lactide-co-trimethylene carbonate), collagen, heparinized collagen, poly(caprolactone), poly(glycolic acid), and/or other polymer or copolymer.

In some embodiments, the drug is compounded with one or more polymers to form a gel or paste that assists in determining the elution rate of the drug. Certain preferred formulations have one or more of three properties: high drug component density, and high overall density of the material; ability to be free flowing to facilitate filing of the device to prevent air gaps or other wasted space in the device, which may include thixotropic materials; flexible or malleable finished form of the drug after being placed in the implant for designs that might flex in use or during implantation; and an ability to remain effective in delivering the drug even if the encapsulating polymer were to be ruptured, broken or otherwise damaged.

The two examples that follow provide illustrations of two types of drug matrices, a gel formulation and a paste formulation, that meet some or all of the preferred properties noted above. The Examples are provided for illustration only and are not meant to be limiting. Other suitable materials and techniques may be substituted for those mentioned in the Examples as will be understood by those skilled in the art.

### Example 1 Gel Formulation, Elution Device Assembly and Testing

300 mg of polyurethane polymer (Lubrizol, p/n PT83AS100) was dissolved in 10 ml tetrahydrofuran, for a final concentration of 30 mg/ml. An aliquot of 1 ml of the above solution was then added to 100 mg of travoprost oil. This creates a composition of 100 mg travoprost to 130 mg total weight once all solvent is removed. Upon evaporation of THF, a clear gel material remained. The net density of the drug matrix material was approximately 1.2 g/cm³.

An aliquot of the gel material was aspirated into a 0.012 inch x 0.025 inch (0.3 mm x 0.635 mm) silicone tube that was approximately 3 mm long. One end of the tube was sealed with RTV (room temperature vulcanization) silicone and the RTV was allowed to cure. The other end of the tube was cut flush with a blade such that the gel face was coincident with the end of the tube. The entire assembly was placed into a small glass vial, 1 ml of PBS buffer (phosphate-buffered saline) was added, and the vial placed into a shaker bath. The buffer was sampled at approximately 4 day intervals, and at the time of sampling, the entire 1 ml volume was replaced with fresh buffer. The samples were analyzed by HPLC for the presence of travoprost.

One advantage of the gel as described above is that the drug concentration can be very high, including over 50%, over 55%, over 60% by weight and higher. The high concentrations can be achieved in part because the drug acts as a plasticizer for the polymer which allows the polymer to have greater fluidity allowing it to be more easily loaded into an implant. The density of the drug matrix formed is greater than 1.0 g/cm³.

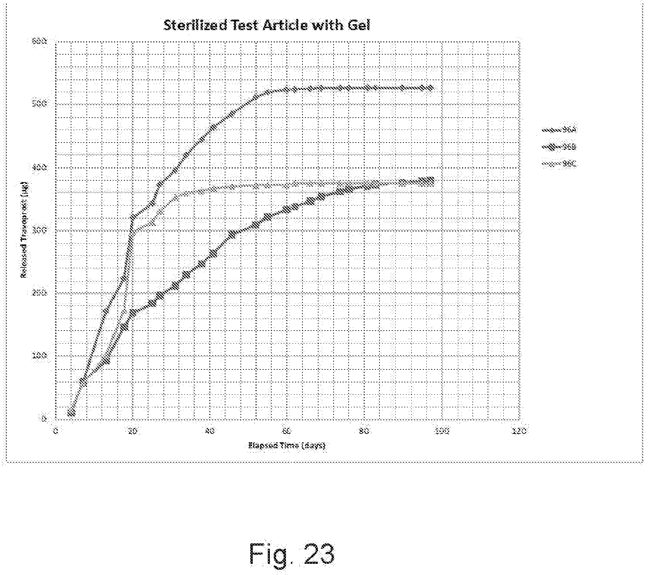

An experiment to measure elution from a simple silicone tube was conducted. A chamber measuring 3.5 mm long and 0.3 mm in diameter was loaded with three different gel formulations prepared as provided above having an average of 400 µg of travoprost, a drug density of 0.6 g/ml, and a gel density of 1.1 g/ml. The gel was allowed to elute for over 90 days and the concentration of travoprost was measured at increments of 2 to 7 days. The results are presented in Figure 23. The figure illustrates that the gel in the device can deliver drug in a reasonably linear manner until all the drug is exhausted for the gel.

### Example 2 - Paste Formulation, Elution Device Assembly and Testing

A quantity of 150 mg of propylene glycol (Spectrum, p/n PR130) and 350 mg of brimonidine free base were transferred to a mortar and mixed until a uniform paste developed. The net density of the material was approximately 1.025 g/cm³.

An aliquot of the paste drug matrix material was transferred to a 1cc syringe and injected through a 30G needle into a 0.018 inch x 0.020 inch (0.457 mm x 0.508 mm) polymer tube that was approximately 3 mm long. Both ends of the tube were sealed with RTV silicone and the RTV was allowed to cure. The entire assembly was placed into a small glass vial, 1 ml of PBS buffer was added, and the vial was placed into a shaker bath. The buffer was sampled at approximately 4 day intervals, and at the time of sampling, the entire 1 ml was replaced with fresh buffer. The samples were analyzed by HPLC for the presence of brimonidine.

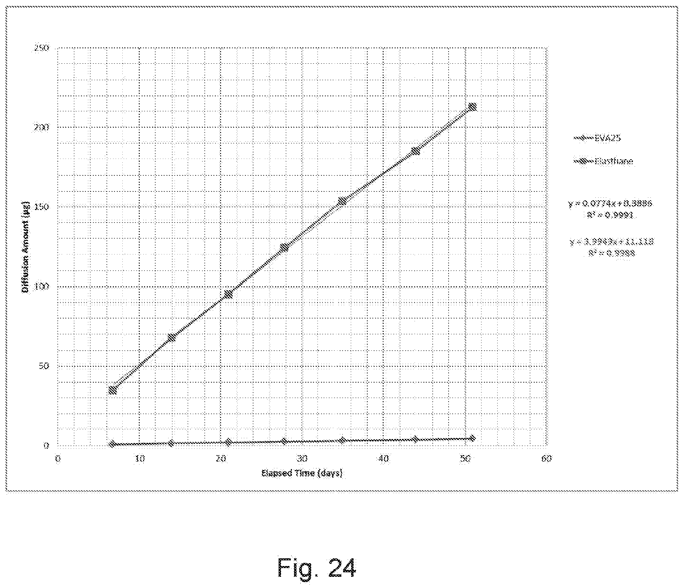

An experiment to measure elution from a simple tube was conducted. A chamber measuring 1.5 mm long and 0.5 mm in diameter was loaded with a paste formulation prepared as provided above having an average of 150 µg of brimonidine, a drug density of 0.7 g/ml, and a gel density of 1.025 g/ml. The paste was allowed to elute for over 50 days and the concentration of brimonidine was measured at increments of 7 to 9 days. The results are presented in Figure 24. The figure illustrates that the paste in the device can deliver drug in a fairly linear manner, the rate being dependent at least in part on the polymer tube thickness and particular polymers chosen for both the tube and the paste.

One advantage of the paste as described above is that the drug concentration can be very high, including over 50%, over 55%, over 60% by weight and higher. The high concentrations can be achieved in part because the drug acts as a plasticizer for the polymer which allows the polymer to have greater fluidity allowing it to be more easily loaded into an implant. The density of the drug matrix formed is greater than 1.0 g/cm³.

In some embodiments, as discussed above, the drug or drugs employed may take one or more forms. For example, multiple pellets of single or multiple drug(s) are placed within an interior lumen of the implant, such as illustrated by Figure 4.

In some embodiments, the therapeutic agent (or agents) is formulated as micro-pellets or micro-tablets. Additionally, in some embodiments, micro-tablets allow a greater amount of the therapeutic agent to be used in an implant. This is because, in some embodiments, tableting achieves a greater density in a pellet than can be achieved by packing a device. Greater amounts of drug in a given volume may also be achieved by decreasing the amount of excipient used as a percentage by weight of the whole tablet, which has been found by the inventors to be possible when creating tablets of a very small size while retaining the integrity of the tablet. In some embodiments, the percentage of active therapeutic (by weight) is about 70% or higher. As discussed herein, the therapeutic agent can be combined with excipients or binders that are known in the art. In some embodiments, the percentage of therapeutic agent ranges from about 70% to about 95%, from about 75 to 85%, from about 75 to 90%, from about 70 to 75%, from about 75% to about 80% from about 80% to about 85%, from about 85% to about 90%, from about 90% to about 95%, from about 95% to about 99%, from about 99% to about 99.9%, and overlapping ranges thereof. In some embodiments, the percentage of therapeutic agent ranges from about 80% to about 85%, including 81, 82, 83, and 84% by weight.

In several embodiments, micro-tablets provide an advantage with respect to the amount of an agent that can be packed, tamped, or otherwise placed into an implant disclosed herein. The resultant implant comprising micro-tablets, in some embodiments, thus comprises therapeutic agent at a higher density than can be achieved with non-micro-tablet forms. For example, in some embodiments, the density of the micro-pellet form of an agent within an implant ranges from about 0.7 g/cc to about 1.6 g/cc. In some embodiments, the density used in an implant ranges from about 0.7 g/cc to about 0.9 g/cc, from about 0.9 g/cc to about 1.1 g/cc, from about 1.1 g/cc to about 1.3 g/cc, from about 1.1 g/cc to about 1.5 g/cc, from about 1.3 g/cc to about 1.5 g/cc, from about 1.5 g/cc to about 1.6 g/cc, and overlapping ranges thereof. In some embodiments, densities of therapeutic agent that are greater than 1.6 g/cc are used.

In one embodiment, micro-tablets with the above properties, or any combination thereof, are made using known techniques in the art including tableting, lyophilization, granulation (wet or dry), flaking, direct compression, molding, extrusion, and the like. Moreover, as discussed below, alterations in the above-discussed characteristics can be used to tailor the release profile of the micro-tableted therapeutic agent from an implant.

In several embodiments, lyophilization of a therapeutic agent is used prior to compounding, micro-pelleting or other uses. In some embodiments, lyophilization improves the stability of the therapeutic agent especially if used in a solid state, such as in a micro-tablet. In some embodiments, lyophilization allows for a greater concentration of therapeutic to be obtained, thereby enhancing the ability to achieve the high percentages of active therapeutic agents that are desirable in some embodiments. For example, many commercially available therapeutic agents useful to treat ocular diseases are developed as first-line agents for other diseases. As such, their original formulation may not be suitable or ideal for administration to an ocular target via an ocular implant such as those disclosed herein. For example, several anti-VEGF compounds are supplied as sterile liquid in single use vials meant to be administered intravenously, such as bevacizumab. As a result, such a liquid formulation is less preferred for formation of micro-pellets or other solid forms as compared to a solid, though a liquid therapeutic agent may optionally be used in some embodiments. To achieve high percentages of therapeutic agent in a solid form, such liquid formulations may be frozen, such as stored at temperatures between -20°C and -80°C for 16 to 24 hours or longer, and then subject to lyophilization until dry. Alternatively, air spraying, crystallization, or other means may optionally be used to dry the therapeutic agent.

In several embodiments, the therapeutic agent is a protein, and in such embodiments, drying and/or tabletization should be completed under conditions, such as particular temperature, acid/base, and the like, that do not adversely affect the biological activity of the therapeutic agent. To assist in maintenance of biological activity of micro-pelleted therapeutic agents, in some embodiments, protein therapeutics are formulated with a stabilizing agent, such as mannitol, trehalose, starch, or other poly-hydroxy polymers, to maintain the structure (and therefore activity) of the therapeutic protein.

As mentioned above, depending on the embodiment, the drug or drugs to be administered via the drug delivery implant may be in the form of a nanodispersion. Nanodispersions are particularly advantageous when the drug (or drugs) to be administered is poorly soluble or insoluble in aqueous solutions, which can lead to instability and/or reduced bioavailability.

As used herein, the term "nanodispersion" shall be given its ordinary meaning and shall refer to a composition comprising nanoparticles comprising a drug and/or an aqueous vehicle. In several embodiments, the aqueous vehicle comprises a water miscible solvent and water. In several embodiments, the nanoparticles may comprise a drug, a polymer and a surfactant comprising a mixture of fatty acids or its salts and sterol or its derivatives or its salts, in some embodiments.

The term "nanoparticle" as used herein shall be given its ordinary meaning and shall also refer to particles having controlled dimensions of the order of nanometers. For example the nanoparticles, in several embodiments, are a polymeric nanoparticle (matrix of polymer entrapping the drug) and/or a polymeric nanovesicle (polymer stabilized nano sized vesicle encapsulating the drug.) and/or a polymeric nanocapsule (polymeric membrane surrounding drug in core) and/or nano sized particles of the drug stabilized by surfactants, and the like the nanoparticles having mean size less than about 300 nm, such as ranging from about 10 nm to about 275 nm, or in the range of about 10 nm to about 200 nm.

In several embodiments, the water miscible solvent used in the nanodispersion comprises one or more of alcohols, glycols and its derivatives, polyalkylene glycols and its derivatives, glycerol, glycofurol and combinations thereof. Additional non-limiting examples include, but are not limited to, alcohols such as ethanol, n-propanol, isopropanol; glycols such as ethylene glycol, propylene glycol, butylene glycol and its derivatives; polyethylene glycols like PEG 400 or PEG 3350; polypropylene glycol and its derivatives such as PPG-10 butanediol, PPG-10 methyl glucose ether, PPG-20 methyl glucose ether, PPG-15 stearyl ether; glycerol; glycofurol and the like and mixtures thereof. In still additional embodiments, the non-aqueous solvent is selected from the group consisting of alcohols, polyethylene glycols and/or mixtures thereof, such as, for example, a mixture of ethanol and PEG (polyethylene glycol). In some embodiments, in which ethanol is used in the nanodispersion, ethanol is present in an amount ranging from about 0.001% w/v to about 5% w/v, more preferably from about 0.05% w/v to about 0.5% w/v and most preferably from about 0.1% w/v to about 0.25% w/v. Polyethylene glycols which are used preferably, include PEG-400 and PEG-3350. PEG-400 is used, depending on the embodiment, in an amount ranging from about 0.01% w/v to about 20.0% w/v, more preferably from about 0.05% w/v to about 5.0% w/v and most preferably from about 1.0% w/v to about 2.5% w/v. PEG-3350 is used, depending on the embodiment, in an amount ranging from about 0.001% w/v to about 10.0% w/v, more preferably from about 0.05% w/v to about 5.0% w/v and most preferably from about 0.1% w/v to about 3% w/v.

In some embodiments, the nanoparticles comprise one or more polymers. The polymer(s) used in several embodiments are preferably, water soluble. Polyvinylpyrrolidone, one such water soluble polymer used in several embodiments, is a tertiary amide polymer having linearly arranged monomer units of 1-vinyl-2-pyrrolidone. It has mean molecular weights ranging from about 10,000 to about 700,000. Other grades of polyvinylpyrrolidone are used in some embodiments, with molecular weights ranging from about 2000 to about 3000, about 7000 to about 11,000, about 28,000 to about 34,000, or about 1,000,000 to about 1,5000,000. In still additional embodiments, polyvinylpyrrolidone use for the polymer have molecular weight in the range from about 1,000 to about 45,000, preferably, from about 4,000 to about 30,000. According several embodiments, the amount of polymer used in the nanodispersion ranges from about 0.001% w/v to about 20% w/v, including preferably about 0.01% w/v to about 5.0% w/v and also about 0.01% w/v to about 1.0% w/v.

Polyethylene glycol is used in several embodiments, either in addition or in place of polyvinylpyrrolidone. In several embodiments, the amount of polymer used in the nanodispersion ranges from about 0.001% w/v to about 20% w/v, including about 0.01% w/v to about 5.0% w/v, and in some embodiments, about 0.01% w/v to about 1.0% w/v.

Surfactants are used in some embodiments of the nanodispersions for drug(s). In several embodiments, the surfactants comprise a mixture of fatty acid or its salts and sterol or its derivatives or its salts.

As used herein, the term "fatty acids" shall be given its ordinary meaning and shall also include aliphatic (saturated or unsaturated) monocarboxylic acids derived from or contained in esterified form, in an animal or vegetable fat, oil or wax. Non-limiting examples of fatty acids (or its salts) that may be used in in several embodiments include, but are not limited to, fatty acids or its salts having 'n' number of carbon atoms wherein 'n' ranges from about 4 to about 28. The fatty acid may be a saturated fatty acid or an unsaturated fatty acid, and their salt and combinations thereof. Depending on the embodiment, the saturated fatty acid and its salts may be selected from butyric acid, caproic acid, caprylic acid, capric acid, lauric acid, myristic acid, palmitic acid, stearic acid, arachidic acid, behenic acid, sodium caprylate, sodium laurate, sodium myristate, sodium palmitate and the like and/or mixtures thereof. The unsaturated fatty acid and its salts may be selected from myristoleic acid, palmitoleic acid, oleic acid, linoleic acid, alpha linolenic acid, arachidonic acid, eicosapentaenoic acid, erucic acid, docosahexaenoic acid, sodium oleate, sodium arachidonate and the like and/or mixtures thereof.

Additionally, non-limiting examples, of sterol or its derivative or its salts that may be used in the nanodispersion or nanoparticles may be acid esters of sterols. The sterols that may be suitable, but are not limited to, cholesterol, phytosterols, ergosterol, bile acids salts and mixtures thereof. Acid salts of cholesterol that may be used include, but are not limited to, cholesteryl sulfate, cholesterol acetate, cholesterol chloroacetate, cholesterol benzoate, cholesterol myristate, cholesterol hemisuccinate, cholesterol phosphate, cholesterol phosphate, phosphonate, borate, nitrate, cholesterol cinnamate, cholesterol crotanoate, cholesterol butyrate, cholesterol heptanoate, cholesterol hexanoate, cholesterol octanoate, cholesterol nonanoate, cholesterol decanoate, cholesterol oleate, cholesterol propionate, cholesterol valerate, dicholesteryl carbonate and the like and mixtures thereof. Phytosterols that may be used in the compositions include sitosterol, campesterol, stigmasterol, brassicasterol and its derivatives, salts and mixture thereof. For example, Phytosterols marketed by Sigma, U.S.A. containing bsitosterol, campesterol and dihydrobrassicasterol. Bile acids include cholic acid, chenodeoxycholic acid, deoxycholic acid, glycocholic acid, taurocholic acid, ursodeoxycholic acid and its derivatives, salts and mixture thereof. The sterols can also be esters of cholesterol including cholesterol hemi-succinate, salts of cholesterol including cholesterol hydrogen sulfate and cholesterol sulfate, ergosterol, esters of ergosterol including ergosterol hemi-succinate, salts of ergosterol including ergosterol hydrogen sulfate and ergosterol sulfate, lanosterol, esters of lanosterol including lanosterol hemi-succinate, salts of lanosterol including lanosterol hydrogen sulfate and lanosterol sulfate.

According to one embodiment, the nanoparticles comprise a surfactant which is a mixture of sterol or its derivatives or its salts and fatty acids or its salts. In an additional embodiment, the nanoparticles comprise of cholesterol ester of polar acids. In still further embodiments, the surfactant used in the nanodispersion is a mixture of caprylic acid and cholesteryl sulfate. Caprylic acid, also known as octanoic acid may be used in such embodiments in an amount ranging from about 0.001% w/v to about 5.0% w/v, more preferably from about 0.01% w/v to about 1.0% w/v and most preferably from about 0.01% w/v to about 0.5% w/v. Cholesteryl sulfate is used in certain embodiments in an amount ranging from about 0.001% w/v to about 5.0% w/v, more preferably from about 0.01% w/v to about 1.0% w/v and most preferably from about 0.01% w/v to about 0.5% w/v. In one embodiment, the surfactant used is selected from oleic acid and cholesteryl sulphate and/or mixtures thereof. In some embodiments, the surfactant used is selected from saturated fatty acid and bile acid or bile salt and/or mixtures thereof. Bile salts, when used according to some embodiments, are present in an amount ranging from about 0.001% w/v to about 5.0% w/v, more preferably from about 0.01% w/v to about 1.0% w/v and most preferably from about 0.01% w/v to about 0.75% w/v. Other amounts may be used in conjunction with other embodiments disclosed herein. Nanodispersions can be generated by methods appreciated in the art, such as those methods (and the resulting nanodispersions) disclosed in US Patent No. 8,778,364, which is incorporated by reference in its entirety herein.

In addition, one or more of the therapeutic drug regions may comprise drug-cyclodextrin inclusion complexes; liposome encapsulation; micelles based on polymers such as polysaccharide, poly (ethylene glycol)-poly(lactide), methoxy poly(ethylene glycol)-poly(hexyl-lactide), or hydrophobically-modified hydroxypropylcellulose; nanoparticles of amorphous drug formed by antisolvent precipitation and stabilized with surfactant such as poysorbate 80 or polyoxyl 15 hydroxystearate; nanoparticles having a mean size less than 500 nm containing one or more drugs, a polymer, and a surfactant, where the surfactant may include a mixture of fatty acids or its salts and sterol or its derivitatives or its salts; drug co-processed or granulated with excipients such as microcrystalline cellulose, lactose, hydroxypropyl methyl cellulose, or povidone; added polyethylene glycol chains to the drug, polymer, or surfactant (PEGylation); solid dispersions in polymeric carriers such as hypromellose acetate succinate, copolymers based on dimethylaminoethyl methacrylate, butyl methacrylate, and methyl methacrylate, poly(vinylpyrrolidone-vinyl acetate), or lauroyl macrogolglycerides; or microspheres (for example, based on PLGA or chitosan).

The therapeutic agents utilized with the drug delivery implant, may include one or more drugs provided below, either alone or in combination. The drugs utilized may also be the equivalent of, derivatives of, or analogs of one or more of the drugs provided below.

When more than one drug is desired for treatment of a particular pathology or when a second drug is administered such as to counteract a side effect of the first drug, some embodiments may utilize two agents of the same form. In other embodiments, agents in different form may be used. Likewise, should one or more drugs utilize an adjuvant, excipient, or auxiliary compound, for example to enhance stability or tailor the elution profile, that compound or compounds may also be in any form that is compatible with the drug and can be reasonably retained with the implant. In some embodiments, treatment of particular pathology with a drug released from the implant may not only treat the pathology, but also induce certain undesirable side effects.

The drugs may include but are not limited to pharmaceutical agents including anti-glaucoma medications, ocular agents, antimicrobial agents, such as antibiotic, antiviral, antiparasitic, and antifungal agents, anti-inflammatory agents (including steroids or non-steroidal anti-inflammatory), biological agents including hormones, enzymes or enzyme-related components, antibodies or antibody-related components, oligonucleotides (including DNA, RNA, short-interfering RNA, antisense oligonucleotides, and the like), DNA/RNA vectors, viruses (either wild type or genetically modified) or viral vectors, peptides, proteins, enzymes, extracellular matrix components, and live cells configured to produce one or more biological components. The use of any particular drug is not limited to its primary effect or regulatory body-approved treatment indication or manner of use. Drugs also include compounds or other materials that reduce or treat one or more side effects of another drug or therapeutic agent. As many drugs have more than a single mode of action, the listing of any particular drug within any one therapeutic class below is only representative of one possible use of the drug and is not intended to limit the scope of its use with the ophthalmic implant system.

As discussed above, the therapeutic agents may be combined with any number of excipients as is known in the art. In addition to the biodegradable polymeric excipients discussed above, other excipients may be used, including, but not limited to, benzyl alcohol, ethylcellulose, methylcellulose, hydroxymethylcellulose, cetyl alcohol, croscarmellose sodium, dextrans, dextrose, fructose, gelatin, glycerin, monoglycerides, diglycerides, kaolin, calcium chloride, lactose, lactose monohydrate, maltodextrins, polysorbates, pregelatinized starch, calcium stearate, magnesium stearate, silicon dioxide, cornstarch, talc, and the like. The one or more excipients may be included in total amounts as low as about 1%, 5%, or 10% and in other embodiments may be included in total amounts as high as 50%, 70% or 90%.

Examples of drugs may include various anti-secretory agents; antimitotics and other anti-proliferative agents, including among others, anti-angiogenesis agents such as angiostatin, anecortave acetate, thrombospondin, VEGF receptor tyrosine kinase inhibitors and anti-vascular endothelial growth factor (anti-VEGF) drugs such as ranibizumab (LUCENTIS^{®}) and bevacizumab (AVASTIN^{®}), pegaptanib (MACUGEN^{®}), aflibercept (EYLEA), sunitinib and sorafenib and any of a variety of known small-molecule and transcription inhibitors having anti-angiogenesis effect; classes of known ophthalmic drugs, including: glaucoma agents, such as adrenergic antagonists, including for example, beta-blocker agents such as atenolol propranolol, metipranolol, betaxolol, carteolol, levobetaxolol, levobunolol and timolol; adrenergic agonists or sympathomimetic agents such as epinephrine, dipivefrin, clonidine, aparclonidine, and brimonidine; parasympathomimetics or cholingeric agonists such as pilocarpine, carbachol, phospholine iodine, and physostigmine, salicylate, acetylcholine chloride, eserine, diisopropyl fluorophosphate, demecarium bromide); muscarinics; carbonic anhydrase inhibitor agents, including topical and/or systemic agents, for example acetozolamide, brinzolamide, dorzolamide and methazolamide, ethoxzolamide, diamox, and dichlorphenamide; mydriatic-cycloplegic agents such as atropine, cyclopentolate, succinylcholine, homatropine, phenylephrine, scopolamine and tropicamide; prostaglandins such as prostaglandin F2 alpha, antiprostaglandins, prostaglandin precursors, or prostaglandin analog agents such as bimatoprost, latanoprost, travoprost and unoprostone.

Other examples of drugs may also include anti-inflammatory agents including for example glucocorticoids and corticosteroids such as betamethasone, cortisone, dexamethasone, dexamethasone 21-phosphate, methylprednisolone, prednisolone 21-phosphate, prednisolone acetate, prednisolone, fluroometholone, loteprednol, medrysone, fluocinolone acetonide, triamcinolone acetonide, triamcinolone, triamcinolone acetonide, beclomethasone, budesonide, flunisolide, fluorometholone, fluticasone, hydrocortisone, hydrocortisone acetate, loteprednol, rimexolone and non-steroidal anti-inflammatory agents including, for example, diclofenac, flurbiprofen, ibuprofen, bromfenac, nepafenac, and ketorolac, salicylate, indomethacin, ibuprofen, naxopren, piroxicam and nabumetone; anti-infective or antimicrobial agents such as antibiotics including, for example, tetracycline, chlortetracycline, bacitracin, neomycin, polymyxin, gramicidin, cephalexin, oxytetracycline, chloramphenicol, rifampicin, ciprofloxacin, tobramycin, gentamycin, erythromycin, penicillin, sulfonamides, sulfadiazine, sulfacetamide, sulfamethizole, sulfisoxazole, nitrofurazone, sodium propionate, aminoglycosides such as gentamicin and tobramycin; fluoroquinolones such as ciprofloxacin, gatifloxacin, levofloxacin, moxifloxacin, norfloxacin, ofloxacin; bacitracin, erythromycin, fusidic acid, neomycin, polymyxin B, gramicidin, trimethoprim and sulfacetamide; antifungals such as amphotericin B and miconazole; antivirals such as idoxuridine trifluorothymidine, acyclovir, gancyclovir, interferon; antimicotics; immune-modulating agents such as antiallergenics, including, for example, sodium chromoglycate, antazoline, methapyriline, chlorpheniramine, cetrizine, pyrilamine, prophenpyridamine; anti-histamine agents such as azelastine, emedastine and levocabastine; immunological drugs (such as vaccines, immune stimulants, and/or immunosuppressants); MAST cell stabilizer agents such as cromolyn sodium, ketotifen, lodoxamide, nedocrimil, olopatadine and pemirolastciliary body ablative agents, such as gentimicin and cidofovir; and other ophthalmic agents such as verteporfin, proparacaine, tetracaine, cyclosporine and pilocarpine; inhibitors of cell-surface glycoprotein receptors; decongestants such as phenylephrine, naphazoline, tetrahydrazoline; lipids or hypotensive lipids; dopaminergic agonists and/or antagonists such as quinpirole, fenoldopam, and ibopamine; vasospasm inhibitors; vasodilators; antihypertensive agents; angiotensin converting enzyme (ACE) inhibitors; angiotensin-1 receptor antagonists such as olmesartan; microtubule inhibitors; molecular motor (dynein and/or kinesin) inhibitors; actin cytoskeleton regulatory agents such as cyctchalasin, latrunculin, swinholide A, ethacrynic acid, H-7, and Rho-kinase (ROCK) inhibitors; remodeling inhibitors; modulators of the extracellular matrix such as tert-butylhydro-quinolone and AL-3037A; adenosine receptor agonists and/or antagonists such as N-6-cylclophexyladenosine and (R)-phenylisopropyladenosine; serotonin agonists; hormonal agents such as estrogens, estradiol, progestational hormones, progesterone, insulin, calcitonin, parathyroid hormone, peptide and vasopressin hypothalamus releasing factor; growth factor antagonists or growth factors, including, for example, epidermal growth factor, fibroblast growth factor, platelet derived growth factor or antagonists thereof (such as those disclosed in United States Patent 7,759,472 or United States Patent Application Nos. 12/465,051, 12/564,863, or 12/641,270, each of which is incorporated in its entirety by reference herein), transforming growth factor beta, somatotrapin, fibronectin, connective tissue growth factor, bone morphogenic proteins (BMPs); cytokines such as interleukins, CD44, cochlin, and serum amyloids, such as serum amyloid A.

Other therapeutic agents may include neuroprotective agents such as lubezole, nimodipine and related compounds, and including blood flow enhancers such as dorzolamide or betaxolol; compounds that promote blood oxygenation such as erythropoeitin; sodium channels blockers; calcium channel blockers such as nilvadipine or lomerizine; glutamate inhibitors such as memantine nitromemantine, riluzole, dextromethorphan or agmatine; acetylcholinsterase inhibitors such as galantamine; hydroxylamines or derivatives thereof, such as the water soluble hydroxylamine derivative OT-440; synaptic modulators such as hydrogen sulfide compounds containing flavonoid glycosides and/or terpenoids, such as ginkgo biloba; neurotrophic factors such as glial cell-line derived neutrophic factor, brain derived neurotrophic factor; cytokines of the IL-6 family of proteins such as ciliary neurotrophic factor or leukemia inhibitory factor; compounds or factors that affect nitric oxide levels, such as nitric oxide, nitroglycerin, or nitric oxide synthase inhibitors; cannabinoid receptor agonsists such as WIN55-212-2; free radical scavengers such as methoxypolyethylene glycol thioester (MPDTE) or methoxypolyethlene glycol thiol coupled with EDTA methyl triester (MPSEDE); anti-oxidants such as astaxathin, dithiolethione, vitamin E, or metallocorroles, such as iron, manganese or gallium corroles; compounds or factors involved in oxygen homeostasis such as neuroglobin or cytoglobin; inhibitors or factors that impact mitochondrial division or fission, such as Mdivi-1 (a selective inhibitor of dynamin related protein 1 (Drp1)); kinase inhibitors or modulators such as the Rho-kinase inhibitor H-1152 or the tyrosine kinase inhibitor AG1478; compounds or factors that affect integrin function, such as the Beta 1-integrin activating antibody HUTS-21; N-acyl-ethanaolamines and their precursors, N-acyl-ethanolamine phospholipids; stimulators of glucagon-like peptide 1 receptors, such as glucagon-like peptide 1; polyphenol containing compounds such as resveratrol; chelating compounds; apoptosis-related protease inhibitors; compounds that reduce new protein synthesis; radiotherapeutic agents; photodynamic therapy agents; gene therapy agents; genetic modulators; auto-immune modulators that prevent damage to nerves or portions of nerves, like demyelination, such as glatimir; myelin inhibitors such as anti-NgR Blocking Protein, NgR(310)ecto-Fc; other immune modulators such as FK506 binding proteins, such as FKBP51; and dry eye medications such as cyclosporine, cyclosporine A, delmulcents, and sodium hyaluronate.

Other therapeutic agents that may be used include: other beta-blocker agents such as acebutolol, atenolol, bisoprolol, carvedilol, asmolol, labetalol, nadolol, penbutolol, and pindolol; other corticosteroidal and non-steroidal anti-inflammatory agents such aspirin, betamethasone, cortisone, diflunisal, etodolac, fenoprofen, fludrocortisone, flurbiprofen, hydrocortisone, ibuprofen, indomethacine, ketoprofen, meclofenamate, mefenamic acid, meloxicam, methylprednisolone, nabumetone, naproxen, oxaprozin, prednisolone, prioxicam, salsalate, sulindac and tolmetin; COX-2 inhibitors like celecoxib, rofecoxib and valdecoxib; other immune-modulating agents such as aldesleukin, adalimumab (HUMIRA^{®}), azathioprine, basiliximab, daclizumab, etanercept (ENBREL^{®}), hydroxychloroquine, infliximab (REMICADE^{®}), leflunomide, methotrexate, mycophenolate mofetil, and sulfasalazine; other anti-histamine agents such as loratadine, desloratadine, cetirizine, diphenhydramine, chlorpheniramine, dexchlorpheniramine, clemastine, cyproheptadine, fexofenadine, hydroxyzine and promethazine; other anti-infective agents such as aminoglycosides such as amikacin and streptomycin; anti-fungal agents such as amphotericin B, caspofungin, clotrimazole, fluconazole, itraconazole, ketoconazole, voriconazole, terbinafine and nystatin; anti-malarial agents such as chloroquine, atovaquone, mefloquine, primaquine, quinidine and quinine; anti-mycobacterium agents such as ethambutol, isoniazid, pyrazinamide, rifampin and rifabutin; anti-parasitic agents such as albendazole, mebendazole, thiobendazole, metronidazole, pyrantel, atovaquone, iodoquinaol, ivermectin, paromycin, praziquantel, and trimatrexate; other anti-viral agents, including anti-CMV or anti-herpetic agents such as acyclovir, cidofovir, famciclovir, gangciclovir, valacyclovir, valganciclovir, vidarabine, trifluridine and foscarnet; protease inhibitors such as ritonavir, saquinavir, lopinavir, indinavir, atazanavir, amprenavir and nelfinavir; nucleotide/nucleoside/non-nucleoside reverse transcriptase inhibitors such as abacavir, ddI, 3TC, d4T, ddC, tenofovir and emtricitabine, delavirdine, efavirenz and nevirapine; other anti-viral agents such as interferons, ribavirin and trifluridiene; other anti-bacterial agents, including cabapenems like ertapenem, imipenem and meropenem; cephalosporins such as cefadroxil, cefazolin, cefdinir, cefditoren, cephalexin, cefaclor, cefepime, cefoperazone, cefotaxime, cefotetan, cefoxitin, cefpodoxime, cefprozil, ceftaxidime, ceftibuten, ceftizoxime, ceftriaxone, cefuroxime and loracarbef; other macrolides and ketolides such as azithromycin, clarithromycin, dirithromycin and telithromycin; penicillins (with and without clavulanate) including amoxicillin, ampicillin, pivampicillin, dicloxacillin, nafcillin, oxacillin, piperacillin, and ticarcillin; tetracyclines such as doxycycline, minocycline and tetracycline; other anti-bacterials such as aztreonam, chloramphenicol, clindamycin, linezolid, nitrofurantoin and vancomycin; alpha blocker agents such as doxazosin, prazosin and terazosin; calcium-channel blockers such as amlodipine, bepridil, diltiazem, felodipine, isradipine, nicardipine, nifedipine, nisoldipine and verapamil; other anti-hypertensive agents such as clonidine, diazoxide, fenoldopan, hydralazine, minoxidil, nitroprusside, phenoxybenzamine, epoprostenol, tolazoline, treprostinil and nitrate-based agents; anti-coagulant agents, including heparins and heparinoids such as heparin, dalteparin, enoxaparin, tinzaparin and fondaparinux; other anti-coagulant agents such as hirudin, aprotinin, argatroban, bivalirudin, desirudin, lepirudin, warfarin and ximelagatran; anti-platelet agents such as abciximab, clopidogrel, dipyridamole, optifibatide, ticlopidine and tirofiban; prostaglandin PDE-5 inhibitors and other prostaglandin agents such as alprostadil, carboprost, sildenafil, tadalafil and vardenafil; thrombin inhibitors; antithrombogenic agents; anti-platelet aggregating agents; thrombolytic agents and/or fibrinolytic agents such as alteplase, anistreplase, reteplase, streptokinase, tenecteplase and urokinase; anti-proliferative agents such as sirolimus, tacrolimus, everolimus, zotarolimus, paclitaxel and mycophenolic acid; hormonal-related agents including levothyroxine, fluoxymestrone, methyltestosterone, nandrolone, oxandrolone, testosterone, estradiol, estrone, estropipate, clomiphene, gonadotropins, hydroxyprogesterone, levonorgestrel, medroxyprogesterone, megestrol, mifepristone, norethindrone, oxytocin, progesterone, raloxifene and tamoxifen; anti-neoplastic agents, including alkylating agents such as carmustine lomustine, melphalan, cisplatin, fluorouracil3, and procarbazine antibiotic-like agents such as bleomycin, daunorubicin, doxorubicin, idarubicin, mitomycin and plicamycin; anti proliferative agents (such as 1,3-cis retinoic acid, 5-fluorouracil, taxol, rapamycin, mitomycin C and cisplatin); antimetabolite agents such as cytarabine, fludarabine, hydroxyurea, mercaptopurine and 5-fluorouracil (5-FU); immune modulating agents such as aldesleukin, imatinib, rituximab and tositumomab; mitotic inhibitors docetaxel, etoposide, vinblastine and vincristine; radioactive agents such as strontium-89; and other anti-neoplastic agents such as irinotecan, topotecan and mitotane.

## Claims

1. An implantable drug delivery device comprising:
a proximal portion;
a distal portion (104); and
an elongate portion (160) disposed between the proximal portion and the distal portion;
wherein the proximal portion is angled, relative to the elongate portion,
wherein the distal portion is angled, relative to the elongate portion,
wherein the proximal portion comprises flange (110) disposed at a proximal end of the implantable drug delivery device,
wherein the proximal portion further comprises a retention portion (130) disposed between the flange and the elongate portion, wherein the retention portion comprises an elliptical cross-section, and
wherein the drug delivery device is configured for insertion into a punctum of an eye, such that at least the distal portion and the elongate portion fully reside within the punctum of the eye when the drug delivery device is inserted.

2. The implantable drug delivery device of Claim 1, wherein an angle between the proximal portion and the elongate portion is a right angle.

3. The implantable drug delivery device of Claim 1, wherein an angle between the distal portion and the elongate portion is an obtuse angle.

4. The implantable drug delivery device of Claim 1, further comprising a lumen extending from a proximal end of the drug delivery device into the proximal portion.

5. The implantable drug delivery device of Claim 4, wherein the lumen extends from the proximal end of the drug delivery device through the proximal portion and into the elongate portion.

6. The implantable drug delivery device of Claim 4, wherein a drug is disposed within the lumen.

7. The implantable drug delivery device of Claim 6, wherein the drug is one of a powdered drug, a pelletized drug, and a liquid drug suspended in a viscous medium.

8. The implantable drug delivery device of Claim 1, wherein the flange further includes a membrane. membrane (330).

9. The implantable drug delivery device of Claim **1,** wherein the flange includes one of an elliptical or semi-elliptical shape.

10. The implantable drug delivery device of Claim 1, further comprising an elbow, disposed between the proximal portion and the elongate portion.

11. The implantable drug delivery device of Claim 10, wherein a cross-sectional area of the elbow is less than a cross sectional area of the elongate portion proximate to the elbow.

12. The implantable drug delivery device of Claim 1, wherein a cross-sectional area of the elongate portion varies across a length of the elongate portion, such that the elongate portion is tapered.

13. An implantable drug delivery system comprising:
a drug delivery device according to claim 1; and
an applicator, configured for inserting the drug delivery device into a punctum of an eye, such that at least the distal portion and the elongate portion fully reside within the punctum of the eye when the drug delivery device is inserted.

14. The implantable drug delivery system of Claim 13, wherein the applicator includes an engagement mechanism, configured to engage the flange of the drug delivery device.

## Patentansprüche

1. Implantierbare Arzneimittelabgabevorrichtung, umfassend:
einen proximalen Abschnitt;
einen distalen Abschnitt (104); und
einen länglichen Abschnitt (160), der zwischen dem proximalen Abschnitt und dem distalen Abschnitt angeordnet ist:
wobei der proximale Abschnitt in Bezug auf den länglichen Abschnitt abgewinkelt ist,
wobei der distale Abschnitt in Bezug auf den länglichen Abschnitt abgewinkelt ist,
wobei der proximale Abschnitt einen Flansch (110) umfasst, der an einem proximalen Ende der implantierbaren Arzneimittelabgabevorrichtung angeordnet ist,
wobei der proximale Abschnitt ferner einen Rückhalteabschnitt (130) umfasst, der zwischen dem Flansch und dem länglichen Abschnitt angeordnet ist, wobei der Rückhalteabschnitt einen elliptischen Querschnitt umfasst, und
wobei die Arzneimittelabgabevorrichtung für eine Einführung in einen Punkt eines Auges konfiguriert ist, sodass mindestens der distale Abschnitt und der längliche Abschnitt vollständig innerhalb des Punktes des Auges liegen, wenn die Arzneimittelabgabevorrichtung eingeführt ist.

2. Implantierbare Arzneimittelabgabevorrichtung nach Anspruch 1, wobei ein Winkel zwischen dem proximalen Abschnitt und dem länglichen Abschnitt ein rechter Winkel ist.

3. Implantierbare Arzneimittelabgabevorrichtung nach Anspruch 1, wobei ein Winkel zwischen dem distalen Abschnitt und dem länglichen Abschnitt ein stumpfer Winkel ist.

4. Implantierbare Arzneimittelabgabevorrichtung nach Anspruch 1, ferner umfassend ein Lumen, das sich von einem proximalen Ende der Arzneimittelabgabevorrichtung in den proximalen Abschnitt erstreckt.

5. Implantierbare Arzneimittelabgabevorrichtung nach Anspruch 4, wobei sich das Lumen von dem proximalen Ende der Arzneimittelabgabevorrichtung durch den proximalen Abschnitt und in den länglichen Abschnitt erstreckt.

6. Implantierbare Arzneimittelabgabevorrichtung nach Anspruch 4, wobei ein Arzneimittel innerhalb des Lumens angeordnet ist.

7. Implantierbare Arzneimittelabgabevorrichtung nach Anspruch 6, wobei das Arzneimittel eines von einem pulverförmigen Arzneimittel, einem pelletierten Arzneimittel und einem flüssigen Arzneimittel ist, das in einem viskosen Medium suspendiert ist.

8. Implantierbare Arzneimittelabgabevorrichtung nach Anspruch 1, wobei der Flansch ferner eine Membran (330) einschließt.

9. Implantierbare Arzneimittelabgabevorrichtung nach Anspruch 1, wobei der Flansch eine elliptische oder eine halbelliptische Form umfasst.

10. Implantierbare Arzneimittelabgabevorrichtung nach Anspruch 1, ferner umfassend einen Ellbogen, der zwischen dem proximalen Abschnitt und dem länglichen Abschnitt angeordnet ist.

11. Implantierbare Arzneimittelabgabevorrichtung nach Anspruch 10, wobei ein Querschnittsbereich des Ellenbogens kleiner ist als ein Querschnittsbereich des länglichen Abschnitts in der Nähe des Ellenbogens.

12. Implantierbare Arzneimittelabgabevorrichtung nach Anspruch 1, wobei ein Querschnittsbereich des länglichen Abschnitts über eine Länge des länglichen Abschnitts hinweg variiert, sodass der längliche Abschnitt verjüngt ist.

13. Implantierbares Arzneimittelabgabesystem, umfassend:
eine Arzneimittelabgabevorrichtung nach Anspruch 1; und
einen Applikator, der zum Einführen der Arzneimittelabgabevorrichtung in einen Punkt eines Auges konfiguriert ist, sodass mindestens der distale Abschnitt und der längliche Abschnitt vollständig innerhalb des Punktes des Auges liegen, wenn die Arzneimittelabgabevorrichtung eingeführt ist.

14. Implantierbares Arzneimittelabgabesystem nach Anspruch 13, wobei der Applikator einen Eingriffsmechanismus einschließt, der konfiguriert ist, um mit dem Flansch der Arzneimittelabgabevorrichtung in Kontakt zu stehen.

## Revendications

1. Dispositif implantable d'administration de médicaments comprenant :
une partie proximale ;
une partie distale (104) ; et
une partie allongée (160) disposée entre la partie proximale et la partie distale :
dans lequel la partie proximale est inclinée par rapport à la partie allongée,
dans lequel la partie distale est inclinée par rapport à la partie allongée,
dans lequel la partie proximale comprend une bride (110) disposée au niveau d'une extrémité proximale du dispositif implantable d'administration de médicaments,
dans lequel la partie proximale comprend en outre une partie de rétention (130) disposée entre la bride et la partie allongée, dans lequel la partie de rétention comprend une section transversale elliptique, et
dans lequel le dispositif d'administration de médicaments est conçu pour être inséré dans un punctum d'un œil, de telle sorte qu'au moins la partie distale et la partie allongée se trouvent entièrement dans le punctum de l'œil lorsque le dispositif d'administration de médicaments est inséré.

2. Dispositif implantable d'administration de médicaments selon la revendication 1, dans lequel un angle entre la partie proximale et la partie allongée est un angle droit.

3. Dispositif implantable d'administration de médicaments selon la revendication 1, dans lequel un angle entre la partie distale et la partie allongée est un angle obtus.

4. Dispositif implantable d'administration de médicaments selon la revendication 1, comprenant en outre une lumière s'étendant d'une extrémité proximale du dispositif d'administration de médicaments dans la partie proximale.

5. Dispositif implantable d'administration de médicaments selon la revendication 4, dans lequel la lumière s'étend de l'extrémité proximale du dispositif d'administration de médicaments à travers la partie proximale et dans la partie allongée.

6. Dispositif implantable d'administration de médicaments selon la revendication 4, dans lequel un médicament est disposé dans la lumière.

7. Dispositif implantable d'administration de médicaments selon la revendication 6, dans lequel le médicament est l'un parmi un médicament en poudre, un médicament sous forme de granules, et un médicament liquide en suspension dans un milieu visqueux.

8. Dispositif implantable d'administration de médicaments selon la revendication 1, dans lequel la bride comporte en outre une membrane (330).

9. Dispositif implantable d'administration de médicaments selon la revendication 1, dans lequel la bride comporte l'une parmi une forme elliptique ou semi-elliptique.

10. Dispositif implantable d'administration de médicaments selon la revendication 1, comprenant en outre un coude, disposé entre la partie proximale et la partie allongée.

11. Dispositif implantable d'administration de médicaments selon la revendication 10, dans lequel une zone de section transversale du coude est inférieure à une zone de section transversale de la partie allongée proche du coude.

12. Dispositif implantable d'administration de médicaments selon la revendication 1, dans lequel une zone de section transversale de la partie allongée varie sur l'ensemble d'une longueur de la partie allongée, de telle sorte que la partie allongée est effilée.

13. Système implantable d'administration de médicaments comprenant :
un dispositif d'administration de médicaments selon la revendication 1 ; et
un applicateur, conçu pour insérer le dispositif d'administration de médicaments dans un punctum d'un œil, de telle sorte qu'au moins la partie distale et la partie allongée se trouvent entièrement dans le punctum de l'œil lorsque le dispositif d'administration de médicaments est inséré.

14. Système implantable d'administration de médicaments selon la revendication 13, dans lequel l'applicateur comporte un mécanisme de mise en prise, conçu pour mettre en prise la bride du dispositif d'administration de médicaments.
